# EUROPEAN PATENT APPLICATION

(11) **EP 4 269 622 A1**
(43) Date of publication of application: **01.11.2023**
(21) Application number: 22382387.3
(22) Date of filing: 25.04.2022
(51) Int. Cl.: C12Q 1/6886

(54) **DNA COPY NUMBER ALTERATIONS FOR PREDICTING TREATMENT RESPONSE IN PATIENTS WITH BREAST CANCER**

(71) Applicant: Hospital Clínic de Barcelona, 08036 Barcelona (ES); Institut d'Investigacions Biomèdiques August Pi i Sunyer, 08036 Barcelona (ES); Universitat de Barcelona, 08028 Barcelona (ES); Fundació Privada Institut d'Investigació Oncològica de Vall-Hebron, 08035 Barcelona (ES); Reveal Genomics S.L., 08036 Barcelona (ES)
(72) Inventor: PRAT APARICIO, Aleix, 08036 Barcelona (ES); BRASÓ MARISTANY, Fara, 08036 Barcelona (ES); VILLAGRASA-GONZALEZ, Patricia, 08036 Barcelona (ES); VIVANCOS, Ana, 08035 Barcelona (ES)
(74) Representative: Hoffmann Eitle

(57) **Abstract**

The present invention refers to the *in vitro* use of DNA copy number alterations (CNAs) for predicting the response of patients with HR+/HER2- breast cancer to a treatment comprising targeted therapy, such as CDK4/6 inhibitors, and/or endocrine therapy; for the prognosis of patients with HR+/HER2- breast cancer; for monitoring patients with HR+/HER2- breast cancer; or for classifying patients with HR+/HER2- breast cancer into responder or non-responder to a treatment comprising targeted therapy, such as CDK4/6 inhibitors, and/or endocrine therapy.

## Description

### FIELD OF THE INVENTION

The present invention refers to the medical field. Particularly, the present invention refers to the *in vitro* use of DNA copy number alterations (CNAs) for predicting the response of patients with HR+/HER2- breast cancer to a treatment comprising targeted therapy such as a CDK4/6 inhibitor and/or endocrine therapy, for the prognosis of patients with HR+/HER2- breast cancer, for monitoring patients with HR+/HER2- breast cancer, or for classifying patients with HR+/HER2-breast cancer into responder or non-responder to a treatment comprising targeted therapy and/or endocrine therapy.

### STATE OF THE ART

Sequencing of tumor DNA has brought many new biomarkers and possibilities to precision oncology. Detection of somatic gene mutations, amplifications, and gene fusions allows the delivery of targeted therapies in multiple cancer-types, such as lung cancer, colorectal, melanoma and breast cancer. In addition, detection of a high number of somatic mutations (i.e., tumor mutational burden) or a microsatellite instability-high phenotype can help identify candidates for anti-PD1/PDL1 immune checkpoint inhibitors. Importantly, sequencing of circulating tumor DNA in blood samples (i.e., the so-called liquid biopsy, and henceforth called "ctDNA") allows an easy access to some tumor-based genetic information at any given timepoint and can replace a tumor tissue biopsy in some cases, thus avoiding delays and complications of a solid tumor invasive biopsy procedure, which can be quite challenging in the metastatic setting.

Identification of single tumor DNA alterations can be clinically useful. However, cancer is highly complex and additional biological information is likely needed to refine the prediction of patients' prognosis and/or treatment benefit. Breast cancer is the perfect example since RNA-based signature profiling tests provide clinical and biological useful information beyond individual somatic gene mutations or amplifications of genes such as *PIK3CA* or *ERBB2.* In early disease, multi-gene RNA-based prognostic assays (e.g., OncotypeDX, Mammaprint and Prosigna) are available and recommended by clinical guidelines. In advanced disease, RNA-based profiling is becoming a promising prognostic and predictive tool. Unfortunately, tissue samples in patients with advanced disease are not readily available and, even so, the type of metastatic organ or site can compromise the expression patterns obtained from bulk RNA and might not reflect the intra-patient tumor heterogeneity.

The present invention is focused on solving this technical and clinical problem and it is herein proposed to use DNA sequencing, preferably DNA sequencing of ctDNA in plasma, serum, breast milk, cerebrospinal fluid or blood to capture clinically relevant information beyond simple single genetic alterations. This approach could be highly relevant in the metastatic setting, where ctDNA might be the only readily available genetic material from tumors.

Although the ability of ctDNA to capture complex data with clinical value is unknown in the context of metastatic breast cancer, the present invention demonstrates that complex and clinically relevant tumor phenotypic traits can be identified in DNA, particularly in ctDNA.

### DESCRIPTION OF THE INVENTION

### Brief description of the invention

The present invention refers to the *in vitro* use of CNAs for predicting the response of HR+/HER2-breast cancer to a treatment comprising targeted therapy, such as CDK4/6 inhibition, and/or endocrine therapy, for the prognosis of patients with HR+/HER2- breast cancer, for monitoring patients with HR+/HER2-breast cancer, or for classifying patients with HR+/HER2- breast cancer into responder or non-responder to a treatment comprising targeted therapy, such as CDK4/6 inhibition, and/or endocrine therapy.

Particularly, the present invention is focused on the use of CNAs, preferably from ctDNA, to capture complex and clinically relevant tumor phenotypes in breast cancer.

The inventors of the present invention herein demonstrate that machine learning multi-gene signatures obtained from DNA, preferably ctDNA, sense many parts of a given pathway and identify several complex biological features, including measures of tumor proliferation and estrogen receptor signalling, similar to what is accomplished using direct tumor RNA profiling. For instance, it is herein demonstrated that a ctDNA-based genomic signature tracking retinoblastoma loss-of-heterozygosity (RB-LOH) is significantly associated with poor prognosis and drug response in patients with metastatic breast cancer treated with a CDK4/6 inhibitor and/or endocrine therapy, independently of tumor cell fraction and other clinical-pathological variables.

Kindly note that the contribution made by the present invention to the prior art is the possibility of using CNA over a plurality of DNA segments for predicting complex phenotypes in patients with HR+/HER2- breast cancer, for instance to predict whether the patient will respond to targeted therapy and/or endocrine therapy. So, the *special technical* feature conferring unity of invention to the present invention would be the assessment of the presence of CNA over a plurality of DNA segments on a plurality of chromosomes precisely in this clinical context, preferably departing for blood, serum, breast milk, cerebrospinal fluid or plasma samples.

So, the first embodiment of the present invention refers to an *in vitro* method for predicting the response of patients with HR+/HER2- breast cancer to a treatment comprising targeted therapy, such as CDK4/6 inhibitors, and/or endocrine therapy, which comprises: a) assessing the presence of CNA over a plurality of DNA segments on a plurality of chromosomes in a biological sample obtained from the patient; b) processing the measured CNAs in order to obtain a score; and c) wherein if a deviation or variation of the score value is identified, as compared with a pre-established reference value, this is indicative that the patient with HR+/HER2- breast cancer will respond or will be resistant to the treatment.

The second embodiment of the present invention refers to an *in vitro* method for the prognosis of patients with HR+/HER2- breast cancer which comprises: a) assessing the presence of CNAs over a plurality of DNA segments on a plurality of chromosomes in a biological sample obtained from the patient; b) processing the measured CNAs in order to obtain a score; and c) wherein if a deviation or variation of the score value is identified, as compared with a pre-established reference value, this is indicative of the prognosis of the patient with HR+/HER2- breast cancer.

The third embodiment of the present invention refers to an *in vitro* method for monitoring patients with HR+/HER2- breast cancer to assess whether they are responding to treatment comprising targeted therapy, such as CDK4/6 inhibitors, and/or endocrine therapy, which comprises: a) assessing the presence of CNAs over a plurality of DNA segments on a plurality of chromosomes in a biological sample obtained from the patient; b) processing the measured CNAs in order to obtain a score; and c) wherein if a deviation or variation of the score value is identified, as compared with a pre-established reference value, this is indicative that the patient with HR+/HER2- breast cancer will respond or will be resistant to the treatment.

The fourth embodiment of the present invention refers to an *in vitro* method for classifying patients with HR+/HER2- breast cancer into biologically and clinically relevant groups which are associated with a different response to a treatment comprising targeted therapy, such as CDK4/6 inhibitors, and/or endocrine therapy, which comprises: a) assessing the presence of CNAs over a plurality of DNA segments on a plurality of chromosomes in a biological sample obtained from the patient; b) processing the measured CNAs in order to obtain multiple scores; c) classifying breast cancer samples based on their scoring profile into different groups as compared with a pre-established reference value; and d) wherein each group is indicative whether the patient with HR+/HER2- breast cancer will respond to the treatment.

In a preferred embodiment of the invention, the method is a computer-implemented method which comprises: a) receiving a plurality of CNAs data sets from the patient; b) processing the information according to step a) for finding a statistically significant variations or deviations; and c) providing a result by the computer system based on the information received according to the step a) and a pre-established standard already stored in the computer.

In a preferred embodiment, the "score" is obtained after assessing the presence of CNAs in tumoral DNA segments which are present in the biological sample. The signal of each segment is calculated by averaging the signal of each gene within each segment. The final score is calculated by multiplying the signal of each DNA segment by a previously established coefficient or weight and summing up all of them. After processing all the values, a single score is obtained. This single score will be compared with the "pre-established reference value" to finally make clinical decisions. The "pre-established reference value" is a threshold value obtained after assessing the presence of CNAs in "normal DNA" segments (i.e. non-tumoral DNA) segments, which are present in the biological sample. Particularly, if a deviation or variation of the "score" value is identified, typically a "score" value higher or lower than the "pre-established reference value", this is indicative that the patient with HR+/HER2- breast cancer will respond or will be resistant to the treatment, or that the patient will have a good or poor prognosis.

In a preferred embodiment of the invention, the presence of CNAs is assessed in any of the following the DNA segments (see **Table 1, Table 3** and **Table 4)** (the nomenclature of the segments is as found in NCBI Genome data): chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 or chr3:58626894-61524607.

In a preferred embodiment of the invention, the presence of CNAs is assessed in all the following the DNA segments (see **Table 1, Table 3** and **Table 4**): chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 or chr3:58626894-61524607.

**Table 1. A list of the 16 preferred DNA segments is shown.**

| Segment number | Segment |
|---|---|
| 1 | chr16:1-38200000 |
| 2 | chr17:1-22200000 |
| 3 | chr2:1-93300000 |
| 4 | chr10:129812260-135374737 |
| 5 | chr12:1-1311104 |
| 6 | chr13:46362859-48209064 |
| 7 | chr17:63 9421 09-65847254 |
| 8 | chr17:7471230-7717938 |
| 9 | chr19:1-526082 |
| 10 | chr2:32460827-55039898 |
| 11 | chr20:33386980-33969561 |
| 12 | chr3:58626894-61524607 |
| 13 | chr4:83634873-83961360 |
| 14 | chr5:76408288-81082828 |
| 15 | chr7:16017926-18944036 |
| 16 | chr8:128774432-128849112 |

**Table 2. A list of the 27 preferred analysed phenotypes is shown.**

| |
|---|
| 1. Histology: IDC vs. ILC_PMID.31827079 |
| 2. Chemo_Endocnne_Score_PMID.27903675 |
| 3. Knudsen_Neo_common_Median_Clin.Cancer.Res.2014_PMID .25047707 |
| 4. MDACC_P53_ER.Pos_Median_CCR.2011_PMID.21248301 |
| 5. Pcorr_bronchioid_PLOS.2012_PMID.22590557 |
| 6. Pcorr_CSR_Activated_Correlauon_PNAS.2005_PMID.15701700 |
| 7. Pcorr_NKI70_Good_Correlauon_Nature.2002_PMID.11823860 |
| 8. Scorr_PTEN_Present_Correlauon_PNAS.2007_PMID.17452630 |
| 9. Stingl_Day7_Upregulated_Median_Nat.Cell.Biol.2014_PMID.25173976 |
| 10. UNC_8q_Amplicon_Median_BMC.Med.Genomics.2011_PMID.21214954 |
| 11. UNC_bMYB_Signature_Median_Oncogene.2009_PMID.19043454 |
| 12. UNC_HER1_Cluster1_Median_BMC.Genomics.2007_PMID.17663798 |
| 13. UNC_HS_Red23_Median_BMC.Med.Genomics.2011_PMID.21214954 |
| 14. UNC_MM_C3Tag_1pFDR_UP_Median_Genome.Biology.2007_PMID.17493263 |
| 15. UNC_MM_C3Tag.2012_Median_Genome.Biol.2013_PMID.24220145 |
| 16. UNC_MM_Green19_Median_BMC.Med.Genomics.2011PMID.21214954 |
| 17. UNC_MM_Myc.2012_Median_Genome.Biol.2013_PMID.24220145 |
| 18. UNC_MM_p53null.Luminal_Median_Genome.Biol.2013_PMID.24220145 |
| 19. UNC_MM_Red10_Median_BMC.Med.Genomics.2011_PMID.21214954 |
| 20. UNC_MM_Red5_Median_BMC.Med.Genomics.2011_PMID.21214954 |
| 21. UNC_MM_WAPTag_1pFDR_UP_Median_Genome.Biology.2007_PMID.17493263 |
| 22. UNC_MProliferauon_Median_BMC.Med.Genomics.2011_PMID.21214954 |
| 23. UNC_PIK3CA.Hutti_Median_Cancer.Res.2012_PMID.22552288 |
| 24. UNC_Proliferation_Cluster_Median_BMC.Med.Genomics.2011_PMID.21214954 |
| 25. UNC_RB_LOH_Median_Breast.Cancer.Res.2008_PMID.18782450 |
| 26. UNC_Scorr_LumA_Correlation_JCO.2009_PMID.19204204 |
| 27. Wirapati_Proliferation_Median_Breast.Cancer.Res.2008_PMID.18662380 |

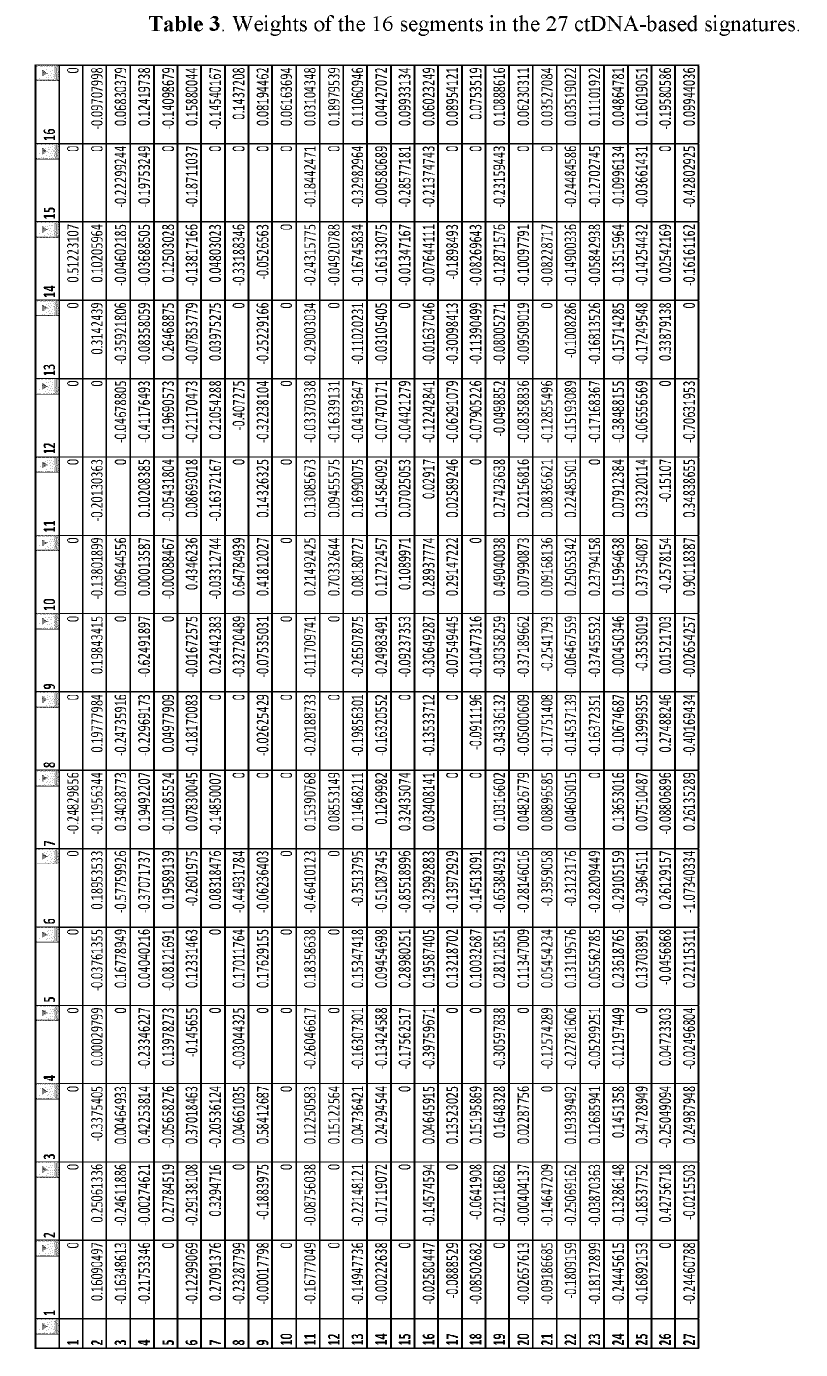

**Table 4. List of the preferred 16 segments and the number of times they are present in paired combinations associated with progression-free survival (PFS) in 87 patients with advanced HR+/HER2- breast cancer treated with CDK4/6 inhibitors and endocrine therapy.**

| **Segment** | **number of combinations where the segment is present** |
|---|---|
| chr20:33386980-33969561 | 10 |
| chr13:46362859-48209064 | 8 |
| chr17:63942109-65847254 | 8 |
| chr17:1-22200000 | 6 |
| chr10:129812260-135374737 | 6 |
| chr17:7471230-7717938 | 6 |
| chr2:32460827-55039898 | 6 |
| chr7:16017926-18944036 | 6 |
| chr2:1-93300000 | 5 |
| chr8:128774432-128849112 | 5 |
| chr12:1-1311104 | 4 |
| chr16:1-38200000 | 3 |
| chr4:83634873-83961360 | 3 |
| chr5:76408288-81082828 | 2 |
| chr19:1-526082 | 1 |
| chr3:58626894-61524607 | 1 |

In a preferred embodiment of the invention, the presence of CNAs is assessed in any or all the DNA segments of **Table 5**, **Table 6** or **Table 7**.

**Table 5. Quantitative SAM analysis of chromosomic regions associated with RB-LOH signature score.**

| **Segment** | **Score(d)** | **Numerator(r)** | **Denominator(s+s0)** | **q-value(%)** |
|---|---|---|---|---|
| chr8:116186189-120600761 | 5.3003766 | 0.00629524 | 0.0011877 | 0 |
| chr8:101163387-103693879 | 5.137426892 | 0.006102016 | 0.001187757 | 0 |
| chr8:128774432-128849112 | 5.13213462 | 0.00610846 | 0.00119024 | 0 |
| chr8:128414451-128718816 | 5.13213462 | 0.00610846 | 0.00119024 | 0 |
| chr8:45200000-146274826 | 4.66055289 | 0.00512744 | 0.00110018 | 0 |
| chr8:133698327-133914560 | 4.54256238 | 0.00545578 | 0.00120104 | 0 |
| chr8:108043446-108683856 | 4.46955751 | 0.00549422 | 0.00122925 | 0 |
| chr8:94587173-100685605 | 4.31815782 | 0.00519255 | 0.00120249 | 0 |
| chr8:128823745-146264218 | 4.24336667 | 0.004946 | 0.00116558 | 0 |
| chr8:82099049-82634968 | 4.07171132 | 0.0049816 | 0.00122347 | 0 |
| chr8:140458177-146274826 | 4.0562674 | 0.004774 | 0.00117694 | 0 |
| chr21:38584860-42033506 | 4.04725769 | 0.0060921 | 0.00150524 | 0 |
| chr8:81242335-81979194 | 4.01486916 | 0.00492876 | 0.00122763 | 0 |
| chr8:84799647-85622331 | 3.837371 | 0.00471717 | 0.00122927 | 0 |
| chr21:46690655-46902240 | 3.82366685 | 0.00577451 | 0.0015102 | 0 |
| chr21:12300000-46944323 | 3.80979025 | 0.0054265 | 0.00142436 | 0 |
| chr21:44316476-46902240 | 3.77631373 | 0.00560693 | 0.00148476 | 0 |
| chr8:42971602-72924037 | 3.77245098 | 0.00431187 | 0.00114299 | 0 |
| chr8:62174237-62716885 | 3.51328293 | 0.00445035 | 0.00126672 | 0 |
| chr20:51603033-51989829 | 3.27286425 | 0.00342639 | 0.00104691 | 0 |
| chr20:49438571-52269898 | 3.25747736 | 0.00338854 | 0.00104023 | 0 |
| chr3:170024984-173604597 | 3.03221587 | 0.00261865 | 0.00086361 | 0 |
| chr20:59868877-61137250 | 2.80223893 | 0.00296153 | 0.00105684 | 0 |
| chr10:3811260-6662244 | 2.78225763 | 0.00288947 | 0.00103854 | 0 |
| chr20:61329497-62435964 | 2.76031759 | 0.00292199 | 0.00105857 | 0 |
| chr10:101955-12088176 | 2.73575873 | 0.0028391 | 0.00103777 | 0 |
| chr10:3811260-5139878 | 2.69853071 | 0.0027569 | 0.00102163 | 0 |
| chr10:7244255-12332593 | 2.68151831 | 0.00286274 | 0.00106758 | 0 |
| chr10:3099740-3205003 | 2.67585994 | 0.00275233 | 0.00102858 | 0 |
| chr10:12431738-17283687 | 2.55219414 | 0.0026964 | 0.0010565 | 0 |
| chr6:110391-1254918 | 2.53580076 | 0.00233928 | 0.0009225 | 0 |
| chr6:1543157-2570302 | 2.53580076 | 0.00233928 | 0.0009225 | 0 |
| chr10:1-1042949 | 2.52543302 | 0.00259658 | 0.00102817 | 0 |
| chr10:170643-1769670 | 2.52543302 | 0.00259658 | 0.00102817 | 0 |
| chr10:20145174-20609292 | 2.5132206 | 0.00263163 | 0.00104711 | 0 |
| chr3:91700000-199501827 | 2.51320583 | 0.00180226 | 0.00071711 | 0 |
| chr3:175446835-178263192 | 2.46718428 | 0.00227534 | 0.00092224 | 0 |
| chr10:17311250-19896508 | 2.4660255 | 0.00258327 | 0.00104754 | 0 |
| chr10:21110098-22746531 | 2.44993088 | 0.00254624 | 0.00103931 | 0 |
| chr20:45719063-49181611 | 2.4388184 | 0.00222031 | 0.0009104 | 0 |
| chr3:178149984-199501827 | 2.37693371 | 0.00217223 | 0.00091388 | 0 |
| chr21:16248572-25859193 | 2.33781941 | 0.00327629 | 0.00140143 | 0 |
| chr20:27100000-62435964 | 2.29216979 | 0.00190642 | 0.00083171 | 0 |
| chr10:1-40300000 | 2.26259622 | 0.00214422 | 0.00094768 | 0 |
| chr20:33386980-33969561 | 2.17758855 | 0.00219335 | 0.00100724 | 0 |
| chr6:1-23628840 | 2.15463532 | 0.00186587 | 0.00086598 | 0 |
| chr10:22863772-24876778 | 2.14904487 | 0.00222963 | 0.0010375 | 0 |
| chr6:38787570-41935171 | 2.09586129 | 0.00178632 | 0.00085231 | 0.47635892 |
| chr6:4058046-25852810 | 1.95784386 | 0.00170288 | 0.00086977 | 1.07965964 |
| chr6:1-60500000 | 1.75099348 | 0.00147141 | 0.00084033 | 3.27496757 |
| chr1:144078052-144943150 | 1.70464266 | 0.00178049 | 0.00104449 | 3.27496757 |
| chr6:42772314-43039595 | 1.67693013 | 0.00154288 | 0.00092006 | 3.27496757 |
| chr6:43556800-44361368 | 1.67693013 | 0.00154288 | 0.00092006 | 3.27496757 |
| chr17:62318152-63890591 | 1.6407882 | 0.00184485 | 0.00112437 | 4.7635892 |
| chr6:13729714-14088212 | 1.58375 | 0.00145365 | 0.00091785 | 4.7635892 |
| chr6:63255006-65243766 | 1.58270163 | 0.00172889 | 0.00109237 | 4.7635892 |
| chr1:119996566-120303234 | 1.54578695 | 0.00164566 | 0.00106461 | 7.4508556 |
| chr20:34179462-45265004 | 1.51785675 | 0.00132033 | 0.00086987 | 7.4508556 |
| chr17:63942109-65847254 | 1.48432477 | 0.00173498 | 0.00116887 | 10.4028382 |
| chr1:148661965-149063439 | 1.43727936 | 0.0015353 | 0.0010682 | 10.4028382 |
| chr10:19022250-42889659 | 1.41700448 | 0.00128342 | 0.00090573 | 11.4572533 |
| chr3:116900556-120107320 | 1.41619007 | 0.00118201 | 0.00083464 | 11.4572533 |
| chr11:73357303-74035012 | 1.41531748 | 0.00182604 | 0.0012902 | 11.4572533 |
| chr1:151026302-152973244 | 1.38133007 | 0.00136715 | 0.00098973 | 11.4572533 |
| chr6:30420923-31893702 | 1.36922994 | 0.00135466 | 0.00098936 | 11.4572533 |
| chr20:40134806-41603948 | 1.36750746 | 0.00130123 | 0.00095153 | 11.4572533 |
| chr1:201678483-203358272 | 1.36119565 | 0.00144726 | 0.00106322 | 11.4572533 |
| chr6:31910383-31915520 | 1.3077885 | 0.00131158 | 0.0010029 | 11.8387027 |
| chr10:73394126-73443318 | 1.28884887 | 0.00130623 | 0.00101349 | 11.8387027 |
| chr11:74085123-74337880 | 1.28326942 | 0.00171741 | 0.00133831 | 11.8387027 |
| chr11:74091491-74180816 | 1.20752764 | 0.00162112 | 0.00134251 | 12.5320741 |
| chr2:61834240-63151654 | 1.18673185 | 0.0009452 | 0.00079648 | 12.6363364 |
| chr17:59852249-60135967 | 1.1800452 | 0.00136421 | 0.00115607 | 12.6363364 |
| chr10:31648148-32903498 | 1.12185768 | 0.00108129 | 0.00096384 | 13.019503 |
| chr17:75915141-77213225 | 1.04435439 | 0.00117388 | 0.00112402 | 14.1947848 |
| chr1:39907605-40263248 | 1.03886469 | 0.00099998 | 0.00096257 | 14.1947848 |
| chr1:124300000-247249719 | 1.02965604 | 0.00098561 | 0.00095722 | 14.1947848 |
| chr10:24604620-28074753 | 1.02811318 | 0.00107529 | 0.00104589 | 14.1947848 |
| chr20:41621022-45719023 | 1.02785527 | 0.0009419 | 0.00091637 | 14.1947848 |
| chr1:118282114-118807006 | 0.99919901 | 0.00112778 | 0.00112869 | 14.1947848 |
| chr6:54352087-55392731 | 0.99540114 | 0.00093459 | 0.00093891 | 14.1947848 |
| chr1:162199047-163197517 | 0.99308973 | 0.00101973 | 0.00102682 | 14.1947848 |
| chr1:158317017-159953843 | 0.98598124 | 0.00099625 | 0.00101042 | 14.1947848 |
| chr10:74560456-82020637 | 0.97550233 | 0.00096302 | 0.00098721 | 14.1947848 |
| chr3:41866-18694914 | 0.96701605 | 0.00090276 | 0.00093356 | 14.1947848 |
| chr17:58249995-58447640 | 0.95898481 | 0.00110905 | 0.00115649 | 14.1947848 |
| chr8:42006632-42404492 | 0.95158576 | 0.00139808 | 0.00146922 | 14.4517464 |
| chr9:71280626-72167093 | 0.94777358 | 0.0008859 | 0.00093472 | 14.4517464 |
| chr10:75957348-77987139 | 0.93496583 | 0.00096031 | 0.00102711 | 14.4517464 |
| chr1:163619952-164752515 | 0.92960478 | 0.00095317 | 0.00102534 | 14.4517464 |
| chr17:55144989-55540417 | 0.92802914 | 0.0010748 | 0.00115816 | 14.4517464 |
| chr1:71284749-74440273 | 0.92167152 | 0.00091507 | 0.00099283 | 14.4517464 |
| chr17:51748757-53292386 | 0.88588428 | 0.00101297 | 0.00114345 | 14.4517464 |
| chr9:130999928-132303780 | 0.88382423 | 0.00079188 | 0.00089597 | 14.4517464 |
| chr11:69588488-69842871 | 0.88258376 | 0.00112371 | 0.0012732 | 14.4517464 |
| chr8:41004735-41425303 | 0.87128674 | 0.00128089 | 0.00147012 | 14.4517464 |
| chr17:70767943-71305641 | 0.85525577 | 0.00094659 | 0.0011068 | 15.4312031 |
| chr20:29526118-29834552 | 0.8278442 | 0.00078403 | 0.00094707 | 15.4312031 |
| chr17:77653047-78605474 | 0.82552159 | 0.00092869 | 0.00112498 | 15.4312031 |
| chr17:78087533-78774742 | 0.82552159 | 0.00092869 | 0.00112498 | 15.4312031 |
| chr6:137619861-138589104 | 0.82308109 | 0.00116712 | 0.00141799 | 15.4312031 |
| chr10:28141103-31360860 | 0.81750584 | 0.00083209 | 0.00101784 | 15.4312031 |
| chr6:139381116-139689363 | 0.78281881 | 0.00111217 | 0.00142073 | 15.9412506 |
| chr10:35175415-37212810 | 0.76697814 | 0.00071645 | 0.00093413 | 15.9412506 |
| chr11:32027116-37799354 | 0.76180283 | 0.00079147 | 0.00103895 | 15.9412506 |
| chr10:54201467-57779853 | 0.7417371 | 0.00068959 | 0.00092969 | 16.7023346 |
| chr1:41717036-43943775 | 0.73122334 | 0.00069289 | 0.00094758 | 16.7023346 |
| chr1:46541957-46555032 | 0.72269967 | 0.00069339 | 0.00095944 | 16.7023346 |
| chr2:1-15244284 | 0.70449366 | 0.00058097 | 0.00082466 | 16.7023346 |
| chr11:42387077-47453403 | 0.69995923 | 0.00066864 | 0.00095525 | 16.7023346 |
| chr1:43945805-44960161 | 0.67748052 | 0.00064437 | 0.00095112 | 17.6207495 |
| chr9:122980237-124410900 | 0.67564568 | 0.000628 | 0.00092949 | 17.6207495 |
| chr6:135561194-135665525 | 0.67460049 | 0.00096427 | 0.00142939 | 17.6207495 |
| chr10:38685321-38707438 | 0.67392033 | 0.00058498 | 0.00086803 | 17.6207495 |
| chr11:69098089-69278404 | 0.6721418 | 0.00081826 | 0.0012174 | 17.6207495 |
| chr19:63402921-63811651 | 0.66228232 | 0.00088462 | 0.00133571 | 17.6207495 |
| chr14:1-23145193 | 0.63596986 | 0.0005652 | 0.00088871 | 17.9594996 |
| chr1:58658784-60221344 | 0.63479055 | 0.00063065 | 0.00099348 | 17.9594996 |
| chr10:52313829-53768264 | 0.62842742 | 0.00058047 | 0.00092369 | 17.9594996 |
| chr19:49818193-50114786 | 0.62660054 | 0.00071265 | 0.00113733 | 17.9594996 |
| chr9:51800000-140273252 | 0.60246899 | 0.00051957 | 0.0008624 | 17.9594996 |
| chr9:110838572-127593510 | 0.59084838 | 0.00053778 | 0.00091018 | 17.9594996 |
| chr19:59066340-59471027 | 0.58885663 | 0.00077729 | 0.00132001 | 19.1856368 |
| chr11:76699529-78005085 | 0.58002569 | 0.0008884 | 0.00153166 | 19.1856368 |
| chr1:223876038-247249719 | 0.57964344 | 0.00060661 | 0.00104652 | 19.1856368 |
| chr6:129217882-131730157 | 0.57681315 | 0.0008314 | 0.00144138 | 19.1856368 |
| chr2:15977811-16073001 | 0.56933889 | 0.00046155 | 0.00081067 | 19.1856368 |
| chr19:34975531-35098303 | 0.56267814 | 0.00069003 | 0.00122633 | 19.1856368 |
| chr19:28500000-63811651 | 0.552155 | 0.00063991 | 0.00115894 | 19.1856368 |
| chr2:1-93300000 | 0.53609764 | 0.00040209 | 0.00075003 | 19.1856368 |
| chr9:137859478-140273252 | 0.53046348 | 0.00048932 | 0.00092243 | 19.1856368 |
| chr1:166729757-167532460 | 0.51390769 | 0.00052646 | 0.00102442 | 20.2054555 |
| chr3:1-2121282 | 0.51123367 | 0.00047958 | 0.00093808 | 20.2054555 |
| chr14:1-29140968 | 0.5099195 | 0.00045469 | 0.00089168 | 20.2054555 |
| chr19:47121673-49919582 | 0.50912972 | 0.00057712 | 0.00113354 | 20.2054555 |
| chr9:110661885-116923292 | 0.50767768 | 0.00046437 | 0.00091469 | 20.2054555 |
| chr19:52031294-53331283 | 0.49578954 | 0.00056535 | 0.0011403 | 20.2054555 |
| chr19:43177306-45393020 | 0.46798857 | 0.00055513 | 0.0011862 | 21.2538712 |
| chr14:21961377-23608756 | 0.46346961 | 0.0004173 | 0.00090038 | 21.2538712 |
| chr16:5062786-7709383 | 0.45020506 | 0.00047605 | 0.0010574 | 21.2538712 |
| chr17:33754231-34162198 | 0.4197212 | 0.00057301 | 0.00136521 | 21.9497827 |
| chr9:97183463-97625342 | 0.40889668 | 0.00037209 | 0.00090999 | 22.1434356 |
| chr11:68230745-68388280 | 0.38810406 | 0.00047993 | 0.00123661 | 22.1434356 |
| chr2:79257340-80427237 | 0.36733518 | 0.00028656 | 0.0007801 | 22.1434356 |
| chr19:53372336-53572212 | 0.35416223 | 0.00042797 | 0.0012084 | 22.1434356 |
| chr7:16017926-18944036 | 0.33265925 | 0.00031642 | 0.00095117 | 22.1434356 |
| chr11:57861150-58074632 | 0.31607261 | 0.00038022 | 0.00120295 | 22.1434356 |
| chr11:26341787-27574592 | 0.31228436 | 0.00031686 | 0.00101465 | 22.1434356 |
| chr17:45731662-46009941 | 0.30718334 | 0.00037479 | 0.00122009 | 22.1434356 |
| chr17:31895171-33758810 | 0.29259588 | 0.00038169 | 0.00130449 | 22.1434356 |
| chr19:44889764-44995371 | 0.27209068 | 0.00031983 | 0.00117545 | 22.1434356 |
| chr16:11352227-11658331 | 0.27202404 | 0.00028983 | 0.00106546 | 22.1434356 |
| chr11:57344931-58658339 | 0.23916241 | 0.00028341 | 0.00118502 | 22.1434356 |
| chr6:161612277-163134099 | 0.22230252 | 0.00026356 | 0.0011856 | 22.1434356 |
| chr17:44673157-45060263 | 0.21871394 | 0.00026532 | 0.00121309 | 22.1434356 |
| chr14:23609961-31698685 | 0.21819655 | 0.0001949 | 0.00089324 | 22.1434356 |
| chr12:67440273-67566002 | 0.2051435 | 0.00023152 | 0.00112857 | 22.1434356 |
| chr12:68249634-68327233 | 0.2051435 | 0.00023152 | 0.00112857 | 22.1434356 |
| chr6:76630464-105342994 | 0.20086661 | 0.0002787 | 0.00138748 | 22.1434356 |
| chr15:96891354-97698742 | 0.17471052 | 0.0001798 | 0.00102911 | 22.1434356 |
| chr19:3 9664720-3 9962225 | 0.16240819 | 0.00019281 | 0.00118719 | 22.1434356 |
| chr19:39677306-41155075 | 0.14167743 | 0.0001678 | 0.00118438 | 22.1434356 |
| chr7:1-59100000 | 0.14063166 | 0.00012015 | 0.00085433 | 22.1434356 |
| chr2:32460827-55039898 | 0.13778739 | 0.00011008 | 0.00079892 | 22.1434356 |
| chr12:64461446-64607139 | 0.12017571 | 0.00013309 | 0.0011075 | 22.1434356 |
| chr1:114889793-115149999 | 0.12014553 | 0.00013264 | 0.00110401 | 22.1434356 |
| chr12:70849987-70966467 | 0.11819621 | 0.00012459 | 0.00105409 | 22.1434356 |
| chr1:174611641-175371076 | 0.08927859 | 9.45E-05 | 0.00105821 | 22.1434356 |
| chr12:64149851-66323376 | 0.08869595 | 9.85E-05 | 0.00111036 | 22.1434356 |
| chr1:241364021-247249719 | 0.08102443 | 9.09E-05 | 0.001122 | 22.1434356 |
| chr1:168438419-168880930 | 0.05502224 | 5.84E-05 | 0.00106167 | 22.1434356 |
| chr1:169549478-170484405 | 0.05502224 | 5.84E-05 | 0.00106167 | 22.1434356 |
| chr19:32764651-33569283 | 0.04880496 | 6.04E-05 | 0.00123786 | 22.1434356 |
| chr9:21489625-22474701 | 0.04218328 | 5.94E-05 | 0.00140742 | 22.1434356 |
| chr9:23819824-26891068 | 0.04218328 | 5.94E-05 | 0.00140742 | 22.1434356 |
| chr9 :26573226-29470065 | 0.04218328 | 5.94E-05 | 0.00140742 | 22.1434356 |
| chr6:60500000-170899992 | 0.04067353 | 4.39E-05 | 0.00107956 | 22.1434356 |
| chr13:92308911-94031607 | 0.02810376 | 3.17E-05 | 0.00112682 | 22.1434356 |
| chr6:106604408-107782614 | 0.00688749 | 1.03E-05 | 0.00148888 | 22.1434356 |
| chr12:33156845-33480710 | 0.00178158 | 1.82E-06 | 0.00102158 | 22.1434356 |
| chr17:22200000-78774742 | 0.00176928 | 1.74E-06 | 0.00098444 | 22.1434356 |
| chr13:89500014-93206506 | -0.0166041 | -1.85E-05 | 0.00111151 | 11.4572533 |
| chr11:76710709-85146692 | -0.0246286 | -2.78E-05 | 0.0011305 | 11.4572533 |
| chr9:34233377-35934224 | -0.0255578 | -2.62E-05 | 0.00102332 | 11.4572533 |
| chr10:61909390-66364553 | -0.0302223 | -2.71E-05 | 0.00089686 | 11.4572533 |
| chr9:37861914-38370450 | -0.0353309 | -3.55E-05 | 0.00100448 | 11.4572533 |
| chr19:37265855-38702029 | -0.0495488 | -7.31E-05 | 0.00147521 | 11.4572533 |
| chr11:55503467-56066044 | -0.0810423 | -8.44E-05 | 0.00104124 | 11.4572533 |
| chr16:85920445-87225756 | -0.0863115 | -0.0001042 | 0.00120704 | 11.4572533 |
| chr16:87232502-87749670 | -0.0863115 | -0.0001042 | 0.00120704 | 11.4572533 |
| chr16:88436931-88827254 | -0.0863115 | -0.0001042 | 0.00120704 | 11.4572533 |
| chr16:45546775-51138307 | -0.1094873 | -0.0001299 | 0.00118642 | 11.4572533 |
| chr1:110339388-119426489 | -0.1104935 | -0.000116 | 0.00105013 | 11.4572533 |
| chr17:35067383-35272328 | -0.1150276 | -0.0001517 | 0.00131867 | 11.4572533 |
| chr8:39008109-41238710 | -0.1191558 | -0.0001734 | 0.00145503 | 11.4572533 |
| chr15:88253886-88997242 | -0.1221732 | -0.00012 | 0.00098241 | 11.4572533 |
| chr7:3046420-4279470 | -0.1264798 | -0.0001128 | 0.00089145 | 11.4572533 |
| chr9:1-51800000 | -0.1270563 | -0.0001288 | 0.00101387 | 11.4572533 |
| chr14:31868274-34078694 | -0.1561626 | -0.0001412 | 0.00090433 | 11.4572533 |
| chr11:52900000-134452384 | -0.1574913 | -0.0001361 | 0.00086389 | 11.4572533 |
| chr14:28763785-29367842 | -0.1592217 | -0.0001437 | 0.00090262 | 11.4572533 |
| chr12:131913408-132349534 | -0.159644 | -0.0001848 | 0.00115745 | 11.4572533 |
| chr7:3516940-6019790 | -0.1612888 | -0.0001432 | 0.000888 | 11.4572533 |
| chr9:7161607-12713130 | -0.1688728 | -0.0001824 | 0.00108025 | 11.4572533 |
| chr11:116803699-119605859 | -0.1700913 | -0.0002016 | 0.00118515 | 11.4572533 |
| chr16:31854743-53525739 | -0.1807616 | -0.0002091 | 0.00115665 | 11.4572533 |
| chr12:30999223-32594050 | -0.1910233 | -0.0001925 | 0.00100783 | 11.4572533 |
| chrl:1-124300000 | -0.2038957 | -0.0001794 | 0.00087993 | 11.4572533 |
| chr12:63329154-63614508 | -0.2242889 | -0.0002394 | 0.00106732 | 11.4572533 |
| chr16:82644872-85172804 | -0.2251834 | -0.0002672 | 0.00118681 | 11.4572533 |
| chr16:51646446-54466783 | -0.2292261 | -0.0002733 | 0.00119243 | 11.4572533 |
| chr2:121187279-122198612 | -0.2299891 | -0.0001839 | 0.00079968 | 11.4572533 |
| chr9:12683435-13935586 | -0.2310786 | -0.0002483 | 0.0010745 | 11.4572533 |
| chr6:101000242-121511318 | -0.2419333 | -0.000348 | 0.00143845 | 11.4572533 |
| chr16:69398793-73198518 | -0.2517979 | -0.0002997 | 0.00119041 | 11.4572533 |
| chr16:80759878-82408573 | -0.2624179 | -0.0003089 | 0.00117709 | 11.4572533 |
| chr6:157695838-157823719 | -0.2635729 | -0.0003046 | 0.00115582 | 11.4572533 |
| chr16:80588753-82633263 | -0.2783021 | -0.0003275 | 0.00117687 | 11.4572533 |
| chr16:45393460-45522699 | -0.281091 | -0.0003401 | 0.00120979 | 11.4572533 |
| chr9:831690-1047552 | -0.2814488 | -0.0003022 | 0.0010738 | 11.4572533 |
| chr3:86250885-95164178 | -0.2995918 | -0.0002849 | 0.00095093 | 11.4572533 |
| chr16:38200000-88827254 | -0.3047566 | -0.0003521 | 0.00115529 | 11.4572533 |
| chr9:36365710-37139941 | -0.3079381 | -0.0003135 | 0.00101802 | 11.4572533 |
| chr9:235706-8362053 | -0.3119499 | -0.0003342 | 0.00107136 | 11.4572533 |
| chr16:76685816-78205652 | -0.3189586 | -0.0003749 | 0.00117542 | 11.4572533 |
| chr16:79132355-80549399 | -0.3189586 | -0.0003749 | 0.00117542 | 11.4572533 |
| chr12:94480961-96479233 | -0.3224384 | -0.0003264 | 0.0010123 | 11.4572533 |
| chr20:22719364-23648289 | -0.3267909 | -0.000441 | 0.00134936 | 11.4572533 |
| chr9:14071847-14900353 | -0.342786 | -0.0003664 | 0.00106875 | 11.4572533 |
| chr17:41015664-41353348 | -0.3451816 | -0.0003851 | 0.00111574 | 11.4572533 |
| chr16:73739922-78192112 | -0.3581572 | -0.0004235 | 0.00118254 | 11.4572533 |
| chr6:117109043-117359993 | -0.3630232 | -0.0005294 | 0.00145839 | 11.4572533 |
| chr16:54782803-56638303 | -0.3727896 | -0.0004413 | 0.00118389 | 11.4572533 |
| chr6:116369389-117096650 | -0.3752109 | -0.0005472 | 0.00145837 | 11.4572533 |
| chr16:56791457-72871981 | -0.3852302 | -0.0004527 | 0.00117516 | 11.4572533 |
| chr12:29425476-31105002 | -0.3926835 | -0.0003998 | 0.00101815 | 11.4572533 |
| chr16:65206155-69392572 | -0.3980973 | -0.0004682 | 0.00117611 | 11.4572533 |
| chr14:34100051-35410919 | -0.4169905 | -0.0003742 | 0.00089744 | 11.4572533 |
| chr9:36988416-36998984 | -0.4242783 | -0.0004337 | 0.00102224 | 11.4572533 |
| chr11:112785528-114880322 | -0.4301101 | -0.0005166 | 0.00120101 | 11.4572533 |
| chr6:119295855-120272161 | -0.4358272 | -0.0006381 | 0.00146409 | 11.4572533 |
| chr12:95089777-95350380 | -0.4387441 | -0.0004503 | 0.00102643 | 11.4572533 |
| chr12:85072329-85674601 | -0.4407872 | -0.0004469 | 0.00101385 | 11.4572533 |
| chr7:83246850-83689170 | -0.4426787 | -0.0004764 | 0.00107615 | 11.4572533 |
| chr16:56705000-65205296 | -0.4508006 | -0.0005302 | 0.0011762 | 11.4572533 |
| chr13:106620319-107317084 | -0.4512139 | -0.0005225 | 0.00115802 | 11.4572533 |
| chr13:72212826-74986423 | -0.4554302 | -0.0005126 | 0.00112553 | 11.4572533 |
| chr7:86091574-88424037 | -0.4572051 | -0.0004897 | 0.00107111 | 11.4572533 |
| chr11:1-52900000 | -0.4641962 | -0.0004319 | 0.00093051 | 11.4572533 |
| chr12:76852527-77064746 | -0.4840784 | -0.0004773 | 0.00098602 | 11.4572533 |
| chr6:143157329-144194971 | -0.5038925 | -0.0005633 | 0.00111799 | 11.4572533 |
| chr7:129281946-131188950 | -0.5050909 | -0.0005009 | 0.0009918 | 11.4572533 |
| chr18:1-587750 | -0.52301 | -0.000756 | 0.00144557 | 11.4572533 |
| chr11:107086196-116175885 | -0.5517536 | -0.0006585 | 0.00119354 | 11.4572533 |
| chr7:156893473-158821424 | -0.5549579 | -0.0005754 | 0.00103692 | 11.4572533 |
| chr10:40300000-135374737 | -0.5584822 | -0.0004758 | 0.00085192 | 11.4572533 |
| chr12:97551177-99047626 | -0.5691787 | -0.0005842 | 0.00102643 | 11.4572533 |
| chr11:110631916-112776199 | -0.5710002 | -0.0006859 | 0.00120125 | 11.4572533 |
| chr14:35708407-36097605 | -0.5816034 | -0.0005157 | 0.0008867 | 11.4572533 |
| chr16:1-38200000 | -0.5852785 | -0.0006176 | 0.00105521 | 11.4572533 |
| chr20:20585029-20740450 | -0.6349983 | -0.0008609 | 0.00135569 | 11.4572533 |
| chr12:56419524-56488685 | -0.6387167 | -0.0006132 | 0.0009601 | 11.4572533 |
| chr12:56621627-57600529 | -0.6387167 | -0.0006132 | 0.0009601 | 11.4572533 |
| chr12:50987234-51048711 | -0.6398433 | -0.0005901 | 0.00092222 | 11.4572533 |
| chr12:35400000-132349534 | -0.6563379 | -0.0005876 | 0.00089535 | 11.4572533 |
| chr7:89924533-98997268 | -0.6645494 | -0.0007092 | 0.00106714 | 10.4028382 |
| chr8:38252951-38460772 | -0.6691616 | -0.001053 | 0.0015736 | 10.4028382 |
| chr20:1-27100000 | -0.6818861 | -0.0009139 | 0.00134031 | 10.4028382 |
| chr12:27342867-29425476 | -0.6876428 | -0.0007233 | 0.00105188 | 10.4028382 |
| chr13:104916365-104941384 | -0.6992566 | -0.0007965 | 0.00113906 | 10.4028382 |
| chr12:25189655-25352305 | -0.7005308 | -0.0007597 | 0.00108452 | 10.4028382 |
| chr10:89467202-90419015 | -0.7062195 | -0.0007164 | 0.00101439 | 10.4028382 |
| chr14:93528266-103946058 | -0.7109456 | -0.0006622 | 0.00093146 | 10.4028382 |
| chr14:35837522-37090214 | -0.7189251 | -0.0006381 | 0.00088751 | 10.4028382 |
| chr19:21788507-34401877 | -0.7352329 | -0.0007598 | 0.00103342 | 10.4028382 |
| chr14:50062686-50445987 | -0.7397515 | -0.0006671 | 0.0009018 | 10.4028382 |
| chr14:49113809-52311409 | -0.7422276 | -0.0006686 | 0.00090083 | 10.4028382 |
| chr4:1234177-1683843 | -0.7479452 | -0.0007258 | 0.00097037 | 10.4028382 |
| chrX:1-60000000 | -0.7479452 | -0.0007258 | 0.00097037 | 10.4028382 |
| chr12:38788913-42596599 | -0.7495694 | -0.0006737 | 0.00089879 | 10.4028382 |
| chr10:123223889-124739898 | -0.75371 | -0.0008367 | 0.00111013 | 10.4028382 |
| chr15:75011134-75564998 | -0.7571752 | -0.0007428 | 0.00098101 | 10.4028382 |
| chr10:122206456-122883255 | -0.7632871 | -0.0008469 | 0.00110955 | 10.4028382 |
| chr10:123214869-123367187 | -0.7632871 | -0.0008469 | 0.00110955 | 10.4028382 |
| chr14:80741860-106368585 | -0.774043 | -0.0007096 | 0.00091677 | 7.4508556 |
| chr7:92829306-132566935 | -0.7763619 | -0.0007761 | 0.00099968 | 7.4508556 |
| chr13:108477140-110084607 | -0.7768017 | -0.0008758 | 0.0011275 | 7.4508556 |
| chr13:109973420-114110898 | -0.7768017 | -0.0008758 | 0.0011275 | 7.4508556 |
| chr13:111767404-114142980 | -0.7768017 | -0.0008758 | 0.0011275 | 7.4508556 |
| chr13:112030830-113770959 | -0.7768017 | -0.0008758 | 0.0011275 | 7.4508556 |
| chr12:42034279-44588086 | -0.7773755 | -0.0007254 | 0.00093311 | 7.4508556 |
| chr19:15641747-15670750 | -0.7817297 | -0.0009459 | 0.00121005 | 7.4508556 |
| chr12:48421630-53099317 | -0.7829861 | -0.0006899 | 0.00088108 | 7.4508556 |
| chr12:44599181-47397048 | -0.7848609 | -0.0007172 | 0.00091377 | 7.4508556 |
| chr12:34188071-44391168 | -0.7963667 | -0.0006983 | 0.00087692 | 7.4508556 |
| chr5:1212750-1378766 | -0.7981315 | -0.0007462 | 0.00093494 | 7.4508556 |
| chr7:102988499-103837783 | -0.8089388 | -0.0008467 | 0.00104673 | 7.4508556 |
| chr11:104262627-109547776 | -0.8107626 | -0.0009698 | 0.0011962 | 7.4508556 |
| chr14:37128947-38971371 | -0.8115281 | -0.0007373 | 0.00090852 | 7.4508556 |
| chr20:14210829-15988895 | -0.8160765 | -0.0011206 | 0.00137319 | 7.4508556 |
| chr7:137042370-158819393 | -0.8170859 | -0.0008019 | 0.00098139 | 7.4508556 |
| chr6:149442557-149779188 | -0.8288159 | -0.0009453 | 0.00114053 | 7.4508556 |
| chr7:59100000-158821424 | -0.8482617 | -0.0007996 | 0.00094268 | 7.4508556 |
| chr4:71492582-75938920 | -0.8591653 | -0.0007918 | 0.00092154 | 7.4508556 |
| chr4:3220565-5553626 | -0.8646474 | -0.0008403 | 0.00097187 | 7.4508556 |
| chr17:39354620-40542083 | -0.8647376 | -0.0009792 | 0.00113233 | 7.4508556 |
| chr12:47446248-48387464 | -0.8647719 | -0.0007718 | 0.00089254 | 7.4508556 |
| chr11:130280899-134452384 | -0.8670652 | -0.0010587 | 0.00122105 | 7.4508556 |
| chr14:95740950-100105884 | -0.8774088 | -0.0008518 | 0.00097078 | 7.4508556 |
| chr7:144118814-148066271 | -0.8774395 | -0.0008764 | 0.00099886 | 7.4508556 |
| chr17:28881201-29652449 | -0.877642 | -0.0010036 | 0.00114355 | 7.4508556 |
| chr14:92049878-92765306 | -0.880296 | -0.0008595 | 0.00097635 | 7.4508556 |
| chr1:27351829-27460967 | -0.8810531 | -0.0009325 | 0.00105834 | 7.4508556 |
| chr14:15600000-106368585 | -0.8846342 | -0.0007169 | 0.00081041 | 7.4508556 |
| chr4:4349500-4427489 | -0.8883977 | -0.0008655 | 0.00097426 | 7.4508556 |
| chr4:73526461-75252649 | -0.897603 | -0.0008287 | 0.00092326 | 7.4508556 |
| chr14:52311662-54325595 | -0.9103266 | -0.0008142 | 0.00089438 | 7.4508556 |
| chr14:92773649-95250286 | -0.914688 | -0.0008912 | 0.00097432 | 7.4508556 |
| chr18:1-16100000 | -0.9147003 | -0.0011034 | 0.00120631 | 7.4508556 |
| chr5:1473801-6790134 | -0.9157002 | -0.0008533 | 0.00093182 | 7.4508556 |
| chr18:3200634-3858775 | -0.9166401 | -0.0012623 | 0.0013771 | 7.4508556 |
| chr1:26377344-27532551 | -0.923862 | -0.00098 | 0.00106081 | 7.4508556 |
| chr7 :8779703 8-89779004 | -0.9252993 | -0.0010171 | 0.00109924 | 7.4508556 |
| chr4:5577784-10295484 | -0.9285538 | -0.0009084 | 0.00097833 | 7.4508556 |
| chr17:24112056-24310787 | -0.94273 | -0.0010813 | 0.00114697 | 7.4508556 |
| chr7:65877239-79629882 | -0.9489197 | -0.0010012 | 0.00105513 | 7.4508556 |
| chr7:141592807-142264966 | -0.9630961 | -0.0009476 | 0.00098386 | 4.7635892 |
| chr7:115981465-116676953 | -0.9677616 | -0.0009944 | 0.00102752 | 4.7635892 |
| chr18:3240326-5937123 | -0.9720502 | -0.0013391 | 0.0013776 | 4.7635892 |
| chr14:54378476-55837783 | -0.9763001 | -0.0008863 | 0.00090782 | 4.7635892 |
| chr3:36286137-40063081 | -0.9817658 | -0.0009609 | 0.0009788 | 4.7635892 |
| chr17:26185485-27216066 | -0.9976164 | -0.0011051 | 0.00110775 | 4.7635892 |
| chr17:24936541-25468309 | -1.005183 | -0.0011442 | 0.00113828 | 4.7635892 |
| chr11:101433436-102134907 | -1.0126869 | -0.0012132 | 0.00119798 | 4.7635892 |
| chr18:13905410-14018535 | -1.0143458 | -0.0012254 | 0.0012081 | 4.7635892 |
| chr5:9224578-10673735 | -1.0202278 | -0.000923 | 0.00090474 | 4.7635892 |
| chr12:55201993-55269875 | -1.0351051 | -0.0009623 | 0.00092971 | 4.7635892 |
| chr5:174477192-180857866 | -1.0415768 | -0.0008836 | 0.00084829 | 4.7635892 |
| chr5:177541057-180857866 | -1.0424417 | -0.0008755 | 0.00083989 | 4.7635892 |
| chr12:53136012-55168448 | -1.043954 | -0.0009635 | 0.0009229 | 4.7635892 |
| chr5:176660805-180009206 | -1.06051 | -0.0009083 | 0.00085648 | 4.7635892 |
| chr17:22479313-22877776 | -1.0700316 | -0.0011922 | 0.00111418 | 4.7635892 |
| chr14:56307190-56415610 | -1.0734828 | -0.0009664 | 0.00090029 | 4.7635892 |
| chr11:82612034-85091467 | -1.0763629 | -0.0012653 | 0.00117558 | 4.7635892 |
| chr18:75796373-76117153 | -1.0782692 | -0.0013502 | 0.00125221 | 4.7635892 |
| chr19:10895846-11164554 | -1.0852356 | -0.0013043 | 0.00120182 | 4.7635892 |
| chr5:172591744-174888201 | -1.0923146 | -0.0009099 | 0.00083302 | 4.7635892 |
| chr11:1-1391954 | -1.1301839 | -0.0011754 | 0.00104002 | 3.27496757 |
| chr7:54899301-55275419 | -1.133896 | -0.0011154 | 0.00098365 | 3.27496757 |
| chr5:147238467-151765017 | -1.1607336 | -0.0009536 | 0.00082159 | 3.27496757 |
| chr18:32113759-32649538 | -1.1692999 | -0.0013982 | 0.00119574 | 3.27496757 |
| chr5:159922707-167623739 | -1.2122794 | -0.0009966 | 0.00082209 | 2.33926255 |
| chr4:104889590-106814504 | -1.2310621 | -0.0010559 | 0.00085772 | 2.33926255 |
| chr5:167651670-171366482 | -1.2382693 | -0.0010219 | 0.00082527 | 2.33926255 |
| chr5:36750649-37111693 | -1.241524 | -0.0010466 | 0.000843 | 2.33926255 |
| chr5:139008130-142795270 | -1.2518205 | -0.0010066 | 0.00080411 | 2.33926255 |
| chr5:155686345-159845035 | -1.2670465 | -0.001055 | 0.00083261 | 2.33926255 |
| chr5:152850499-154326721 | -1.2949012 | -0.0010781 | 0.00083258 | 2.33926255 |
| chr22:15272143-16594738 | -1.2961111 | -0.0015239 | 0.00117578 | 2.33926255 |
| chr5:143171919-147191453 | -1.299711 | -0.0010429 | 0.0008024 | 1.57988386 |
| chr5:39149695-41885012 | -1.3065881 | -0.001073 | 0.00082123 | 1.57988386 |
| chr5:43612947-44998256 | -1.30938 | -0.001067 | 0.00081492 | 1.57988386 |
| chr5:1-47700000 | -1.3275708 | -0.0010974 | 0.00082665 | 1.57988386 |
| chr10:129812260-135374737 | -1.3487054 | -0.0013325 | 0.00098798 | 1.57988386 |
| chr14:77311255-79682157 | -1.3608459 | -0.0013419 | 0.00098605 | 1.57988386 |
| chr13:31211674-31275009 | -1.3691428 | -0.0014998 | 0.00109545 | 1.57988386 |
| chr14:77945724-77992686 | -1.3814739 | -0.0013654 | 0.00098838 | 1.57988386 |
| chr5:45312870-49697231 | -1.3934592 | -0.0011313 | 0.00081186 | 1.57988386 |
| chr15:1-24740084 | -1.3975233 | -0.0018409 | 0.00131722 | 1.57988386 |
| chr2:241477619-242951149 | -1.4376724 | -0.0012804 | 0.00089059 | 1.07965964 |
| chr15:29020705-31945591 | -1.4401086 | -0.0018944 | 0.00131545 | 1.07965964 |
| chr10:128584013-129240925 | -1.4427769 | -0.0014177 | 0.00098262 | 1.07965964 |
| chr22:18689393-20139185 | -1.4587589 | -0.0016922 | 0.00116001 | 1.07965964 |
| chr22:19172385-19746441 | -1.4587589 | -0.0016922 | 0.00116001 | 1.07965964 |
| chr18:17749667-22797232 | -1.4600665 | -0.0017547 | 0.00120177 | 1.07965964 |
| chr22:20517661-21169423 | -1.4655849 | -0.0017015 | 0.001161 | 1.07965964 |
| chr4:76623381-79684447 | -1.4720549 | -0.0013265 | 0.00090111 | 1.07965964 |
| chr15:17000000-100338915 | -1.4737108 | -0.0013063 | 0.00088642 | 1.07965964 |
| chr13:16000000-114142980 | -1.4758197 | -0.0014491 | 0.00098186 | 1.07965964 |
| chr18:14720419-16909449 | -1.4758657 | -0.0017047 | 0.00115502 | 1.07965964 |
| chr4:54471680-55980061 | -1.4809675 | -0.0013721 | 0.00092646 | 1.07965964 |
| chr5:135493104-138988200 | -1.5069862 | -0.0012226 | 0.0008113 | 1.07965964 |
| chr15:28193434-29022600 | -1.5199031 | -0.0020041 | 0.00131859 | 1.07965964 |
| chr22:45488286-49691432 | -1.5490682 | -0.0017757 | 0.00114628 | 1.07965964 |
| chr11:85633463-87710586 | -1.5536148 | -0.0017586 | 0.00113195 | 1.07965964 |
| chr18:16100000-76117153 | -1.5536994 | -0.0017908 | 0.0011526 | 1.07965964 |
| chr2:93300000-242951149 | -1.55383 | -0.0011384 | 0.00073266 | 1.07965964 |
| chr11:92342437-95765250 | -1.5553948 | -0.0017904 | 0.00115111 | 1.07965964 |
| chr2:164158152-168812365 | -1.5591787 | -0.0013054 | 0.00083723 | 1.07965964 |
| chr13:27774389-30804409 | -1.5678278 | -0.00171 | 0.00109071 | 1.07965964 |
| chr4:19864333-22430289 | -1.5812074 | -0.0015102 | 0.00095506 | 0.47635892 |
| chr4:1-50700000 | -1.5935275 | -0.0013894 | 0.00087188 | 0.47635892 |
| chr3:66644057-68118027 | -1.5965089 | -0.0016332 | 0.00102296 | 0.47635892 |
| chr18:44506813-45038631 | -1.6070326 | -0.0019429 | 0.00120901 | 0.47635892 |
| chr1:3756302-6867390 | -1.6213378 | -0.0017371 | 0.00107143 | 0.47635892 |
| chrl5:31945721-37862331 | -1.6301753 | -0.0015391 | 0.00094413 | 0.47635892 |
| chr14:57736586-58085299 | -1.639074 | -0.001512 | 0.00092246 | 0.47635892 |
| chr10:119032598-121691235 | -1.6502918 | -0.0015852 | 0.00096059 | 0.47635892 |
| chr2:173927807-175001974 | -1.6643745 | -0.0014083 | 0.00084617 | 0.47635892 |
| chrX:60000000-154913754 | -1.6995564 | -0.0013618 | 0.00080128 | 0.47635892 |
| chr15:65899096-66370691 | -1.6996425 | -0.0016356 | 0.00096234 | 0.47635892 |
| chr10:118946990-119027085 | -1.709839 | -0.0016332 | 0.00095519 | 0.47635892 |
| chr5:18240864-21565169 | -1.7116989 | -0.001532 | 0.00089502 | 0.47635892 |
| chr3:1-91700000 | -1.7177989 | -0.0014055 | 0.00081822 | 0.47635892 |
| chr3:70107050-74383584 | -1.717818 | -0.001772 | 0.00103152 | 0.47635892 |
| chr11:87881006-92269284 | -1.723718 | -0.0019504 | 0.00113148 | 0.47635892 |
| chr22:22437934-22507511 | -1.7405956 | -0.0020288 | 0.00116559 | 0.47635892 |
| chr22:23139372-23249329 | -1.7405956 | -0.0020288 | 0.00116559 | 0.47635892 |
| chr14:65275722-67085224 | -1.7431414 | -0.0016084 | 0.00092267 | 0.47635892 |
| chr4:10979549-15266111 | -1.7458908 | -0.0016324 | 0.00093502 | 0 |
| chr14:69312342-69568836 | -1.7483944 | -0.0016704 | 0.00095538 | 0 |
| chr19:1-28500000 | -1.759343 | -0.0019648 | 0.00111677 | 0 |
| chr4:15313739-17632477 | -1.7650118 | -0.0016589 | 0.00093991 | 0 |
| chr13:46362859-48209064 | -1.7666444 | -0.0019393 | 0.00109773 | 0 |
| chr18:46172638-49935241 | -1.7718409 | -0.0021514 | 0.00121419 | 0 |
| chrl7:36989609-37319012 | -1.7767687 | -0.0020312 | 0.00114318 | 0 |
| chr4:23402742-26636101 | -1.7966181 | -0.0017308 | 0.00096334 | 0 |
| chr15:61583863-61913200 | -1.805715 | -0.0017166 | 0.00095062 | 0 |
| chr2:159800558-163403274 | -1.8397898 | -0.0015116 | 0.0008216 | 0 |
| chr15:57184612-61461128 | -1.8449377 | -0.0017516 | 0.00094942 | 0 |
| chr4:13238819-37565122 | -1.8551281 | -0.0016906 | 0.00091133 | 0 |
| chr22:37740208-38258806 | -1.8582233 | -0.0020814 | 0.00112009 | 0 |
| chr15:56490060-57176541 | -1.8613536 | -0.0017678 | 0.00094975 | 0 |
| chr22:11800000-49691432 | -1.8626608 | -0.0020146 | 0.00108159 | 0 |
| chr15:37880223-38115089 | -1.8667224 | -0.0017634 | 0.00094467 | 0 |
| chr17:37319013-37988602 | -1.9065006 | -0.002186 | 0.00114658 | 0 |
| chr3:58626894-61524607 | -1.9087834 | -0.0019337 | 0.00101307 | 0 |
| chr4:162524501-164492534 | -1.9286324 | -0.0017269 | 0.00089541 | 0 |
| chr22:36048950-38681384 | -1.9309272 | -0.0021579 | 0.00111754 | 0 |
| chr4:147085600-153449318 | -1.9338501 | -0.0018038 | 0.00093276 | 0 |
| chr4:30331135-30753569 | -1.9434351 | -0.0018451 | 0.0009494 | 0 |
| chr2:204533830-206266883 | -1.9448685 | -0.0016747 | 0.00086109 | 0 |
| chr15:35140533-43473382 | -1.9470978 | -0.0018123 | 0.00093078 | 0 |
| chr12:20728852-21227330 | -1.955489 | -0.0020395 | 0.00104297 | 0 |
| chr4:143168636-143603331 | -1.9801697 | -0.0018376 | 0.00092799 | 0 |
| chr15:49136093-51017946 | -1.9810067 | -0.0018728 | 0.00094539 | 0 |
| chr4:83634873-83961360 | -1.9839594 | -0.0018106 | 0.00091262 | 0 |
| chr4:91089383-93486891 | -2.0110536 | -0.001843 | 0.00091645 | 0 |
| chr4:37121738-38804810 | -2.0254757 | -0.0019023 | 0.0009392 | 0 |
| chr22:31596415-33790650 | -2.0365988 | -0.002298 | 0.00112837 | 0 |
| chr17:20027154-20122158 | -2.0366542 | -0.0022049 | 0.00108263 | 0 |
| chr15:51593230-55368004 | -2.0385425 | -0.0019395 | 0.00095142 | 0 |
| chr4:112023722-118112513 | -2.0451326 | -0.001877 | 0.0009178 | 0 |
| chr4:114219937-114355024 | -2.0451326 | -0.001877 | 0.0009178 | 0 |
| chr4:79691766-80079606 | -2.0551421 | -0.0019237 | 0.00093605 | 0 |
| chr4:50700000-191273063 | -2.0627651 | -0.0016651 | 0.00080721 | 0 |
| chr12:18393150-19784941 | -2.0690252 | -0.0021768 | 0.0010521 | 0 |
| chr4:41057559-42353879 | -2.0740921 | -0.0019463 | 0.00093838 | 0 |
| chr4:164612456-170167997 | -2.074466 | -0.0018775 | 0.00090506 | 0 |
| chr2:156889194-159797412 | -2.0786615 | -0.0017358 | 0.00083505 | 0 |
| chr17:18837023-19933105 | -2.0829317 | -0.0022834 | 0.00109622 | 0 |
| chr3:43362938-44130419 | -2.1007513 | -0.0020688 | 0.00098479 | 0 |
| chr4:170251982-170915637 | -2.1127349 | -0.0019197 | 0.00090862 | 0 |
| chr2:154436672-155018734 | -2.1201817 | -0.0017863 | 0.00084252 | 0 |
| chr2:138479322-143365272 | -2.1736868 | -0.0018192 | 0.00083692 | 0 |
| chr13:1-40829685 | -2.1809576 | -0.0022405 | 0.0010273 | 0 |
| chr4:155375138-160500747 | -2.2049435 | -0.0020318 | 0.00092148 | 0 |
| chr4:151218876-154929678 | -2.2057694 | -0.0020738 | 0.00094017 | 0 |
| chr19:8920808-8939190 | -2.2155813 | -0.0026582 | 0.00119977 | 0 |
| chr2:135029078-135167772 | -2.2472858 | -0.0019035 | 0.000847 | 0 |
| chr12:1-35400000 | -2.2573634 | -0.0021071 | 0.00093345 | 0 |
| chr12:8398512-8740196 | -2.2743612 | -0.0023051 | 0.00101354 | 0 |
| chr13:27611014-27767472 | -2.2874149 | -0.0025275 | 0.00110496 | 0 |
| chr5:130522776-135427403 | -2.330097 | -0.0018754 | 0.00080486 | 0 |
| chr12:1-1311104 | -2.4001165 | -0.0025112 | 0.00104628 | 0 |
| chr5:47700000-180857866 | -2.428013 | -0.0018486 | 0.00076135 | 0 |
| chr5:49730236-50173926 | -2.4594689 | -0.0020061 | 0.00081564 | 0 |
| chr4:186684565-191273063 | -2.4686529 | -0.002398 | 0.00097139 | 0 |
| chr4:174326479-176135905 | -2.4891423 | -0.0022684 | 0.00091133 | 0 |
| chr17:1-22200000 | -2.5049077 | -0.0026399 | 0.00105389 | 0 |
| chr19:1-526082 | -2.5327771 | -0.0030374 | 0.00119924 | 0 |
| chr19:1818912-2004994 | -2.5327771 | -0.0030374 | 0.00119924 | 0 |
| chr17:7471230-7717938 | -2.5333603 | -0.0026308 | 0.00103848 | 0 |
| chr13:1-23902184 | -2.6579671 | -0.002832 | 0.00106546 | 0 |
| chr19:7321723-7480918 | -2.6725998 | -0.0032092 | 0.00120078 | 0 |
| chr4:175486008-184028292 | -2.6937691 | -0.0024622 | 0.00091402 | 0 |
| chr4:176791083-177486490 | -2.7624226 | -0.0025336 | 0.00091715 | 0 |
| chr5:125787000-126200608 | -2.7762469 | -0.0023794 | 0.00085705 | 0 |
| chr17:10675416-12635879 | -2.8303527 | -0.003032 | 0.00107125 | 0 |
| chr12:10732932-10778896 | -2.874595 | -0.0029599 | 0.00102969 | 0 |
| chr5:127447382-129549383 | -2.9037578 | -0.0024592 | 0.0008469 | 0 |
| chr5:128458341-128477620 | -2.9255023 | -0.0024909 | 0.00085146 | 0 |
| chr12:11410696-12118386 | -2.9631474 | -0.0030496 | 0.00102918 | 0 |
| chr5:113725565-115180304 | -2.9995395 | -0.0026383 | 0.00087958 | 0 |
| chr5:121675719-127022221 | -3.0056697 | -0.0025526 | 0.00084928 | 0 |
| chr5:115193714-121517156 | -3.0190448 | -0.0025876 | 0.00085708 | 0 |
| chr8:1-45200000 | -3.0441332 | -0.0029888 | 0.00098183 | 0 |
| chr5:68425638-72913540 | -3.2283443 | -0.0027177 | 0.00084182 | 0 |
| chr5:111507433-112958878 | -3.2686529 | -0.0028497 | 0.00087181 | 0 |
| chr5:85837489-133480433 | -3.3397323 | -0.0027278 | 0.00081679 | 0 |
| chr8:29285985-30473918 | -3.3577614 | -0.0039464 | 0.0011753 | 0 |
| chr5:109877429-111137815 | -3.3616543 | -0.0029395 | 0.00087441 | 0 |
| chr5:68425638-71536822 | -3.3961629 | -0.0028831 | 0.00084891 | 0 |
| chr5:73958991-76396789 | -3.4122036 | -0.0029714 | 0.00087082 | 0 |
| chr5:106744250-109231328 | -3.4575266 | -0.0030235 | 0.00087448 | 0 |
| chr5:81303630-82685333 | -3.5895262 | -0.0030967 | 0.0008627 | 0 |
| chr8:2053441-6259545 | -3.5970802 | -0.0041789 | 0.00116175 | 0 |
| chr5:76408288-81082828 | -3.6041408 | -0.0031133 | 0.00086382 | 0 |
| chr8:11575198-11708771 | -3.6044478 | -0.0041609 | 0.00115437 | 0 |
| chr8:22125332-30139123 | -3.6343855 | -0.0040338 | 0.00110989 | 0 |
| chr5:66513872-67633403 | -3.6744984 | -0.0032384 | 0.00088132 | 0 |
| chr5:82803339-90714877 | -3.7074602 | -0.0031969 | 0.00086228 | 0 |
| chr5:63838208-66501179 | -3.712216 | -0.0032613 | 0.00087854 | 0 |
| chr5:101597589-102642260 | -3.7481316 | -0.0032872 | 0.00087702 | 0 |
| chr5:102912456-102926389 | -3.7481316 | -0.0032872 | 0.00087702 | 0 |
| chr5:102912456-102926389 | -3.7481316 | -0.0032872 | 0.00087702 | 0 |
| chr5:52119531-58183162 | -3.7549358 | -0.0032484 | 0.00086509 | 0 |
| chr5:63292034-63701397 | -3.7607731 | -0.0033079 | 0.00087959 | 0 |
| chr5:58300629-62108926 | -3.83131 | -0.0033379 | 0.00087122 | 0 |
| chr5:98132900-102642260 | -3.8387521 | -0.0033423 | 0.00087068 | 0 |
| chr5:56146657-56594666 | -3.8495857 | -0.0033901 | 0.00088063 | 0 |
| chr5:58300629-59320301 | -3.8873655 | -0.003392 | 0.00087256 | 0 |
| chr5:57754754-59053198 | -3.8873655 | -0.003392 | 0.00087256 | 0 |
| chr5:92250850-96544700 | -3.9489415 | -0.0034033 | 0.00086183 | 0 |

**Table 6. Univariate analyses of 491 DNA segments for PFS in 87 patients with advanced HR+/HER2- breast cancer treated with CDK4/6 inhibitors and endocrine therapy.**

| **Segment** | **n** | **n_event** | **HR** | **Lower_HR** | **UpperHR** | **pvalue** |
|---|---|---|---|---|---|---|
| chr1:110339388-119426489 | 87 | 49 | 0.61729139 | 0.46885328 | 0.81272475 | 0.00058691 |
| chr1:114889793-115149999 | 87 | 49 | 0.6199867 | 0.47118196 | 0.81578572 | 0.00064025 |
| chr1:118282114-118807006 | 87 | 49 | 0.70879712 | 0.52051839 | 0.96517888 | 0.02889213 |
| chr1:119996566-120303234 | 87 | 49 | 0.76971137 | 0.53988135 | 1.09738111 | 0.14805624 |
| chr1:148661965-149063439 | 87 | 49 | 1.10286053 | 0.84013924 | 1.44773782 | 0.48065512 |
| chr1:151026302-152973244 | 87 | 49 | 1.13949444 | 0.87802446 | 1.47882848 | 0.32616081 |
| chr1:158317017-159953843 | 87 | 49 | 1.16067112 | 0.87945269 | 1.53181344 | 0.29255162 |
| chr1:162199047-163197517 | 87 | 49 | 1.17273846 | 0.88940564 | 1.54633098 | 0.25876636 |
| chr1:163619952-164752515 | 87 | 49 | 1.16611756 | 0.88581119 | 1.53512416 | 0.27326615 |
| chr1:166729757-167532460 | 87 | 49 | 1.08499997 | 0.81685476 | 1.44116799 | 0.57326036 |
| chr1:168438419-168880930 | 87 | 49 | 1.0471868 | 0.78668331 | 1.3939538 | 0.75205276 |
| chr1:169549478-170484405 | 87 | 49 | 1.0471868 | 0.78668331 | 1.3939538 | 0.75205276 |
| chr1:174611641-175371076 | 87 | 49 | 1.04852789 | 0.78932623 | 1.39284709 | 0.74361106 |
| chr1:201678483-203358272 | 87 | 49 | 1.19477942 | 0.91050789 | 1.56780394 | 0.19924868 |
| chr1:223876038-247249719 | 87 | 49 | 1.04929423 | 0.79006353 | 1.39358209 | 0.7396208 |
| chr1:241364021-247249719 | 87 | 49 | 1.03819062 | 0.78163384 | 1.3789574 | 0.79579403 |
| chr1:26377344-27532551 | 87 | 49 | 0.91919159 | 0.69834575 | 1.20987802 | 0.54782783 |
| chr1:27351829-27460967 | 87 | 49 | 0.91919159 | 0.69834575 | 1.20987802 | 0.54782783 |
| chr1:3756302-6867390 | 87 | 49 | 0.86030528 | 0.66056778 | 1.12043791 | 0.264295 |
| chr1:39907605-40263248 | 87 | 49 | 1.08922327 | 0.81086128 | 1.46314465 | 0.57031551 |
| chr1:41717036-43943775 | 87 | 49 | 1.09059504 | 0.80419844 | 1.47898514 | 0.57686721 |
| chr1:43945805-44960161 | 87 | 49 | 1.07657443 | 0.79618028 | 1.45571616 | 0.63171838 |
| chr1:46541957-46555032 | 87 | 49 | 1.07657443 | 0.79618028 | 1.45571616 | 0.63171838 |
| chr1:58658784-60221344 | 87 | 49 | 1.0958222 | 0.80429224 | 1.49302236 | 0.5620153 |
| chr1:71284749-74440273 | 87 | 49 | 1.06592203 | 0.7910904 | 1.43623255 | 0.67476106 |
| chr10:101955-12088176 | 87 | 49 | 1.26953224 | 0.89129138 | 1.80828868 | 0.18606624 |
| chr10:1-1042949 | 87 | 49 | 1.32651558 | 0.93254123 | 1.8869338 | 0.11606252 |
| chr10:118946990-119027085 | 87 | 49 | 0.6571099 | 0.50224174 | 0.85973225 | 0.00219798 |
| chr10:119032598-121691235 | 87 | 49 | 0.64533539 | 0.49442209 | 0.84231221 | 0.00127038 |
| chr10:122206456-122883255 | 87 | 49 | 0.64223708 | 0.49145086 | 0.8392873 | 0.00118192 |
| chr10:123214869-123367187 | 87 | 49 | 0.64223708 | 0.49145086 | 0.8392873 | 0.00118192 |
| chr10:123223889-124739898 | 87 | 49 | 0.64223708 | 0.49145086 | 0.8392873 | 0.00118192 |
| chr10:12431738-17283687 | 87 | 49 | 1.12071754 | 0.79087888 | 1.58811652 | 0.52164217 |
| chr10:128584013-129240925 | 87 | 49 | 0.63606796 | 0.48313272 | 0.83741471 | 0.00126188 |
| chr10:129812260-135374737 | 87 | 49 | 0.6349776 | 0.48224908 | 0.83607531 | 0.00121479 |
| chr10:170643-1769670 | 87 | 49 | 1.32651558 | 0.93254123 | 1.8869338 | 0.11606252 |
| chr10:17311250-19896508 | 87 | 49 | 1.08498104 | 0.76990273 | 1.52900335 | 0.64122258 |
| chr10:19022250-42889659 | 87 | 49 | 0.98661304 | 0.71501917 | 1.3613695 | 0.93461275 |
| chr10:20145174-20609292 | 87 | 49 | 1.10707633 | 0.78601309 | 1.55928446 | 0.56049774 |
| chr10:21110098-22746531 | 87 | 49 | 1.10707633 | 0.78601309 | 1.55928446 | 0.56049774 |
| chr10:22863772-24876778 | 87 | 49 | 1.10707633 | 0.78601309 | 1.55928446 | 0.56049774 |
| chr10:24604620-28074753 | 87 | 49 | 0.97762628 | 0.70181879 | 1.36182323 | 0.89355741 |
| chr10:28141103-31360860 | 87 | 49 | 0.97694936 | 0.7031967 | 1.35727321 | 0.88943975 |
| chr10:3099740-3205003 | 87 | 49 | 1.3144386 | 0.92305439 | 1.87177357 | 0.12951771 |
| chr10:31648148-32903498 | 87 | 49 | 0.93695554 | 0.68046826 | 1.29011996 | 0.6898707 |
| chr10:35175415-37212810 | 87 | 49 | 0.92732189 | 0.66640992 | 1.29038578 | 0.6544353 |
| chr10:3811260-5139878 | 87 | 49 | 1.27739586 | 0.89645795 | 1.82020826 | 0.17541518 |
| chr10:3811260-6662244 | 87 | 49 | 1.28524734 | 0.89787496 | 1.83974471 | 0.17027853 |
| chr10:52313829-53768264 | 87 | 49 | 0.89382976 | 0.65717162 | 1.21571231 | 0.47446114 |
| chr10:54201467-57779853 | 87 | 49 | 0.8628509 | 0.63285116 | 1.17644042 | 0.35101207 |
| chr10:61909390-66364553 | 87 | 49 | 0.8544424 | 0.61267088 | 1.19162153 | 0.35396617 |
| chr10:7244255-12332593 | 87 | 49 | 1.18302357 | 0.83471043 | 1.67668296 | 0.34487114 |
| chr10:73394126-73443318 | 87 | 49 | 1.02380573 | 0.69937098 | 1.49874416 | 0.9036942 |
| chr10:74560456-82020637 | 87 | 49 | 0.99732803 | 0.6838262 | 1.45455557 | 0.98891306 |
| chr10:75957348-77987139 | 87 | 49 | 0.94198862 | 0.65019229 | 1.36473868 | 0.75203852 |
| chr10:89467202-90419015 | 87 | 49 | 0.91565689 | 0.64160201 | 1.30677199 | 0.62728201 |
| chr11:101433436-102134907 | 87 | 49 | 0.89808255 | 0.63416072 | 1.27184205 | 0.54485933 |
| chr11:104262627-109547776 | 87 | 49 | 0.88755768 | 0.62690934 | 1.25657505 | 0.50130419 |
| chr11:107086196-116175885 | 87 | 49 | 0.89442419 | 0.6350278 | 1.25977892 | 0.52316778 |
| chr11:110631916-112776199 | 87 | 49 | 0.88595766 | 0.632388 | 1.24120157 | 0.4815075 |
| chr11:112785528-114880322 | 87 | 49 | 0.92384615 | 0.65827158 | 1.29656472 | 0.64691173 |
| chr11:1-1391954 | 87 | 49 | 0.85159455 | 0.64007166 | 1.13301887 | 0.27015205 |
| chr11:116803699-119605859 | 87 | 49 | 0.92384615 | 0.65827158 | 1.29656472 | 0.64691173 |
| chr11:130280899-134452384 | 87 | 49 | 0.9280117 | 0.68459184 | 1.25798419 | 0.63028245 |
| chr11:26341787-27574592 | 87 | 49 | 0.79407565 | 0.59778607 | 1.05481906 | 0.11147967 |
| chr11 :32027116-37799354 | 87 | 49 | 0.96734686 | 0.68520746 | 1.3656593 | 0.85033537 |
| chr11:42387077-47453403 | 87 | 49 | 1.02695222 | 0.73779096 | 1.42944399 | 0.87474991 |
| chr11:55503467-56066044 | 87 | 49 | 0.89338222 | 0.6504056 | 1.22712935 | 0.4863407 |
| chr11:57344931-58658339 | 87 | 49 | 0.81405219 | 0.64610791 | 1.02565059 | 0.08096312 |
| chr11 :57861150-58074632 | 87 | 49 | 0.81716803 | 0.64618288 | 1.03339722 | 0.09185461 |
| chr11:68230745-68388280 | 87 | 49 | 1.00140986 | 0.73073368 | 1.37234908 | 0.9930083 |
| chr11:69588488-69842871 | 87 | 49 | 1.18017249 | 0.88674872 | 1.57068973 | 0.25601746 |
| chr11:73357303-74035012 | 87 | 49 | 1.2469068 | 0.92672476 | 1.67771126 | 0.14501293 |
| chr11:74085123-74337880 | 87 | 49 | 1.13780639 | 0.83981448 | 1.54153494 | 0.40470889 |
| chr11:74091491-74180816 | 87 | 49 | 1.14628786 | 0.84619445 | 1.55280603 | 0.37800183 |
| chr11:76699529-78005085 | 87 | 49 | 1.0023608 | 0.75440659 | 1.33181122 | 0.98702464 |
| chr11:76710709-85146692 | 87 | 49 | 0.87661178 | 0.64514918 | 1.19111709 | 0.39984799 |
| chr11:82612034-85091467 | 87 | 49 | 0.78899271 | 0.58174473 | 1.07007328 | 0.1274214 |
| chr11:85633463-87710586 | 87 | 49 | 0.83763031 | 0.61783908 | 1.1356105 | 0.25386809 |
| chr11:87881006-92269284 | 87 | 49 | 0.92078408 | 0.67285326 | 1.26007165 | 0.60610614 |
| chr11:92342437-95765250 | 87 | 49 | 0.99265919 | 0.72173906 | 1.36527498 | 0.96386169 |
| chr12: 10732932-10778896 | 87 | 49 | 0.87036206 | 0.64336646 | 1.17744732 | 0.36784237 |
| chr12:1-1311104 | 87 | 49 | 0.827865 | 0.61030037 | 1.12298876 | 0.22462232 |
| chr12:11410696-12118386 | 87 | 49 | 0.78903825 | 0.59092778 | 1.05356589 | 0.10822337 |
| chr12:131913408-132349534 | 87 | 49 | 0.74492326 | 0.54886227 | 1.01101988 | 0.05880661 |
| chr12:18393150-19784941 | 87 | 49 | 0.97042775 | 0.70488903 | 1.33599757 | 0.85398731 |
| chr12:20728852-21227330 | 87 | 49 | 0.89190027 | 0.63747813 | 1.24786412 | 0.50435331 |
| chr12:25189655-25352305 | 87 | 49 | 1.06155824 | 0.78179866 | 1.44142725 | 0.7018993 |
| chr12:27342867-29425476 | 87 | 49 | 1.0375137 | 0.75863687 | 1.41890636 | 0.81765415 |
| chr12:29425476-31105002 | 87 | 49 | 0.97819553 | 0.70276114 | 1.36158141 | 0.89604311 |
| chr12:30999223-32594050 | 87 | 49 | 0.96969971 | 0.6942395 | 1.35445694 | 0.8567877 |
| chr12:33156845-33480710 | 87 | 49 | 0.96969971 | 0.6942395 | 1.35445694 | 0.8567877 |
| chr12:34188071-44391168 | 87 | 49 | 0.77500492 | 0.56879741 | 1.05596935 | 0.10632826 |
| chr12:3 8788913 -425 96599 | 87 | 49 | 0.79747364 | 0.60300251 | 1.0546626 | 0.11255917 |
| chr12:42034279-44588086 | 87 | 49 | 0.80409382 | 0.62994122 | 1.02639238 | 0.07998217 |
| chr12:44599181-47397048 | 87 | 49 | 0.86775932 | 0.66573622 | 1.13108798 | 0.29418483 |
| chr12:47446248-48387464 | 87 | 49 | 0.83711643 | 0.60892864 | 1.1508145 | 0.27355907 |
| chr12:48421630-53099317 | 87 | 49 | 0.89278129 | 0.64115174 | 1.24316661 | 0.5019608 |
| chr12:50987234-51048711 | 87 | 49 | 0.96332849 | 0.67815402 | 1.36842331 | 0.83475365 |
| chr12:53136012-55168448 | 87 | 49 | 0.81685692 | 0.57968039 | 1.15107434 | 0.24769226 |
| chr12:55201993-55269875 | 87 | 49 | 0.81567857 | 0.57878401 | 1.14953336 | 0.24447697 |
| chr12:56419524-56488685 | 87 | 49 | 0.84969715 | 0.60054058 | 1.20222558 | 0.35765724 |
| chr12:56621627-57600529 | 87 | 49 | 0.84969715 | 0.60054058 | 1.20222558 | 0.35765724 |
| chr12:63329154-63614508 | 87 | 49 | 0.97190807 | 0.68996487 | 1.36906287 | 0.870518 |
| chr12:64149851-66323376 | 87 | 49 | 1.11278962 | 0.78220499 | 1.58308979 | 0.55237669 |
| chr12:64461446-64607139 | 87 | 49 | 1.11278962 | 0.78220499 | 1.58308979 | 0.55237669 |
| chr12:67440273-67566002 | 87 | 49 | 1.11309452 | 0.78502711 | 1.57826323 | 0.54757187 |
| chr12:68249634-68327233 | 87 | 49 | 1.11309452 | 0.78502711 | 1.57826323 | 0.54757187 |
| chr12:76852527-77064746 | 87 | 49 | 0.93374481 | 0.65764422 | 1.32576149 | 0.70150156 |
| chr12 :85072329-85674601 | 87 | 49 | 0.92067002 | 0.64777047 | 1.30853956 | 0.64494865 |
| chr12:94480961-96479233 | 87 | 49 | 0.82169261 | 0.58683035 | 1.15055185 | 0.25285807 |
| chr12:95089777-95350380 | 87 | 49 | 0.83782144 | 0.61650359 | 1.13858991 | 0.25820295 |
| chr12:97551177-99047626 | 87 | 49 | 0.81825218 | 0.60576254 | 1.10527903 | 0.19104703 |
| chr13: 104916365-104941384 | 87 | 49 | 0.89108794 | 0.66089717 | 1.20145424 | 0.44948675 |
| chr13:106620319-107317084 | 87 | 49 | 0.92437495 | 0.6810017 | 1.25472383 | 0.61396714 |
| chr13:108477140-110084607 | 87 | 49 | 0.92437495 | 0.6810017 | 1.25472383 | 0.61396714 |
| chr13: 109973420-114110898 | 87 | 49 | 0.92437495 | 0.6810017 | 1.25472383 | 0.61396714 |
| chr13: 111767404-114142980 | 87 | 49 | 0.92437495 | 0.6810017 | 1.25472383 | 0.61396714 |
| chr13: 112030830-113770959 | 87 | 49 | 0.92437495 | 0.6810017 | 1.25472383 | 0.61396714 |
| chr13:1-23902184 | 87 | 49 | 0.68144304 | 0.52407284 | 0.88606884 | 0.00419865 |
| chr13:1-40829685 | 87 | 49 | 0.72923809 | 0.55418586 | 0.95958456 | 0.02416294 |
| chr13:27611014-27767472 | 87 | 49 | 0.68891906 | 0.51635144 | 0.91915977 | 0.01131035 |
| chr13 :27774389-30804409 | 87 | 49 | 0.78495769 | 0.58272231 | 1.05737941 | 0.11118118 |
| chr13 :31211674-31275009 | 87 | 49 | 0.816109 | 0.61133465 | 1.08947513 | 0.16802147 |
| chr13 :46362859-48209064 | 87 | 49 | 0.7950012 | 0.62512543 | 1.01104013 | 0.06142211 |
| chr13 :72212826-74986423 | 87 | 49 | 0.94308927 | 0.72327637 | 1.22970612 | 0.66518513 |
| chr13:89500014-93206506 | 87 | 49 | 0.92302215 | 0.68411107 | 1.2453678 | 0.60018135 |
| chr13 :92308911-94031607 | 87 | 49 | 0.92302215 | 0.68411107 | 1.2453678 | 0.60018135 |
| chr14:1-23145193 | 87 | 49 | 0.93474445 | 0.67032346 | 1.303471 | 0.69080225 |
| chr14: 1-29140968 | 87 | 49 | 0.90411251 | 0.6496113 | 1.25832083 | 0.55008089 |
| chr14:21961377-23608756 | 87 | 49 | 0.89999882 | 0.64869639 | 1.24865484 | 0.52824446 |
| chr14:23609961-31698685 | 87 | 49 | 0.88306242 | 0.63406407 | 1.22984297 | 0.46183552 |
| chr14:31868274-34078694 | 87 | 49 | 0.94745977 | 0.67884206 | 1.32236948 | 0.751029 |
| chr14:34100051-35410919 | 87 | 49 | 0.94797834 | 0.68357109 | 1.31465907 | 0.74881056 |
| chr14:35708407-36097605 | 87 | 49 | 0.94831362 | 0.69092289 | 1.30159057 | 0.74254922 |
| chr14:35837522-37090214 | 87 | 49 | 0.90548386 | 0.65786006 | 1.24631525 | 0.54245179 |
| chr14:37128947-38971371 | 87 | 49 | 0.87228934 | 0.62867662 | 1.2103022 | 0.41353244 |
| chr14:49113809-52311409 | 87 | 49 | 0.76048935 | 0.5562191 | 1.03977739 | 0.08624383 |
| chr14:50062686-50445987 | 87 | 49 | 0.76595989 | 0.55944742 | 1.04870365 | 0.09625221 |
| chr14:52311662-54325595 | 87 | 49 | 0.67484707 | 0.50227553 | 0.90671064 | 0.00905752 |
| chr14:54378476-55837783 | 87 | 49 | 0.71158376 | 0.54279739 | 0.93285534 | 0.01377415 |
| chr14:56307190-56415610 | 87 | 49 | 0.72717919 | 0.55348753 | 0.95537757 | 0.02215083 |
| chr14:57736586-58085299 | 87 | 49 | 0.65259559 | 0.50092954 | 0.85018144 | 0.00156316 |
| chr14:65275722-67085224 | 87 | 49 | 0.68092697 | 0.52210942 | 0.88805436 | 0.00456632 |
| chr14:69312342-69568836 | 87 | 49 | 0.68727207 | 0.52724866 | 0.89586361 | 0.00555229 |
| chr14:77311255-79682157 | 87 | 49 | 0.70397875 | 0.53472769 | 0.92680086 | 0.01235765 |
| chr14:77945724-77992686 | 87 | 49 | 0.70397875 | 0.53472769 | 0.92680086 | 0.01235765 |
| chr14:80741860-106368585 | 87 | 49 | 0.83304657 | 0.61791017 | 1.12308655 | 0.23076071 |
| chr14 :92049878-92765306 | 87 | 49 | 0.86663284 | 0.63524423 | 1.18230507 | 0.36640304 |
| chr14 :92773649-95250286 | 87 | 49 | 0.85918499 | 0.62914568 | 1.17333532 | 0.33979312 |
| chr14:93528266-103946058 | 87 | 49 | 0.84682958 | 0.62350256 | 1.15014818 | 0.28715795 |
| chr14:95740950-100105884 | 87 | 49 | 0.84561626 | 0.62310474 | 1.14758695 | 0.28176929 |
| chr15:1-24740084 | 87 | 49 | 0.97337649 | 0.7093518 | 1.33567264 | 0.86725494 |
| chr15:28193434-29022600 | 87 | 49 | 1.01129503 | 0.74705105 | 1.36900636 | 0.94205454 |
| chr15:29020705-31945591 | 87 | 49 | 1.01129503 | 0.74705105 | 1.36900636 | 0.94205454 |
| chrl5:31945721-37862331 | 87 | 49 | 1.05812113 | 0.77304256 | 1.44832948 | 0.72429033 |
| chr15:35140533-43473382 | 87 | 49 | 1.12444463 | 0.81197471 | 1.55716146 | 0.48013748 |
| chr15:37880223-38115089 | 87 | 49 | 1.0928151 | 0.79585511 | 1.50058072 | 0.58327529 |
| chr15:49136093-51017946 | 87 | 49 | 1.14466269 | 0.79950137 | 1.63883731 | 0.46058305 |
| chr15:51593230-55368004 | 87 | 49 | 1.22375361 | 0.83439817 | 1.79479407 | 0.30141309 |
| chr15:56490060-57176541 | 87 | 49 | 1.24759973 | 0.84573381 | 1.84041959 | 0.26473442 |
| chr15:57184612-61461128 | 87 | 49 | 1.24759973 | 0.84573381 | 1.84041959 | 0.26473442 |
| chr15:61583863-61913200 | 87 | 49 | 1.24759973 | 0.84573381 | 1.84041959 | 0.26473442 |
| chr15:65899096-66370691 | 87 | 49 | 1.24759973 | 0.84573381 | 1.84041959 | 0.26473442 |
| chr15:75011134-75564998 | 87 | 49 | 1.1283275 | 0.73869905 | 1.72346634 | 0.57640977 |
| chr15:88253886-88997242 | 87 | 49 | 1.13723206 | 0.75373115 | 1.71585951 | 0.54002288 |
| chr15:96891354-97698742 | 87 | 49 | 1.04907801 | 0.71279609 | 1.54401055 | 0.80802022 |
| chr16:11352227-11658331 | 87 | 49 | 1.45746624 | 1.07307354 | 1.97955476 | 0.01588955 |
| chr16:31854743-53525739 | 87 | 49 | 0.77162888 | 0.53903082 | 1.1045957 | 0.15664438 |
| chr16:45393460-45522699 | 87 | 49 | 0.74812215 | 0.52881147 | 1.05838619 | 0.10113243 |
| chr16:45546775-51138307 | 87 | 49 | 0.76555151 | 0.53589856 | 1.09361949 | 0.1420608 |
| chr16:5062786-7709383 | 87 | 49 | 1.47219055 | 1.08351331 | 2.00029384 | 0.01340588 |
| chr16:51646446-54466783 | 87 | 49 | 0.84056053 | 0.58938172 | 1.19878506 | 0.33758882 |
| chr16:54782803-56638303 | 87 | 49 | 0.85421802 | 0.60080049 | 1.21452701 | 0.38018994 |
| chr16:56705000-65205296 | 87 | 49 | 0.82623471 | 0.58298854 | 1.17097294 | 0.28334374 |
| chr16:56791457-72871981 | 87 | 49 | 0.79264514 | 0.55900506 | 1.12393672 | 0.19215876 |
| chr16:65206155-69392572 | 87 | 49 | 0.79629708 | 0.5617178 | 1.12883913 | 0.20078583 |
| chr16:69398793-73198518 | 87 | 49 | 0.76725504 | 0.54668786 | 1.07681245 | 0.12551731 |
| chr16:73739922-78192112 | 87 | 49 | 0.74724809 | 0.53330451 | 1.04701855 | 0.09045861 |
| chr16:76685816-78205652 | 87 | 49 | 0.74724809 | 0.53330451 | 1.04701855 | 0.09045861 |
| chr16:79132355-80549399 | 87 | 49 | 0.74724809 | 0.53330451 | 1.04701855 | 0.09045861 |
| chr16:80588753-82633263 | 87 | 49 | 0.74724809 | 0.53330451 | 1.04701855 | 0.09045861 |
| chr16:80759878-82408573 | 87 | 49 | 0.74724809 | 0.53330451 | 1.04701855 | 0.09045861 |
| chr16:82644872-85172804 | 87 | 49 | 0.72695489 | 0.51979664 | 1.0166734 | 0.06241426 |
| chr16:85920445-87225756 | 87 | 49 | 0.70534456 | 0.50538142 | 0.98442667 | 0.04014605 |
| chr16:87232502-87749670 | 87 | 49 | 0.70534456 | 0.50538142 | 0.98442667 | 0.04014605 |
| chr16:88436931-88827254 | 87 | 49 | 0.70534456 | 0.50538142 | 0.98442667 | 0.04014605 |
| chr17:10675416-12635879 | 87 | 49 | 0.70167728 | 0.52897973 | 0.9307559 | 0.01398004 |
| chr17:18837023-19933105 | 87 | 49 | 0.84610791 | 0.62555161 | 1.14442769 | 0.27815309 |
| chr17:22479313-22877776 | 87 | 49 | 0.6610181 | 0.4815113 | 0.90744479 | 0.01044496 |
| chr17:24112056-24310787 | 87 | 49 | 0.68494692 | 0.52402753 | 0.89528177 | 0.00561337 |
| chr17:24936541-25468309 | 87 | 49 | 0.69358528 | 0.53283505 | 0.902832 | 0.00653176 |
| chr17:26185485-27216066 | 87 | 49 | 0.71472838 | 0.54223754 | 0.94209016 | 0.01715896 |
| chr17:28881201-29652449 | 87 | 49 | 0.73071227 | 0.5576237 | 0.95752822 | 0.02292875 |
| chr17:31895171-33758810 | 87 | 49 | 0.8129066 | 0.58141146 | 1.13657399 | 0.22577041 |
| chr17:33754231-34162198 | 87 | 49 | 0.82587295 | 0.59061681 | 1.15483696 | 0.26339706 |
| chr17:35067383-35272328 | 87 | 49 | 0.75936304 | 0.57320143 | 1.00598531 | 0.05506322 |
| chrl7:36989609-37319012 | 87 | 49 | 0.7933166 | 0.59739741 | 1.05348837 | 0.10962014 |
| chr17:37319013-37988602 | 87 | 49 | 0.75808787 | 0.5733676 | 1.00231897 | 0.05193067 |
| chr 17:39354620-40542083 | 87 | 49 | 0.83357031 | 0.62446554 | 1.11269463 | 0.2167128 |
| chr17:44673157-45060263 | 87 | 49 | 1.06938289 | 0.77810185 | 1.46970446 | 0.6792559 |
| chr17:45731662-46009941 | 87 | 49 | 1.03148522 | 0.75210971 | 1.41463639 | 0.84746752 |
| chr17:51748757-53292386 | 87 | 49 | 0.89797927 | 0.64398403 | 1.25215339 | 0.52584442 |
| chr17:55144989-55540417 | 87 | 49 | 0.84195198 | 0.60151716 | 1.17849196 | 0.31600381 |
| chr17:58249995-58447640 | 87 | 49 | 0.89140287 | 0.63892333 | 1.24365326 | 0.49866137 |
| chr17:62318152-63890591 | 87 | 49 | 0.97335931 | 0.69111583 | 1.37086768 | 0.87718091 |
| chr17:63942109-65847254 | 87 | 49 | 0.91388683 | 0.64345717 | 1.29797159 | 0.61493682 |
| chr17:70767943-71305641 | 87 | 49 | 0.7638994 | 0.54301724 | 1.07462941 | 0.12195339 |
| chr17:7471230-7717938 | 87 | 49 | 0.78437237 | 0.5791142 | 1.06238115 | 0.11664068 |
| chr17:75915141-77213225 | 87 | 49 | 0.81619979 | 0.58072038 | 1.14716501 | 0.2422316 |
| chr17:77653047-78605474 | 87 | 49 | 0.81426053 | 0.57846825 | 1.14616527 | 0.23883268 |
| chr17:78087533-78774742 | 87 | 49 | 0.81426053 | 0.57846825 | 1.14616527 | 0.23883268 |
| chr18:13905410-14018535 | 87 | 49 | 0.92442534 | 0.675808 | 1.26450442 | 0.62295788 |
| chr18:1-587750 | 87 | 49 | 1.02148326 | 0.70673012 | 1.47641658 | 0.90995397 |
| chr18:17749667-22797232 | 87 | 49 | 0.86118064 | 0.64270316 | 1.15392631 | 0.31681914 |
| chr18:3200634-3858775 | 87 | 49 | 1.23590645 | 0.74999497 | 2.03663335 | 0.40591656 |
| chr18:32113759-32649538 | 87 | 49 | 0.87567833 | 0.64647942 | 1.18613605 | 0.39119938 |
| chr18:3240326-5937123 | 87 | 49 | 1.24723854 | 0.75841242 | 2.05113199 | 0.3840494 |
| chr18:44506813-45038631 | 87 | 49 | 0.87670634 | 0.65497634 | 1.17349888 | 0.37642262 |
| chr18:46172638-49935241 | 87 | 49 | 0.87670634 | 0.65497634 | 1.17349888 | 0.37642262 |
| chr18:75796373-76117153 | 87 | 49 | 1.02170473 | 0.74690833 | 1.39760198 | 0.89313715 |
| chr19:10895846-11164554 | 87 | 49 | 1.01571096 | 0.73084119 | 1.41161825 | 0.92604167 |
| chr19:1-526082 | 87 | 49 | 0.93912751 | 0.69340594 | 1.27192518 | 0.68488956 |
| chr19:15641747-15670750 | 87 | 49 | 0.99279651 | 0.72207935 | 1.36500914 | 0.96450263 |
| chr19:1818912-2004994 | 87 | 49 | 0.93912751 | 0.69340594 | 1.27192518 | 0.68488956 |
| chr19:21788507-34401877 | 87 | 49 | 0.8981637 | 0.65601598 | 1.22969264 | 0.50282964 |
| chr19:32764651-33569283 | 87 | 49 | 1.05250681 | 0.77668968 | 1.42627181 | 0.74135703 |
| chr19:34975531-35098303 | 87 | 49 | 1.06514804 | 0.78710311 | 1.44141262 | 0.68260214 |
| chr19:37265855-38702029 | 87 | 49 | 1.06733595 | 0.78944623 | 1.44304448 | 0.67193172 |
| chr19:3 9664720-3 9962225 | 87 | 49 | 1.00680829 | 0.74690067 | 1.35715895 | 0.96447725 |
| chr19:39677306-41155075 | 87 | 49 | 1.00680829 | 0.74690067 | 1.35715895 | 0.96447725 |
| chr19:43177306-45393020 | 87 | 49 | 1.00269897 | 0.74835519 | 1.34348668 | 0.985594 |
| chr19:44889764-44995371 | 87 | 49 | 1.00269897 | 0.74835519 | 1.34348668 | 0.985594 |
| chr19:47121673-49919582 | 87 | 49 | 0.94750374 | 0.70846321 | 1.26719823 | 0.7162095 |
| chr19:49818193-50114786 | 87 | 49 | 0.92432508 | 0.69049549 | 1.2373388 | 0.59693098 |
| chr19:52031294-53331283 | 87 | 49 | 0.93438384 | 0.69819756 | 1.25046722 | 0.64802698 |
| chr19:53372336-53572212 | 87 | 49 | 0.97854537 | 0.72860595 | 1.31422348 | 0.88540024 |
| chr19:59066340-59471027 | 87 | 49 | 1.04310602 | 0.77354224 | 1.4066073 | 0.78203912 |
| chr19:63402921-63811651 | 87 | 49 | 0.96264233 | 0.71746318 | 1.29160671 | 0.79960995 |
| chr19:7321723-7480918 | 87 | 49 | 0.91349144 | 0.67331118 | 1.23934763 | 0.5610274 |
| chr2:1-15244284 | 87 | 49 | 1.3237171 | 0.97083205 | 1.80487136 | 0.07625602 |
| chr2:121187279-122198612 | 87 | 49 | 1.52051698 | 1.08008858 | 2.14053915 | 0.01632892 |
| chr2:135029078-135167772 | 87 | 49 | 1.08607105 | 0.81672858 | 1.44423783 | 0.57018014 |
| chr2:138479322-143365272 | 87 | 49 | 1.09146514 | 0.81768134 | 1.45691981 | 0.55253846 |
| chr2:154436672-155018734 | 87 | 49 | 1.16613501 | 0.85829245 | 1.58439104 | 0.32570098 |
| chr2:156889194-159797412 | 87 | 49 | 1.1838779 | 0.87717275 | 1.5978231 | 0.26987951 |
| chr2:159800558-163403274 | 87 | 49 | 1.28609327 | 0.9531463 | 1.73534313 | 0.09975701 |
| chr2:164158152-168812365 | 87 | 49 | 1.28609327 | 0.9531463 | 1.73534313 | 0.09975701 |
| chr2:173927807-175001974 | 87 | 49 | 1.28609327 | 0.9531463 | 1.73534313 | 0.09975701 |
| chr2:204533830-206266883 | 87 | 49 | 1.03359222 | 0.76133797 | 1.40320451 | 0.83224592 |
| chr2:241477619-242951149 | 87 | 49 | 1.07483275 | 0.78053583 | 1.48009277 | 0.65842682 |
| chr2:32460827-55039898 | 87 | 49 | 1.24216632 | 0.90439906 | 1.70608003 | 0.18045636 |
| chr2:61834240-63151654 | 87 | 49 | 1.38844834 | 0.99667429 | 1.93422145 | 0.05234696 |
| chr2:79257340-80427237 | 87 | 49 | 1.22196115 | 0.89084705 | 1.67614524 | 0.21380789 |
| chr20:14210829-15988895 | 87 | 49 | 1.4094559 | 0.99857165 | 1.98940752 | 0.0509578 |
| chr20:22719364-23648289 | 87 | 49 | 1.35642195 | 0.96924272 | 1.89826601 | 0.0754397 |
| chr20:27100000-62435964 | 87 | 49 | 1.35111731 | 1.05415739 | 1.73173191 | 0.01747932 |
| chr20:29526118-29834552 | 87 | 49 | 1.59224579 | 1.11644693 | 2.27081701 | 0.01022512 |
| chr20:33386980-33969561 | 87 | 49 | 1.35559707 | 1.03516955 | 1.77521008 | 0.02702351 |
| chr20:34179462-45265004 | 87 | 49 | 1.3619665 | 1.04842559 | 1.76927457 | 0.020656 |
| chr20:40134806-41603948 | 87 | 49 | 1.22845255 | 0.92438895 | 1.6325332 | 0.15616463 |
| chr20:41621022-45719023 | 87 | 49 | 1.29571302 | 0.9868284 | 1.70128082 | 0.06224669 |
| chr20:45719063-49181611 | 87 | 49 | 1.24537789 | 0.96899377 | 1.60059449 | 0.08653746 |
| chr20:49438571-52269898 | 87 | 49 | 1.18528051 | 0.92794429 | 1.51398084 | 0.17347351 |
| chr20:51603033-51989829 | 87 | 49 | 1.1775272 | 0.91960447 | 1.50778988 | 0.19513872 |
| chr20:59868877-61137250 | 87 | 49 | 1.20278865 | 0.92822473 | 1.55856711 | 0.1625335 |
| chr20:61329497-62435964 | 87 | 49 | 1.19310683 | 0.92052085 | 1.54641137 | 0.18214754 |
| chr21:16248572-25859193 | 87 | 49 | 1.03328383 | 0.75113717 | 1.42141212 | 0.84052134 |
| chr21:38584860-42033506 | 87 | 49 | 0.98371879 | 0.75122304 | 1.28816957 | 0.90502166 |
| chr21:44316476-46902240 | 87 | 49 | 1.01049086 | 0.75314389 | 1.35577251 | 0.94452092 |
| chr21:46690655-46902240 | 87 | 49 | 1.01330107 | 0.75343958 | 1.36278886 | 0.93035515 |
| chr22:15272143-16594738 | 87 | 49 | 1.08953259 | 0.78016365 | 1.52157981 | 0.61483301 |
| chr22:20517661-21169423 | 87 | 49 | 1.12352356 | 0.80089056 | 1.57612695 | 0.50007268 |
| chr22:22437934-22507511 | 87 | 49 | 1.0866082 | 0.77951259 | 1.51468673 | 0.62403917 |
| chr22:23139372-23249329 | 87 | 49 | 1.0866082 | 0.77951259 | 1.51468673 | 0.62403917 |
| chr22:36048950-38681384 | 87 | 49 | 0.90166879 | 0.66583542 | 1.22103237 | 0.5034365 |
| chr22:37740208-38258806 | 87 | 49 | 0.90166879 | 0.66583542 | 1.22103237 | 0.5034365 |
| chr22:45488286-49691432 | 87 | 49 | 1.02013548 | 0.74149013 | 1.40349327 | 0.9025236 |
| chr3:116900556-120107320 | 87 | 49 | 0.86719468 | 0.69375988 | 1.08398688 | 0.21071603 |
| chr3:1-2121282 | 87 | 49 | 0.86712308 | 0.61420575 | 1.22418657 | 0.41775466 |
| chr3:170024984-173604597 | 87 | 49 | 1.06675231 | 0.7682009 | 1.48133188 | 0.6996818 |
| chr3:175446835-178263192 | 87 | 49 | 1.00787654 | 0.70194215 | 1.44714935 | 0.96609375 |
| chr3:178149984-199501827 | 87 | 49 | 0.83390399 | 0.58470443 | 1.18931178 | 0.3159626 |
| chr3:36286137-40063081 | 87 | 49 | 0.93155469 | 0.69865404 | 1.24209422 | 0.62908719 |
| chr3:41866-18694914 | 87 | 49 | 0.86610201 | 0.6113629 | 1.22698432 | 0.41857212 |
| chr3:43362938-44130419 | 87 | 49 | 0.91680406 | 0.6855843 | 1.22600486 | 0.55801251 |
| chr3:58626894-61524607 | 87 | 49 | 1.2025079 | 0.85329052 | 1.69464585 | 0.29208902 |
| chr3:66644057-68118027 | 87 | 49 | 1.21670657 | 0.85963505 | 1.72209692 | 0.26844816 |
| chr3:70107050-74383584 | 87 | 49 | 1.19688568 | 0.8523633 | 1.68066286 | 0.29942724 |
| chr3:86250885-95164178 | 87 | 49 | 0.79377931 | 0.59024149 | 1.06750475 | 0.12655691 |
| chr4:104889590-106814504 | 87 | 49 | 0.93716279 | 0.67636616 | 1.2985187 | 0.69651267 |
| chr4:10979549-15266111 | 87 | 49 | 0.8961396 | 0.64740605 | 1.24043664 | 0.50856923 |
| chr4:112023722-118112513 | 87 | 49 | 0.8914431 | 0.63931557 | 1.24300242 | 0.49809597 |
| chr4:114219937-114355024 | 87 | 49 | 0.8914431 | 0.63931557 | 1.24300242 | 0.49809597 |
| chr4:1234177-1683843 | 87 | 49 | 0.91706168 | 0.65679037 | 1.28047269 | 0.61120262 |
| chr4:13238819-37565122 | 87 | 49 | 0.88655891 | 0.63986098 | 1.22837105 | 0.46925253 |
| chr4:147085600-153449318 | 87 | 49 | 0.9776757 | 0.6979547 | 1.36950117 | 0.89553945 |
| chr4:151218876-154929678 | 87 | 49 | 0.95739208 | 0.67714919 | 1.35361544 | 0.8053554 |
| chr4:15313739-17632477 | 87 | 49 | 0.92499828 | 0.66985727 | 1.27731959 | 0.6358695 |
| chr4:155375138-160500747 | 87 | 49 | 0.8363535 | 0.59276536 | 1.18004059 | 0.30894871 |
| chr4:162524501-164492534 | 87 | 49 | 0.84736961 | 0.61616495 | 1.1653296 | 0.30830755 |
| chr4:164612456-170167997 | 87 | 49 | 0.83524343 | 0.61098513 | 1.14181435 | 0.25906811 |
| chr4:170251982-170915637 | 87 | 49 | 0.83205255 | 0.60866129 | 1.13743302 | 0.24905255 |
| chr4:174326479-176135905 | 87 | 49 | 0.81826356 | 0.59401927 | 1.12716082 | 0.21966196 |
| chr4:175486008-184028292 | 87 | 49 | 0.81680808 | 0.5932554 | 1.12460069 | 0.21488894 |
| chr4:176791083-177486490 | 87 | 49 | 0.81826356 | 0.59401927 | 1.12716082 | 0.21966196 |
| chr4:186684565-191273063 | 87 | 49 | 0.83005313 | 0.60796553 | 1.13326852 | 0.24100836 |
| chr4:19864333-22430289 | 87 | 49 | 0.90492329 | 0.65982935 | 1.2410575 | 0.53531686 |
| chr4:23402742-26636101 | 87 | 49 | 0.87945526 | 0.63829293 | 1.21173449 | 0.43215048 |
| chr4:30331135-30753569 | 87 | 49 | 0.84445381 | 0.62136532 | 1.14763765 | 0.28007067 |
| chr4:3220565-5553626 | 87 | 49 | 0.93453326 | 0.66860379 | 1.30623312 | 0.69187909 |
| chr4:3 712173 8-3 88048 10 | 87 | 49 | 0.78076942 | 0.56938634 | 1.07062789 | 0.1244639 |
| chr4:41057559-42353879 | 87 | 49 | 0.78770024 | 0.57370245 | 1.08152174 | 0.14009698 |
| chr4:54471680-55980061 | 87 | 49 | 1.01004723 | 0.70385932 | 1.44943082 | 0.95673543 |
| chr4:5577784-10295484 | 87 | 49 | 0.95207067 | 0.68171618 | 1.32964214 | 0.77319511 |
| chr4:71492582-75938920 | 87 | 49 | 1.19086128 | 0.83093028 | 1.70670225 | 0.34145138 |
| chr4:73526461-75252649 | 87 | 49 | 1.19988333 | 0.83719598 | 1.71969293 | 0.32104702 |
| chr4:76623381-79684447 | 87 | 49 | 0.98794462 | 0.7093607 | 1.37593552 | 0.94279221 |
| chr4:79691766-80079606 | 87 | 49 | 0.96328105 | 0.68832645 | 1.34806731 | 0.82729815 |
| chr4:83634873-83961360 | 87 | 49 | 0.98536883 | 0.70612035 | 1.37505133 | 0.93091627 |
| chr4:91089383-93486891 | 87 | 49 | 1.04173395 | 0.74366904 | 1.45926421 | 0.81206672 |
| chr5:101597589-102642260 | 87 | 49 | 0.64399969 | 0.47731107 | 0.86890001 | 0.00398307 |
| chr5:102912456-102926389 | 87 | 49 | 0.64399969 | 0.47731107 | 0.86890001 | 0.00398307 |
| chr5:102912456-102926389 | 87 | 49 | 0.64399969 | 0.47731107 | 0.86890001 | 0.00398307 |
| chr5:106744250-109231328 | 87 | 49 | 0.63012871 | 0.47211235 | 0.84103326 | 0.00171701 |
| chr5:109877429-111137815 | 87 | 49 | 0.60692171 | 0.45880422 | 0.80285651 | 0.00046837 |
| chr5:111507433-112958878 | 87 | 49 | 0.60692171 | 0.45880422 | 0.80285651 | 0.00046837 |
| chr5:113725565-115180304 | 87 | 49 | 0.61985399 | 0.46518408 | 0.82595039 | 0.0010923 |
| chr5:115193714-121517156 | 87 | 49 | 0.62625225 | 0.47314648 | 0.82890161 | 0.00106833 |
| chr5:1212750-1378766 | 87 | 49 | 1.00369501 | 0.72001765 | 1.39913747 | 0.98263756 |
| chr5:121675719-127022221 | 87 | 49 | 0.61674257 | 0.46441886 | 0.81902659 | 0.00083973 |
| chr5:125787000-126200608 | 87 | 49 | 0.62147541 | 0.4661757 | 0.82851098 | 0.00118564 |
| chr5:127447382-129549383 | 87 | 49 | 0.65795102 | 0.49380245 | 0.87666545 | 0.00425115 |
| chr5:128458341-128477620 | 87 | 49 | 0.65331012 | 0.49062052 | 0.86994753 | 0.00357433 |
| chr5:130522776-135427403 | 87 | 49 | 0.62905077 | 0.45461393 | 0.8704196 | 0.00514985 |
| chr5:135493104-138988200 | 87 | 49 | 0.68372853 | 0.49248549 | 0.9492355 | 0.02313568 |
| chr5:139008130-142795270 | 87 | 49 | 0.71724836 | 0.51733095 | 0.99442187 | 0.04620472 |
| chr5:143171919-147191453 | 87 | 49 | 0.8018972 | 0.57567863 | 1.11701059 | 0.19169412 |
| chr5:147238467-151765017 | 87 | 49 | 0.80507378 | 0.57802536 | 1.12130685 | 0.19961543 |
| chr5:1473801-6790134 | 87 | 49 | 1.00369501 | 0.72001765 | 1.39913747 | 0.98263756 |
| chr5:152850499-154326721 | 87 | 49 | 0.78855109 | 0.56907869 | 1.09266581 | 0.15344864 |
| chr5:155686345-159845035 | 87 | 49 | 0.80600075 | 0.58086873 | 1.11838902 | 0.19688708 |
| chr5:159922707-167623739 | 87 | 49 | 0.86354301 | 0.62509159 | 1.19295562 | 0.37354914 |
| chr5:167651670-171366482 | 87 | 49 | 0.90601193 | 0.66096242 | 1.24191269 | 0.53958067 |
| chr5:172591744-174888201 | 87 | 49 | 0.92563311 | 0.67644759 | 1.26661203 | 0.62913899 |
| chr5:174477192-180857866 | 87 | 49 | 0.86654791 | 0.64709446 | 1.16042608 | 0.33637152 |
| chr5:176660805-180009206 | 87 | 49 | 0.86333787 | 0.64512769 | 1.15535622 | 0.32289472 |
| chr5:177541057-180857866 | 87 | 49 | 0.86748323 | 0.6478697 | 1.16154091 | 0.33982736 |
| chr5:18240864-21565169 | 87 | 49 | 0.92629564 | 0.65875011 | 1.30250242 | 0.65975695 |
| chr5:39149695-41885012 | 87 | 49 | 0.90828987 | 0.6459643 | 1.27714565 | 0.58014449 |
| chr5:43612947-44998256 | 87 | 49 | 0.92158565 | 0.65536076 | 1.29595812 | 0.63872774 |
| chr5:45312870-49697231 | 87 | 49 | 0.8937206 | 0.63503182 | 1.25778973 | 0.51927339 |
| chr5:52119531-58183162 | 87 | 49 | 0.78762889 | 0.57704118 | 1.0750693 | 0.1325944 |
| chr5:56146657-56594666 | 87 | 49 | 0.76711515 | 0.56433481 | 1.0427598 | 0.09052388 |
| chr5:57754754-59053198 | 87 | 49 | 0.73474583 | 0.54638924 | 0.98803451 | 0.04138825 |
| chr5:58300629-59320301 | 87 | 49 | 0.73474583 | 0.54638924 | 0.98803451 | 0.04138825 |
| chr5:58300629-62108926 | 87 | 49 | 0.74297415 | 0.55219738 | 0.99966173 | 0.04973938 |
| chr5:63292034-63701397 | 87 | 49 | 0.76859294 | 0.56709087 | 1.04169392 | 0.08976436 |
| chr5:63838208-66501179 | 87 | 49 | 0.7907369 | 0.57700089 | 1.08364626 | 0.14420068 |
| chr5:66513872-67633403 | 87 | 49 | 0.79669811 | 0.58202762 | 1.09054597 | 0.15594243 |
| chr5:68425638-71536822 | 87 | 49 | 0.70105704 | 0.51112743 | 0.96156252 | 0.02758806 |
| chr5:68425638-72913540 | 87 | 49 | 0.6731588 | 0.49582411 | 0.91391838 | 0.01118177 |
| chr5:73958991-76396789 | 87 | 49 | 0.70402401 | 0.52376128 | 0.9463277 | 0.02004395 |
| chr5:76408288-81082828 | 87 | 49 | 0.6754161 | 0.50423981 | 0.90470228 | 0.00849964 |
| chr5:81303630-82685333 | 87 | 49 | 0.64258655 | 0.48267811 | 0.85547174 | 0.00245227 |
| chr5:82803339-90714877 | 87 | 49 | 0.61527047 | 0.46210864 | 0.81919645 | 0.00088285 |
| chr5:85837489-133480433 | 87 | 49 | 0.59620019 | 0.43772332 | 0.8120533 | 0.00103609 |
| chr5:9224578-10673735 | 87 | 49 | 0.96610462 | 0.69871242 | 1.33582588 | 0.83477865 |
| chr5:92250850-96544700 | 87 | 49 | 0.63934695 | 0.47458364 | 0.86131187 | 0.00326227 |
| chr5:98132900-102642260 | 87 | 49 | 0.64399969 | 0.47731107 | 0.86890001 | 0.00398307 |
| chr6:101000242-121511318 | 87 | 49 | 1.00610187 | 0.7530111 | 1.34425769 | 0.9671776 |
| chr6:106604408-107782614 | 87 | 49 | 0.96192263 | 0.71662439 | 1.29118568 | 0.79604602 |
| chr6:110391-1254918 | 87 | 49 | 0.98752889 | 0.71384995 | 1.36613205 | 0.93958549 |
| chr6:116369389-117096650 | 87 | 49 | 1.03430056 | 0.77227182 | 1.38523461 | 0.82099901 |
| chr6:117109043-117359993 | 87 | 49 | 1.03724647 | 0.77441947 | 1.38927323 | 0.80623568 |
| chr6:119295855-120272161 | 87 | 49 | 1.03724647 | 0.77441947 | 1.38927323 | 0.80623568 |
| chr6:1-23628840 | 87 | 49 | 1.10138454 | 0.7986187 | 1.51893249 | 0.5559825 |
| chr6:129217882-131730157 | 87 | 49 | 0.99149277 | 0.78616302 | 1.25045046 | 0.94247235 |
| chr6:135561194-135665525 | 87 | 49 | 0.98780096 | 0.78693321 | 1.23994097 | 0.91572576 |
| chr6:13729714-14088212 | 87 | 49 | 1.09861444 | 0.81845006 | 1.47468215 | 0.53121607 |
| chr6:137619861-138589104 | 87 | 49 | 0.98567782 | 0.7863423 | 1.23554433 | 0.90041231 |
| chr6:139381116-139689363 | 87 | 49 | 0.98567782 | 0.7863423 | 1.23554433 | 0.90041231 |
| chr6:143157329-144194971 | 87 | 49 | 0.90605798 | 0.67405022 | 1.2179227 | 0.51332529 |
| chr6:149442557-149779188 | 87 | 49 | 0.85184335 | 0.63617154 | 1.14063118 | 0.28167616 |
| chr6:1543157-2570302 | 87 | 49 | 0.98752889 | 0.71384995 | 1.36613205 | 0.93958549 |
| chr6:157695838-157823719 | 87 | 49 | 0.82093564 | 0.62483371 | 1.0785835 | 0.15654885 |
| chr6:161612277-163134099 | 87 | 49 | 0.86712205 | 0.65552978 | 1.14701221 | 0.31781784 |
| chr6:30420923-31893702 | 87 | 49 | 1.09138479 | 0.79598275 | 1.49641529 | 0.58710972 |
| chr6:31910383-31915520 | 87 | 49 | 1.07839244 | 0.77957002 | 1.49175857 | 0.64848634 |
| chr6:38787570-41935171 | 87 | 49 | 1.13856629 | 0.83728555 | 1.54825696 | 0.40794663 |
| chr6:4058046-25852810 | 87 | 49 | 1.13896634 | 0.82732927 | 1.56799036 | 0.42499182 |
| chr6:42772314-43039595 | 87 | 49 | 1.06577632 | 0.78003612 | 1.45618789 | 0.68913504 |
| chr6:43556800-44361368 | 87 | 49 | 1.06577632 | 0.78003612 | 1.45618789 | 0.68913504 |
| chr6:54352087-55392731 | 87 | 49 | 0.98134293 | 0.72350221 | 1.33107257 | 0.90361416 |
| chr6:63255006-65243766 | 87 | 49 | 1.01345772 | 0.74816344 | 1.37282374 | 0.93120561 |
| chr6:76630464-105342994 | 87 | 49 | 0.86797566 | 0.65310396 | 1.15354032 | 0.32921547 |
| chr7:102988499-103837783 | 87 | 49 | 1.37636222 | 0.98779957 | 1.91777058 | 0.05910192 |
| chr7:115981465-116676953 | 87 | 49 | 1.19776042 | 0.87189292 | 1.64541996 | 0.26535973 |
| chr7:129281946-131188950 | 87 | 49 | 1.19431727 | 0.8568427 | 1.66470897 | 0.29460426 |
| chr7:137042370-158819393 | 87 | 49 | 1.40303508 | 0.98351899 | 2.00149407 | 0.06172307 |
| chr7:141592807-142264966 | 87 | 49 | 1.30049859 | 0.93269145 | 1.81335058 | 0.12134974 |
| chr7:144118814-148066271 | 87 | 49 | 1.41835964 | 1.00344159 | 2.0048442 | 0.04776849 |
| chr7:156893473-158821424 | 87 | 49 | 1.50260192 | 1.04169649 | 2.16743796 | 0.02936769 |
| chr7:16017926-18944036 | 87 | 49 | 1.32145938 | 0.96418264 | 1.8111246 | 0.08306589 |
| chr7:3046420-4279470 | 87 | 49 | 1.3460515 | 0.9884063 | 1.83310712 | 0.0593007 |
| chr7:3516940-6019790 | 87 | 49 | 1.32035308 | 0.97071196 | 1.79593157 | 0.0766315 |
| chr7:54899301-55275419 | 87 | 49 | 0.94994093 | 0.62878913 | 1.43511986 | 0.80726921 |
| chr7:65877239-79629882 | 87 | 49 | 1.22657044 | 0.88610485 | 1.69785217 | 0.21830219 |
| chr7:83246850-83689170 | 87 | 49 | 1.56528252 | 1.13729726 | 2.15432626 | 0.00597026 |
| chr7:86091574-88424037 | 87 | 49 | 1.56231299 | 1.12977223 | 2.16045482 | 0.00698146 |
| chr7 :8779703 8-89779004 | 87 | 49 | 1.44430598 | 1.03285579 | 2.01966215 | 0.03163987 |
| chr7:89924533-98997268 | 87 | 49 | 1.42807408 | 1.02146976 | 1.99653053 | 0.0371406 |
| chr7:92829306-132566935 | 87 | 49 | 1.35348736 | 0.97199398 | 1.88471128 | 0.07316338 |
| chr8:101163387-103693879 | 87 | 49 | 1.29684031 | 0.99921389 | 1.6831179 | 0.05069507 |
| chr8:11575198-11708771 | 87 | 49 | 0.73602794 | 0.55553686 | 0.97515965 | 0.03274427 |
| chr8:116186189-120600761 | 87 | 49 | 1.32258349 | 1.01813758 | 1.71806555 | 0.03620382 |
| chr8:128414451-128718816 | 87 | 49 | 1.30196717 | 0.99978568 | 1.69548188 | 0.05018623 |
| chr8:128774432-128849112 | 87 | 49 | 1.30196717 | 0.99978568 | 1.69548188 | 0.05018623 |
| chr8:128823745-146264218 | 87 | 49 | 1.34192122 | 1.04707847 | 1.7197876 | 0.0201581 |
| chr8:133698327-133914560 | 87 | 49 | 1.50645926 | 1.17499962 | 1.93142148 | 0.00122952 |
| chr8:140458177-146274826 | 87 | 49 | 1.31264219 | 1.02428975 | 1.68217003 | 0.03158679 |
| chr8:2053441-6259545 | 87 | 49 | 0.77571516 | 0.5828766 | 1.03235232 | 0.0815752 |
| chr8:22125332-30139123 | 87 | 49 | 0.69169014 | 0.50286091 | 0.95142663 | 0.02344752 |
| chr8:29285985-30473918 | 87 | 49 | 0.72204438 | 0.52703391 | 0.98921165 | 0.042612 |
| chr8:38252951-38460772 | 87 | 49 | 1.24080797 | 0.89273972 | 1.72458375 | 0.19896671 |
| chr8:39008109-41238710 | 87 | 49 | 1.15991504 | 0.84779492 | 1.58694379 | 0.35363864 |
| chr8:41004735-41425303 | 87 | 49 | 1.20866408 | 0.90802227 | 1.60884695 | 0.19403125 |
| chr8:42006632-42404492 | 87 | 49 | 1.23384923 | 0.92832463 | 1.63992625 | 0.147725 |
| chr8:42971602-72924037 | 87 | 49 | 1.29944126 | 0.98530279 | 1.71373471 | 0.06358177 |
| chr8:62174237-62716885 | 87 | 49 | 1.21597542 | 0.93287772 | 1.58498397 | 0.14814106 |
| chr8:81242335-81979194 | 87 | 49 | 1.36271488 | 1.05499797 | 1.76018524 | 0.01779005 |
| chr8:82099049-82634968 | 87 | 49 | 1.36561154 | 1.05649103 | 1.76517815 | 0.0173302 |
| chr8:94587173-100685605 | 87 | 49 | 1.3476981 | 1.040972 | 1.74480214 | 0.02352963 |
| chr9:110661885-116923292 | 87 | 49 | 1.23158334 | 0.87218062 | 1.73908647 | 0.23674428 |
| chr9:110838572-127593510 | 87 | 49 | 1.19715272 | 0.85087924 | 1.68434552 | 0.30161845 |
| chr9:122980237-124410900 | 87 | 49 | 1.14471774 | 0.82216751 | 1.59380989 | 0.42348394 |
| chr9:12683435-13935586 | 87 | 49 | 1.11534391 | 0.79187127 | 1.57095238 | 0.53219981 |
| chr9:130999928-132303780 | 87 | 49 | 1.1191473 | 0.80989318 | 1.54648873 | 0.49513151 |
| chr9:137859478-140273252 | 87 | 49 | 0.97385542 | 0.74438223 | 1.27406907 | 0.84677385 |
| chr9:14071847-14900353 | 87 | 49 | 1.11534391 | 0.79187127 | 1.57095238 | 0.53219981 |
| chr9:21489625-22474701 | 87 | 49 | 1.11033825 | 0.78142585 | 1.57769419 | 0.5592586 |
| chr9:235706-8362053 | 87 | 49 | 1.21251889 | 0.85038636 | 1.72886366 | 0.28705352 |
| chr9:23819824-26891068 | 87 | 49 | 1.11033825 | 0.78142585 | 1.57769419 | 0.5592586 |
| chr9 :26573226-29470065 | 87 | 49 | 1.11033825 | 0.78142585 | 1.57769419 | 0.5592586 |
| chr9:34233377-35934224 | 87 | 49 | 1.12428821 | 0.78034079 | 1.61983585 | 0.52950114 |
| chr9:36365710-37139941 | 87 | 49 | 1.0935926 | 0.76195714 | 1.56956962 | 0.62746449 |
| chr9:37861914-38370450 | 87 | 49 | 1.06160394 | 0.74324652 | 1.51632454 | 0.7424161 |
| chr9:71280626-72167093 | 87 | 49 | 1.26191583 | 0.91270917 | 1.74473052 | 0.15931388 |
| chr9:7161607-12713130 | 87 | 49 | 1.11534391 | 0.79187127 | 1.57095238 | 0.53219981 |
| chr9:831690-1047552 | 87 | 49 | 1.33981897 | 0.91476011 | 1.96238867 | 0.13299431 |
| chr9:97183463-97625342 | 87 | 49 | 1.26540012 | 0.90095025 | 1.77727624 | 0.1744181 |
| chrX:1-60000000 | 87 | 49 | 0.91706168 | 0.65679037 | 1.28047269 | 0.61120262 |
| chrX:60000000-154913754 | 87 | 49 | 0.96037718 | 0.7041487 | 1.30984313 | 0.79846453 |
| chr8:45200000-146274826 | 87 | 49 | 1.34555729 | 1.04208219 | 1.73741039 | 0.02284215 |
| chr5:47700000-180857866 | 87 | 49 | 0.68155455 | 0.48676118 | 0.95430084 | 0.02559301 |
| chr14:15600000-106368585 | 87 | 49 | 0.75965667 | 0.5745715 | 1.00436282 | 0.05368017 |
| chr7:59100000-158821424 | 87 | 49 | 1.36386172 | 0.98890836 | 1.88098196 | 0.05849597 |
| chr7:1-59100000 | 87 | 49 | 1.32985102 | 0.98167616 | 1.80151439 | 0.06568646 |
| chr20:1-27100000 | 87 | 49 | 1.36180773 | 0.97432802 | 1.90338394 | 0.07064959 |
| chr13: 16000000-114142980 | 87 | 49 | 0.779832 | 0.5875058 | 1.0351182 | 0.08523583 |
| chr8:1-45200000 | 87 | 49 | 0.76784595 | 0.5673597 | 1.03917745 | 0.08707196 |
| chr10:40300000-135374737 | 87 | 49 | 0.76217072 | 0.5571058 | 1.04271793 | 0.08943739 |
| chr17:1-22200000 | 87 | 49 | 0.78490418 | 0.59009591 | 1.04402447 | 0.09611901 |
| chr2:1-93300000 | 87 | 49 | 1.30034075 | 0.95281075 | 1.77462952 | 0.09786544 |
| chr17:22200000-78774742 | 87 | 49 | 0.7806912 | 0.58116351 | 1.04872163 | 0.1001645 |
| chr16:38200000-88827254 | 87 | 49 | 0.76025751 | 0.53815667 | 1.07402084 | 0.11997486 |
| chr11:1-52900000 | 87 | 49 | 0.81712887 | 0.61615547 | 1.0836544 | 0.16086213 |
| chr11:52900000-134452384 | 87 | 49 | 0.80189541 | 0.5680123 | 1.13208155 | 0.20953446 |
| chr3:91700000-199501827 | 87 | 49 | 0.83574254 | 0.62316538 | 1.12083504 | 0.23083461 |
| chr12:35400000-132349534 | 87 | 49 | 0.82297288 | 0.58880063 | 1.15027791 | 0.25409825 |
| chr16:1-38200000 | 87 | 49 | 1.20437413 | 0.86517505 | 1.67655903 | 0.27052528 |
| chr2:93300000-242951149 | 87 | 49 | 1.1807093 | 0.87595715 | 1.59148704 | 0.2754818 |
| chr9:51800000-140273252 | 87 | 49 | 1.17588992 | 0.8512488 | 1.62433955 | 0.32563813 |
| chr18:16100000-76117153 | 87 | 49 | 0.87870808 | 0.6548628 | 1.17906819 | 0.38873047 |
| chr12:1-35400000 | 87 | 49 | 0.87681468 | 0.64061718 | 1.20009893 | 0.41169329 |
| chr1:124300000-247249719 | 87 | 49 | 1.11506012 | 0.85475815 | 1.45463262 | 0.42201208 |
| chr15:17000000-100338915 | 87 | 49 | 1.15337846 | 0.79340991 | 1.67666404 | 0.45471319 |
| chr1:1-124300000 | 87 | 49 | 0.89919237 | 0.67902205 | 1.19075208 | 0.45835356 |
| chr9:1-51800000 | 87 | 49 | 1.14272564 | 0.79957835 | 1.63313814 | 0.46399177 |
| chr4:1-50700000 | 87 | 49 | 0.88361455 | 0.62965928 | 1.2399955 | 0.47416647 |
| chr6:60500000-170899992 | 87 | 49 | 0.89951189 | 0.67243472 | 1.20327164 | 0.47558905 |
| chr6:1-60500000 | 87 | 49 | 1.11325534 | 0.81664398 | 1.51759822 | 0.49734275 |
| chr10:1-40300000 | 87 | 49 | 1.09875824 | 0.78610325 | 1.53576478 | 0.58145029 |
| chr18:1-16100000 | 87 | 49 | 1.07476669 | 0.7667957 | 1.50642922 | 0.67554095 |
| chr5:1-47700000 | 87 | 49 | 0.93435393 | 0.67087644 | 1.30130857 | 0.68788213 |
| chr22:11800000-49691432 | 87 | 49 | 0.95826088 | 0.70387387 | 1.30458588 | 0.78650485 |
| chr4:50700000-191273063 | 87 | 49 | 0.95892647 | 0.67528166 | 1.36171323 | 0.81466966 |
| chr19:1-28500000 | 87 | 49 | 0.96812462 | 0.70989572 | 1.32028587 | 0.8378436 |
| chr3:1-91700000 | 87 | 49 | 0.96794901 | 0.70267057 | 1.33337772 | 0.84199514 |
| chr19:28500000-63811651 | 87 | 49 | 0.98683507 | 0.73447086 | 1.32591162 | 0.92992211 |
| chr21:12300000-46944323 | 87 | 49 | 0.9959534 | 0.7525663 | 1.31805419 | 0.97737362 |

**Table 7. Univariate analyses of 491 DNA segments for overall survival (OS) in 87 patients with advanced HR+/HER2- breast cancer treated with CDK4/6 inhibitors and endocrine therapy.**

| **Segment** | **n** | **n_event** | **HR** | **Lower_HR** | **UpperHR** | **pvalue** |
|---|---|---|---|---|---|---|
| chr10:129812260-135374737 | 87 | 20 | 0.42865517 | 0.29378287 | 0.62544578 | 1.11E-05 |
| chr10:128584013-129240925 | 87 | 20 | 0.42950348 | 0.29450862 | 0.62637637 | 1.13E-05 |
| chr10:119032598-121691235 | 87 | 20 | 0.44695452 | 0.31160865 | 0.6410873 | 1.21E-05 |
| chr10:122206456-122883255 | 87 | 20 | 0.44374794 | 0.30826499 | 0.63877588 | 1.23E-05 |
| chr10:123214869-123367187 | 87 | 20 | 0.44374794 | 0.30826499 | 0.63877588 | 1.23E-05 |
| chr10:123223889-124739898 | 87 | 20 | 0.44374794 | 0.30826499 | 0.63877588 | 1.23E-05 |
| chr10:118946990-119027085 | 87 | 20 | 0.4526917 | 0.31703222 | 0.64640045 | 1.30E-05 |
| chr14:57736586-58085299 | 87 | 20 | 0.45785992 | 0.32114751 | 0.65277078 | 1.58E-05 |
| chr14:65275722-67085224 | 87 | 20 | 0.47268739 | 0.33111705 | 0.67478668 | 3.69E-05 |
| chr14:69312342-69568836 | 87 | 20 | 0.47627839 | 0.33329599 | 0.68059958 | 4.65E-05 |
| chr14:77311255-79682157 | 87 | 20 | 0.5180374 | 0.35724188 | 0.75120742 | 0.00052301 |
| chr14:77945724-77992686 | 87 | 20 | 0.5180374 | 0.35724188 | 0.75120742 | 0.00052301 |
| chr14:54378476-55837783 | 87 | 20 | 0.52272601 | 0.36176431 | 0.75530525 | 0.00055162 |
| chr14:56307190-56415610 | 87 | 20 | 0.53175821 | 0.37049274 | 0.76321817 | 0.00061351 |
| chr3:170024984-173604597 | 87 | 20 | 2.03653724 | 1.3552796 | 3.06024229 | 0.00061916 |
| chr14:15600000-106368585 | 87 | 20 | 0.55774166 | 0.39745896 | 0.78266132 | 0.00073123 |
| chr14:52311662-54325595 | 87 | 20 | 0.52561936 | 0.35832356 | 0.77102302 | 0.0010012 |
| chr10:40300000-135374737 | 87 | 20 | 0.50968553 | 0.33767381 | 0.76932037 | 0.00133478 |
| chr13:1-23902184 | 87 | 20 | 0.55981284 | 0.39227197 | 0.79891107 | 0.00138764 |
| chr5:125787000-126200608 | 87 | 20 | 0.56558445 | 0.39400038 | 0.81189201 | 0.00200313 |
| chr5:106744250-109231328 | 87 | 20 | 0.56667135 | 0.39408835 | 0.81483358 | 0.00217689 |
| chr5:121675719-127022221 | 87 | 20 | 0.57212408 | 0.3994166 | 0.81951015 | 0.00232209 |
| chr5:113725565-115180304 | 87 | 20 | 0.56753257 | 0.39302155 | 0.81953068 | 0.00251447 |
| chr14:80741860-106368585 | 87 | 20 | 0.55740773 | 0.38085152 | 0.81581236 | 0.0026341 |
| chr5:109877429-111137815 | 87 | 20 | 0.5740187 | 0.39909079 | 0.82562033 | 0.00276031 |
| chr5:111507433-112958878 | 87 | 20 | 0.5740187 | 0.39909079 | 0.82562033 | 0.00276031 |
| chr5:98132900-102642260 | 87 | 20 | 0.56834382 | 0.39146582 | 0.82514151 | 0.00297447 |
| chr5:101597589-102642260 | 87 | 20 | 0.56834382 | 0.39146582 | 0.82514151 | 0.00297447 |
| chr5:102912456-102926389 | 87 | 20 | 0.56834382 | 0.39146582 | 0.82514151 | 0.00297447 |
| chr5:102912456-102926389 | 87 | 20 | 0.56834382 | 0.39146582 | 0.82514151 | 0.00297447 |
| chr17:10675416-12635879 | 87 | 20 | 0.50216985 | 0.31792145 | 0.79319769 | 0.00314391 |
| chr13:1-40829685 | 87 | 20 | 0.59149511 | 0.41683781 | 0.83933476 | 0.00327277 |
| chr5:92250850-96544700 | 87 | 20 | 0.57217132 | 0.39421176 | 0.83046741 | 0.00331115 |
| chr3:175446835-178263192 | 87 | 20 | 1.93893042 | 1.2449065 | 3.01986628 | 0.0034007 |
| chr12:42034279-44588086 | 87 | 20 | 0.62875251 | 0.46068291 | 0.85813845 | 0.0034552 |
| chr14 :92773649-95250286 | 87 | 20 | 0.56851382 | 0.38224926 | 0.8455424 | 0.00529745 |
| chr14:93528266-103946058 | 87 | 20 | 0.57469714 | 0.38836611 | 0.85042643 | 0.00560127 |
| chr5:85837489-133480433 | 87 | 20 | 0.60486244 | 0.42304583 | 0.86482018 | 0.00584862 |
| chr9:831690-1047552 | 87 | 20 | 2.55610503 | 1.30369584 | 5.01165438 | 0.00629536 |
| chr16:5062786-7709383 | 87 | 20 | 1.76593328 | 1.17351637 | 2.65741528 | 0.0063849 |
| chr14 :92049878-92765306 | 87 | 20 | 0.58206602 | 0.39360183 | 0.86077053 | 0.00670735 |
| chr14:95740950-100105884 | 87 | 20 | 0.58133878 | 0.39135151 | 0.86355811 | 0.00722023 |
| chr5:115193714-121517156 | 87 | 20 | 0.61082458 | 0.42574957 | 0.87635241 | 0.00743648 |
| chr13:31211674-31275009 | 87 | 20 | 0.59573885 | 0.40643103 | 0.87322265 | 0.00793524 |
| chr16:11352227-11658331 | 87 | 20 | 1.73662818 | 1.15486875 | 2.61144605 | 0.00800824 |
| chr14:50062686-50445987 | 87 | 20 | 0.5828126 | 0.384511 | 0.88338311 | 0.01094918 |
| chr14:49113809-52311409 | 87 | 20 | 0.58414109 | 0.38569719 | 0.88468577 | 0.01113321 |
| chr5:128458341-128477620 | 87 | 20 | 0.62277966 | 0.4320413 | 0.89772552 | 0.01114088 |
| chr11:26341787-27574592 | 87 | 20 | 0.62642561 | 0.43439017 | 0.90335617 | 0.01227572 |
| chr13 :27611014-27767472 | 87 | 20 | 0.60392888 | 0.40672759 | 0.89674292 | 0.0124082 |
| chr3:178149984-199501827 | 87 | 20 | 1.74519295 | 1.12435404 | 2.70884288 | 0.01304737 |
| chr8:133698327-133914560 | 87 | 20 | 1.58128694 | 1.10100024 | 2.27108795 | 0.01310545 |
| chr12:34188071-44391168 | 87 | 20 | 0.60771775 | 0.40791119 | 0.90539528 | 0.01434244 |
| chr5:82803339-90714877 | 87 | 20 | 0.63164647 | 0.43563205 | 0.91585838 | 0.01536609 |
| chr5:127447382-129549383 | 87 | 20 | 0.63588704 | 0.44090621 | 0.91709373 | 0.0153851 |
| chr17:24112056-24310787 | 87 | 20 | 0.59117363 | 0.3861159 | 0.90513305 | 0.01558137 |
| chr17:24936541-25468309 | 87 | 20 | 0.59908858 | 0.39472227 | 0.90926497 | 0.01609328 |
| chr11:1-52900000 | 87 | 20 | 0.64643605 | 0.45270111 | 0.92308049 | 0.01638076 |
| chr3:91700000-199501827 | 87 | 20 | 1.69688191 | 1.10115839 | 2.61489013 | 0.01654244 |
| chr8:94587173-100685605 | 87 | 20 | 1.53596064 | 1.08103886 | 2.18232218 | 0.01662991 |
| chr4:71492582-75938920 | 87 | 20 | 1.67234823 | 1.09637029 | 2.55091607 | 0.01698412 |
| chr12:38788913-42596599 | 87 | 20 | 0.63047902 | 0.43154294 | 0.92112223 | 0.01709149 |
| chr13 :46362859-48209064 | 87 | 20 | 0.6742035 | 0.48314021 | 0.94082493 | 0.02040896 |
| chr4:73526461-75252649 | 87 | 20 | 1.648446 | 1.08020487 | 2.51561002 | 0.02046512 |
| chr8:101163387-103693879 | 87 | 20 | 1.51847532 | 1.06335225 | 2.16839461 | 0.0215685 |
| chr8:29285985-30473918 | 87 | 20 | 0.60472111 | 0.39373841 | 0.9287578 | 0.02158688 |
| chr8:81242335-81979194 | 87 | 20 | 1.50165955 | 1.05418716 | 2.13907121 | 0.02430357 |
| chr13: 16000000-114142980 | 87 | 20 | 0.61967461 | 0.40834369 | 0.94037605 | 0.02452212 |
| chr7:54899301-55275419 | 87 | 20 | 0.60059848 | 0.38299346 | 0.94183995 | 0.02635119 |
| chr13 :27774389-30804409 | 87 | 20 | 0.64436279 | 0.43578114 | 0.95277966 | 0.02763984 |
| chr16:1-38200000 | 87 | 20 | 1.62798189 | 1.05209583 | 2.51909091 | 0.02867219 |
| chr8:82099049-82634968 | 87 | 20 | 1.48631674 | 1.03938303 | 2.12543154 | 0.02988343 |
| chr17:26185485-27216066 | 87 | 20 | 0.61153946 | 0.39221962 | 0.95349772 | 0.02999986 |
| chr8:116186189-120600761 | 87 | 20 | 1.48667259 | 1.03838577 | 2.1284916 | 0.03033549 |
| chr11:1-1391954 | 87 | 20 | 0.65339739 | 0.441058 | 0.96796373 | 0.03380867 |
| chr5:57754754-59053198 | 87 | 20 | 0.64148695 | 0.42548535 | 0.96714379 | 0.03405238 |
| chr5:58300629-59320301 | 87 | 20 | 0.64148695 | 0.42548535 | 0.96714379 | 0.03405238 |
| chr5:81303630-82685333 | 87 | 20 | 0.66322944 | 0.45228608 | 0.97255545 | 0.03551439 |
| chr17:28881201-29652449 | 87 | 20 | 0.63526589 | 0.41551497 | 0.97123518 | 0.03619642 |
| chr9:71280626-72167093 | 87 | 20 | 1.77751143 | 1.0288282 | 3.07101504 | 0.03922333 |
| chr5:58300629-62108926 | 87 | 20 | 0.64569592 | 0.42597783 | 0.97874394 | 0.0392835 |
| chr8:45200000-146274826 | 87 | 20 | 1.44811988 | 1.01793523 | 2.06010277 | 0.0395128 |
| chr11:52900000-134452384 | 87 | 20 | 0.64727208 | 0.42768198 | 0.97960908 | 0.03964722 |
| chr8:22125332-30139123 | 87 | 20 | 0.62630159 | 0.39848151 | 0.9843711 | 0.04253541 |
| chr22:36048950-38681384 | 87 | 20 | 0.66234059 | 0.44389067 | 0.9882953 | 0.04363031 |
| chr22:37740208-38258806 | 87 | 20 | 0.66234059 | 0.44389067 | 0.9882953 | 0.04363031 |
| chr5:56146657-56594666 | 87 | 20 | 0.64550243 | 0.41967956 | 0.99283698 | 0.0462954 |
| chr12:20728852-21227330 | 87 | 20 | 0.6620867 | 0.43622571 | 1.00488988 | 0.05273926 |
| chr17:1-22200000 | 87 | 20 | 0.64798842 | 0.41756281 | 1.00557086 | 0.05296747 |
| chr20:59868877-61137250 | 87 | 20 | 1.47900152 | 0.99494869 | 2.19855105 | 0.05299926 |
| chr8:128414451-128718816 | 87 | 20 | 1.44111423 | 0.99360841 | 2.09016974 | 0.0540838 |
| chr8:128774432-128849112 | 87 | 20 | 1.44111423 | 0.99360841 | 2.09016974 | 0.0540838 |
| chr1:114889793-115149999 | 87 | 20 | 0.67553864 | 0.45179384 | 1.01009002 | 0.05599804 |
| chr9:235706-8362053 | 87 | 20 | 1.83168127 | 0.98455794 | 3.40767786 | 0.05602639 |
| chr20:27100000-62435964 | 87 | 20 | 1.45312129 | 0.98861147 | 2.13588608 | 0.05721303 |
| chr12:97551177-99047626 | 87 | 20 | 0.67082774 | 0.44271463 | 1.01647839 | 0.05971622 |
| chr12:44599181-47397048 | 87 | 20 | 0.71194589 | 0.49885106 | 1.01606871 | 0.06118927 |
| chr20:33386980-33969561 | 87 | 20 | 1.45288227 | 0.97962087 | 2.15477942 | 0.06322921 |
| chr5:76408288-81082828 | 87 | 20 | 0.68809452 | 0.46289522 | 1.02285365 | 0.06456688 |
| chr20:34179462-45265004 | 87 | 20 | 1.45262443 | 0.97556618 | 2.16296729 | 0.0660362 |
| chr20:61329497-62435964 | 87 | 20 | 1.44970816 | 0.97419023 | 2.15733403 | 0.06709524 |
| chr11:101433436-102134907 | 87 | 20 | 0.63502995 | 0.38997115 | 1.03408429 | 0.06796463 |
| chr17:7471230-7717938 | 87 | 20 | 0.64741112 | 0.40539353 | 1.03391182 | 0.06870808 |
| chr12:95089777-95350380 | 87 | 20 | 0.67939721 | 0.44774018 | 1.03091167 | 0.06923667 |
| chr17:18837023-19933105 | 87 | 20 | 0.66775973 | 0.42628643 | 1.04601747 | 0.07781715 |
| chr5:63292034-63701397 | 87 | 20 | 0.67848185 | 0.44082536 | 1.04426304 | 0.07788335 |
| chr5:130522776-135427403 | 87 | 20 | 0.7265085 | 0.50920381 | 1.0365488 | 0.07806908 |
| chr8:128823745-146264218 | 87 | 20 | 1.39591712 | 0.95977853 | 2.03024399 | 0.08095486 |
| chr17:22479313-22877776 | 87 | 20 | 0.65436107 | 0.40480967 | 1.05775242 | 0.08348391 |
| chr10:1-1042949 | 87 | 20 | 1.57160569 | 0.93743292 | 2.63479592 | 0.08636602 |
| chr10:170643-1769670 | 87 | 20 | 1.57160569 | 0.93743292 | 2.63479592 | 0.08636602 |
| chr11:82612034-85091467 | 87 | 20 | 0.67716553 | 0.4324228 | 1.06042779 | 0.08846146 |
| chr10:3099740-3205003 | 87 | 20 | 1.56843524 | 0.93128189 | 2.64150858 | 0.09059265 |
| chr12: 18393150-19784941 | 87 | 20 | 0.69549413 | 0.4550619 | 1.06295889 | 0.09337579 |
| chr11:104262627-109547776 | 87 | 20 | 0.65636542 | 0.40073634 | 1.0750599 | 0.0944323 |
| chr8:11575198-11708771 | 87 | 20 | 0.69262469 | 0.45013494 | 1.06574478 | 0.09484646 |
| chr5:63838208-66501179 | 87 | 20 | 0.68495895 | 0.43919702 | 1.06824215 | 0.09515235 |
| chr5:52119531-58183162 | 87 | 20 | 0.69040608 | 0.44325757 | 1.07535794 | 0.10129317 |
| chr8:42006632-42404492 | 87 | 20 | 1.36990088 | 0.93865695 | 1.99926974 | 0.10273033 |
| chr11:76710709-85146692 | 87 | 20 | 0.66436447 | 0.40564689 | 1.08808957 | 0.10425467 |
| chr 10:3811260-6662244 | 87 | 20 | 1.47117295 | 0.92040815 | 2.35151096 | 0.10666586 |
| chr5:68425638-72913540 | 87 | 20 | 0.72695627 | 0.49296339 | 1.07201757 | 0.10760242 |
| chr8:38252951-38460772 | 87 | 20 | 1.40930106 | 0.92627945 | 2.14420117 | 0.10908439 |
| chr2:121187279-122198612 | 87 | 20 | 1.45575316 | 0.91738881 | 2.31005356 | 0.11094227 |
| chr19:10895846-11164554 | 87 | 20 | 1.391825 | 0.92390323 | 2.0967313 | 0.11378957 |
| chr1:110339388-119426489 | 87 | 20 | 0.71932706 | 0.47765512 | 1.08327409 | 0.11478304 |
| chr9:110661885-116923292 | 87 | 20 | 1.6193674 | 0.88576716 | 2.96054189 | 0.11736917 |
| chr8:140458177-146274826 | 87 | 20 | 1.35213756 | 0.92426652 | 1.9780831 | 0.12012864 |
| chr11:85633463-87710586 | 87 | 20 | 0.70602333 | 0.45317736 | 1.09994229 | 0.12383795 |
| chr5:47700000-180857866 | 87 | 20 | 0.74235044 | 0.50749104 | 1.08589932 | 0.12471016 |
| chr10:61909390-66364553 | 87 | 20 | 0.69496226 | 0.4361166 | 1.10743903 | 0.12584355 |
| chr10:3811260-5139878 | 87 | 20 | 1.49444945 | 0.88958568 | 2.51058355 | 0.12903513 |
| chr5:73958991-76396789 | 87 | 20 | 0.72805724 | 0.48278195 | 1.0979436 | 0.12998172 |
| chr20:49438571-52269898 | 87 | 20 | 1.33335655 | 0.91848491 | 1.93562208 | 0.13031972 |
| chr9:110838572-127593510 | 87 | 20 | 1.59753846 | 0.86987274 | 2.93391091 | 0.1309237 |
| chr11:42387077-47453403 | 87 | 20 | 0.73057932 | 0.48565489 | 1.09902351 | 0.13187391 |
| chr10:89467202-90419015 | 87 | 20 | 0.68697261 | 0.42112144 | 1.12065385 | 0.13264846 |
| chr12:47446248-48387464 | 87 | 20 | 0.70848359 | 0.45192805 | 1.11068345 | 0.13300861 |
| chr12:56419524-56488685 | 87 | 20 | 0.6959536 | 0.43346625 | 1.1173913 | 0.13348914 |
| chr12:56621627-57600529 | 87 | 20 | 0.6959536 | 0.43346625 | 1.1173913 | 0.13348914 |
| chr5:68425638-71536822 | 87 | 20 | 0.73253823 | 0.48719986 | 1.10142122 | 0.13472498 |
| chr19:59066340-59471027 | 87 | 20 | 1.39602661 | 0.90082575 | 2.1634487 | 0.13552135 |
| chr10:101955-12088176 | 87 | 20 | 1.42617182 | 0.8942601 | 2.2744681 | 0.13604745 |
| chr8:41004735-41425303 | 87 | 20 | 1.33581067 | 0.91042732 | 1.95994793 | 0.13881549 |
| chr20:51603033-51989829 | 87 | 20 | 1.32881199 | 0.9118551 | 1.9364275 | 0.13895766 |
| chr10:52313829-53768264 | 87 | 20 | 0.71539449 | 0.45884973 | 1.11537449 | 0.1393856 |
| chr11 :32027116-37799354 | 87 | 20 | 0.73092552 | 0.47948491 | 1.11422091 | 0.14507157 |
| chr12:55201993-55269875 | 87 | 20 | 0.70727726 | 0.44092242 | 1.13453322 | 0.15087475 |
| chr12:53136012-55168448 | 87 | 20 | 0.70804322 | 0.44163862 | 1.13514802 | 0.15168661 |
| chr20:45719063-49181611 | 87 | 20 | 1.34080429 | 0.89517427 | 2.00827503 | 0.1548103 |
| chr12 :85072329-85674601 | 87 | 20 | 0.70256236 | 0.43187058 | 1.1429208 | 0.15505501 |
| chr9:122980237-124410900 | 87 | 20 | 1.55473454 | 0.8457706 | 2.85798473 | 0.15540283 |
| chr17:33754231-34162198 | 87 | 20 | 0.69028078 | 0.41301292 | 1.15368681 | 0.15723847 |
| chr12:76852527-77064746 | 87 | 20 | 0.70692637 | 0.43580854 | 1.14670744 | 0.15993501 |
| chr5:66513872-67633403 | 87 | 20 | 0.72514338 | 0.46303445 | 1.13562375 | 0.16024228 |
| chr19:63402921-63811651 | 87 | 20 | 1.3492536 | 0.88694292 | 2.05253939 | 0.161675 |
| chr12:94480961-96479233 | 87 | 20 | 0.72515034 | 0.46228796 | 1.13747938 | 0.16176723 |
| chr11:57344931-58658339 | 87 | 20 | 0.83207984 | 0.64254648 | 1.07752029 | 0.16336429 |
| chr17:37319013-37988602 | 87 | 20 | 0.73273902 | 0.46918538 | 1.1443376 | 0.17156599 |
| chr6:63255006-65243766 | 87 | 20 | 1.35475633 | 0.87595367 | 2.09527601 | 0.17235363 |
| chr8:42971602-72924037 | 87 | 20 | 1.30654437 | 0.88930776 | 1.91953593 | 0.17310911 |
| chr6:13729714-14088212 | 87 | 20 | 1.2972646 | 0.89152007 | 1.88766972 | 0.17384664 |
| chr17:31895171-33758810 | 87 | 20 | 0.70001477 | 0.41748792 | 1.17373619 | 0.17621869 |
| chr1:201678483-203358272 | 87 | 20 | 1.30436228 | 0.88229911 | 1.92832674 | 0.18280943 |
| chr17:35067383-35272328 | 87 | 20 | 0.73802776 | 0.47093904 | 1.15659337 | 0.18507825 |
| chr6:4058046-25852810 | 87 | 20 | 1.29788252 | 0.88058881 | 1.91292352 | 0.18769436 |
| chr19:15641747-15670750 | 87 | 20 | 1.32219788 | 0.87257383 | 2.00350638 | 0.18779085 |
| chr9:1-51800000 | 87 | 20 | 1.46145589 | 0.8295849 | 2.57460485 | 0.18908061 |
| chr19:1-28500000 | 87 | 20 | 1.37719617 | 0.85283487 | 2.22395841 | 0.19056123 |
| chr17:62318152-63890591 | 87 | 20 | 1.36253647 | 0.85678074 | 2.16683867 | 0.19123768 |
| chr8:62174237-62716885 | 87 | 20 | 1.28398001 | 0.87958584 | 1.87429651 | 0.19526219 |
| chr14:37128947-38971371 | 87 | 20 | 0.73449085 | 0.45863692 | 1.17626117 | 0.19903668 |
| chr11 :57861150-58074632 | 87 | 20 | 0.83989086 | 0.64351953 | 1.09618531 | 0.19910688 |
| chr12:131913408-132349534 | 87 | 20 | 0.74316463 | 0.47074865 | 1.1732241 | 0.20259254 |
| chr2:241477619-242951149 | 87 | 20 | 0.79577127 | 0.55827569 | 1.13429964 | 0.20652893 |
| chr12:35400000-132349534 | 87 | 20 | 0.74355323 | 0.46869462 | 1.17959838 | 0.20822451 |
| chr11:87881006-92269284 | 87 | 20 | 0.75130056 | 0.4792315 | 1.17782854 | 0.21258345 |
| chr8:1-45200000 | 87 | 20 | 0.75583602 | 0.48645168 | 1.17439843 | 0.21313242 |
| chr10:35175415-37212810 | 87 | 20 | 0.7467533 | 0.47046106 | 1.18530637 | 0.21542197 |
| chr9:51800000-140273252 | 87 | 20 | 1.42057567 | 0.81511621 | 2.47576385 | 0.21546477 |
| chr20:41621022-45719023 | 87 | 20 | 1.30031601 | 0.85742796 | 1.97196942 | 0.2164594 |
| chr11:130280899-134452384 | 87 | 20 | 0.75151447 | 0.47763963 | 1.182427 | 0.2167083 |
| chr20:40134806-41603948 | 87 | 20 | 1.31182915 | 0.85232288 | 2.01906549 | 0.21732196 |
| chr15:28193434-29022600 | 87 | 20 | 0.77508615 | 0.51619056 | 1.16383092 | 0.21927893 |
| chr15:29020705-31945591 | 87 | 20 | 0.77508615 | 0.51619056 | 1.16383092 | 0.21927893 |
| chr14:23609961-31698685 | 87 | 20 | 0.75528834 | 0.4821227 | 1.18322678 | 0.22043141 |
| chr9:7161607-12713130 | 87 | 20 | 1.42514394 | 0.8075946 | 2.5149193 | 0.22150449 |
| chr9:12683435-13935586 | 87 | 20 | 1.42514394 | 0.8075946 | 2.5149193 | 0.22150449 |
| chr9:14071847-14900353 | 87 | 20 | 1.42514394 | 0.8075946 | 2.5149193 | 0.22150449 |
| chr9:130999928-132303780 | 87 | 20 | 1.50175629 | 0.77339946 | 2.91605059 | 0.22974356 |
| chr6:60500000-170899992 | 87 | 20 | 0.77854917 | 0.51684479 | 1.17276756 | 0.23109164 |
| chr16:73739922-78192112 | 87 | 20 | 0.74120303 | 0.45379688 | 1.21063401 | 0.23155024 |
| chr16:76685816-78205652 | 87 | 20 | 0.74120303 | 0.45379688 | 1.21063401 | 0.23155024 |
| chr16:79132355-80549399 | 87 | 20 | 0.74120303 | 0.45379688 | 1.21063401 | 0.23155024 |
| chr16:80588753-82633263 | 87 | 20 | 0.74120303 | 0.45379688 | 1.21063401 | 0.23155024 |
| chr16:80759878-82408573 | 87 | 20 | 0.74120303 | 0.45379688 | 1.21063401 | 0.23155024 |
| chr7:3046420-4279470 | 87 | 20 | 1.28322084 | 0.85024998 | 1.93667248 | 0.23504026 |
| chr10:54201467-57779853 | 87 | 20 | 0.7581844 | 0.4783153 | 1.20180889 | 0.23886511 |
| chr11:55503467-56066044 | 87 | 20 | 0.78789359 | 0.52935823 | 1.17269606 | 0.2400496 |
| chr22:11800000-49691432 | 87 | 20 | 0.78481719 | 0.51818586 | 1.18864305 | 0.25260927 |
| chr10:75957348-77987139 | 87 | 20 | 0.72747297 | 0.42146237 | 1.25566826 | 0.25325475 |
| chr11:69588488-69842871 | 87 | 20 | 1.27678293 | 0.83896513 | 1.94307795 | 0.25410307 |
| chr6:76630464-105342994 | 87 | 20 | 0.78232823 | 0.51282007 | 1.19347407 | 0.25462631 |
| chr4:54471680-55980061 | 87 | 20 | 1.38106574 | 0.79188761 | 2.40860263 | 0.25524105 |
| chr10:31648148-32903498 | 87 | 20 | 0.77122262 | 0.4922099 | 1.20839569 | 0.25688003 |
| chr11:110631916-112776199 | 87 | 20 | 0.75697746 | 0.46234038 | 1.23937883 | 0.26837273 |
| chr9:21489625-22474701 | 87 | 20 | 1.373531 | 0.7795439 | 2.42011695 | 0.27211087 |
| chr9:23819824-26891068 | 87 | 20 | 1.373531 | 0.7795439 | 2.42011695 | 0.27211087 |
| chr9:26573226-29470065 | 87 | 20 | 1.373531 | 0.7795439 | 2.42011695 | 0.27211087 |
| chr2:159800558-163403274 | 87 | 20 | 1.30707335 | 0.80972447 | 2.10990382 | 0.27304381 |
| chr2:164158152-168812365 | 87 | 20 | 1.30707335 | 0.80972447 | 2.10990382 | 0.27304381 |
| chr2:173927807-175001974 | 87 | 20 | 1.30707335 | 0.80972447 | 2.10990382 | 0.27304381 |
| chr3:116900556-120107320 | 87 | 20 | 1.38785302 | 0.76686416 | 2.51170431 | 0.27884258 |
| chr11:107086196-116175885 | 87 | 20 | 0.75897281 | 0.4604993 | 1.25090252 | 0.27933183 |
| chr5:135493104-138988200 | 87 | 20 | 0.8113317 | 0.55408843 | 1.18800376 | 0.28257224 |
| chr12:1-35400000 | 87 | 20 | 0.79261737 | 0.51816939 | 1.2124265 | 0.28384344 |
| chr16:82644872-85172804 | 87 | 20 | 0.76393399 | 0.46499573 | 1.25505484 | 0.28774778 |
| chr13: 104916365-104941384 | 87 | 20 | 0.78884238 | 0.50706292 | 1.22720923 | 0.29283116 |
| chr6:38787570-41935171 | 87 | 20 | 1.25186115 | 0.82147996 | 1.90772314 | 0.29598707 |
| chr7:65877239-79629882 | 87 | 20 | 0.78276486 | 0.49332713 | 1.24201731 | 0.29842657 |
| chr6:161612277-163134099 | 87 | 20 | 0.7979276 | 0.51898224 | 1.226802 | 0.30368214 |
| chr11:92342437-95765250 | 87 | 20 | 0.78600802 | 0.49613821 | 1.2452349 | 0.30503404 |
| chr7:3516940-6019790 | 87 | 20 | 1.23952787 | 0.81974068 | 1.87428705 | 0.30876598 |
| chr10:7244255-12332593 | 87 | 20 | 1.25488774 | 0.80692665 | 1.95153207 | 0.31356207 |
| chr9:34233377-35934224 | 87 | 20 | 1.32620414 | 0.75958236 | 2.31550588 | 0.32076902 |
| chr1:3756302-6867390 | 87 | 20 | 0.84097101 | 0.5965739 | 1.18548975 | 0.32282726 |
| chr1:158317017-159953843 | 87 | 20 | 1.23097302 | 0.81426322 | 1.86093947 | 0.32437121 |
| chr14:35837522-37090214 | 87 | 20 | 0.78850853 | 0.49018585 | 1.2683877 | 0.32723191 |
| chr20:29526118-29834552 | 87 | 20 | 1.28321875 | 0.77910415 | 2.11351763 | 0.32732673 |
| chr6:1-23628840 | 87 | 20 | 1.21812467 | 0.82040093 | 1.80866168 | 0.32789062 |
| chr8:2053441-6259545 | 87 | 20 | 0.79914758 | 0.5095096 | 1.25343439 | 0.32890131 |
| chr14:21961377-23608756 | 87 | 20 | 0.80458794 | 0.51823289 | 1.24917151 | 0.33268505 |
| chr6:101000242-121511318 | 87 | 20 | 0.80587461 | 0.52047115 | 1.24778078 | 0.33326161 |
| chr13:106620319-107317084 | 87 | 20 | 0.79954402 | 0.505969 | 1.26345812 | 0.33792793 |
| chr13:108477140-110084607 | 87 | 20 | 0.79954402 | 0.505969 | 1.26345812 | 0.33792793 |
| chr13: 109973420-114110898 | 87 | 20 | 0.79954402 | 0.505969 | 1.26345812 | 0.33792793 |
| chr13: 111767404-114142980 | 87 | 20 | 0.79954402 | 0.505969 | 1.26345812 | 0.33792793 |
| chr13: 112030830-113770959 | 87 | 20 | 0.79954402 | 0.505969 | 1.26345812 | 0.33792793 |
| chr6:106604408-107782614 | 87 | 20 | 0.80095754 | 0.50837131 | 1.26193783 | 0.33861103 |
| chr6:54352087-55392731 | 87 | 20 | 1.26452522 | 0.78133881 | 2.04651811 | 0.33934776 |
| chr2:1-15244284 | 87 | 20 | 1.23656143 | 0.79677663 | 1.91908764 | 0.34370037 |
| chr9:37861914-38370450 | 87 | 20 | 1.31108001 | 0.74695627 | 2.3012469 | 0.3453837 |
| chr15:75011134-75564998 | 87 | 20 | 1.33918968 | 0.72939965 | 2.45877416 | 0.34612623 |
| chr1:151026302-152973244 | 87 | 20 | 1.20991319 | 0.81199978 | 1.80282059 | 0.34903048 |
| chr6:116369389-117096650 | 87 | 20 | 0.80964663 | 0.5199149 | 1.26083645 | 0.35011562 |
| chr8:39008109-41238710 | 87 | 20 | 1.22424303 | 0.80045795 | 1.87239193 | 0.35067521 |
| chr6:149442557-149779188 | 87 | 20 | 0.81352123 | 0.52444152 | 1.26194582 | 0.35687247 |
| chr9:36365710-37139941 | 87 | 20 | 1.29596501 | 0.74620876 | 2.25074456 | 0.3573015 |
| chr4:76623381-79684447 | 87 | 20 | 1.25808644 | 0.77083736 | 2.05332743 | 0.35830755 |
| chr6:143157329-144194971 | 87 | 20 | 0.81799246 | 0.53091814 | 1.2602916 | 0.362313 |
| chr1:162199047-163197517 | 87 | 20 | 1.21137422 | 0.80148216 | 1.83089228 | 0.3628735 |
| chr6:117109043-117359993 | 87 | 20 | 0.81682956 | 0.52437742 | 1.27238609 | 0.37094563 |
| chr6:119295855-120272161 | 87 | 20 | 0.81682956 | 0.52437742 | 1.27238609 | 0.37094563 |
| chr21:38584860-42033506 | 87 | 20 | 1.57927745 | 0.57756189 | 4.31835497 | 0.3732618 |
| chr17:63942109-65847254 | 87 | 20 | 1.24346977 | 0.7659108 | 2.01879522 | 0.3781404 |
| chr18:13905410-14018535 | 87 | 20 | 0.82862129 | 0.54453876 | 1.26090792 | 0.38013492 |
| chr19:1-526082 | 87 | 20 | 1.22490159 | 0.77673917 | 1.93164443 | 0.38273724 |
| chr19:1818912-2004994 | 87 | 20 | 1.22490159 | 0.77673917 | 1.93164443 | 0.38273724 |
| chr22:45488286-49691432 | 87 | 20 | 0.82397838 | 0.53273245 | 1.27444905 | 0.38424797 |
| chrl5:31945721-37862331 | 87 | 20 | 0.82976858 | 0.54473815 | 1.2639392 | 0.38480237 |
| chr21:46690655-46902240 | 87 | 20 | 1.60924082 | 0.54321441 | 4.76728158 | 0.390548 |
| chr21:44316476-46902240 | 87 | 20 | 1.6032032 | 0.54444703 | 4.72086424 | 0.39166999 |
| chr18:44506813-45038631 | 87 | 20 | 0.83860528 | 0.5571928 | 1.26214628 | 0.3987626 |
| chr18:46172638-49935241 | 87 | 20 | 0.83860528 | 0.5571928 | 1.26214628 | 0.3987626 |
| chr22:20517661-21169423 | 87 | 20 | 1.26210867 | 0.73374842 | 2.17093251 | 0.40023189 |
| chr2:61834240-63151654 | 87 | 20 | 1.21080154 | 0.77526266 | 1.89102409 | 0.40039958 |
| chr12:1-1311104 | 87 | 20 | 0.84248876 | 0.56379968 | 1.25893527 | 0.4029601 |
| chr11:68230745-68388280 | 87 | 20 | 0.79541463 | 0.4645613 | 1.36189655 | 0.40415587 |
| chr1:163619952-164752515 | 87 | 20 | 1.19096936 | 0.78900562 | 1.797716 | 0.40546017 |
| chr16:85920445-87225756 | 87 | 20 | 0.81653892 | 0.50619745 | 1.31714574 | 0.40608462 |
| chr16:87232502-87749670 | 87 | 20 | 0.81653892 | 0.50619745 | 1.31714574 | 0.40608462 |
| chr16:88436931-88827254 | 87 | 20 | 0.81653892 | 0.50619745 | 1.31714574 | 0.40608462 |
| chr7:16017926-18944036 | 87 | 20 | 1.1960557 | 0.78246334 | 1.82826358 | 0.40828545 |
| chr14:1-29140968 | 87 | 20 | 0.82940322 | 0.53215241 | 1.292693 | 0.40874065 |
| chr7:1-59100000 | 87 | 20 | 1.18507918 | 0.78714377 | 1.78418827 | 0.41596795 |
| chr3:41866-18694914 | 87 | 20 | 1.19444562 | 0.7770205 | 1.83611674 | 0.41797444 |
| chrl7:36989609-37319012 | 87 | 20 | 0.8285265 | 0.52330309 | 1.31177549 | 0.42233584 |
| chr3:66644057-68118027 | 87 | 20 | 1.23596241 | 0.73460531 | 2.07948821 | 0.42482441 |
| chr19:7321723-7480918 | 87 | 20 | 1.20174669 | 0.75876266 | 1.90335554 | 0.43345097 |
| chr19:21788507-34401877 | 87 | 20 | 1.2060608 | 0.7544742 | 1.92794221 | 0.43373036 |
| chr22:15272143-16594738 | 87 | 20 | 1.23802266 | 0.72218873 | 2.12229857 | 0.43749611 |
| chr 10:74560456-82020637 | 87 | 20 | 0.79149462 | 0.43653909 | 1.43506904 | 0.44118236 |
| chr4:41057559-42353879 | 87 | 20 | 0.8329131 | 0.52191347 | 1.32923229 | 0.44331635 |
| chr4:83634873-83961360 | 87 | 20 | 1.2082908 | 0.74462522 | 1.96067312 | 0.44363645 |
| chr3:1-2121282 | 87 | 20 | 1.17963394 | 0.76799249 | 1.81191383 | 0.4505794 |
| chr12:63329154-63614508 | 87 | 20 | 0.82874885 | 0.50837062 | 1.35103138 | 0.45125317 |
| chr17:44673157-45060263 | 87 | 20 | 1.21080519 | 0.73582654 | 1.99238426 | 0.45159003 |
| chr6:1-60500000 | 87 | 20 | 1.16545937 | 0.78149313 | 1.73807738 | 0.45272393 |
| chr5:139008130-142795270 | 87 | 20 | 0.8616424 | 0.58227876 | 1.27503814 | 0.45641196 |
| chr3:58626894-61524607 | 87 | 20 | 1.2182806 | 0.72312922 | 2.0524791 | 0.45815323 |
| chr6:157695838-157823719 | 87 | 20 | 0.85123676 | 0.55444503 | 1.30689967 | 0.46152961 |
| chr16:38200000-88827254 | 87 | 20 | 0.82505798 | 0.49392729 | 1.37817991 | 0.46257549 |
| chr4:37121738-38804810 | 87 | 20 | 0.8385174 | 0.52400769 | 1.34179602 | 0.46280086 |
| chr19:53372336-53572212 | 87 | 20 | 1.17418196 | 0.76347135 | 1.80583497 | 0.46470141 |
| chr15:56490060-57176541 | 87 | 20 | 1.24242972 | 0.69390311 | 2.22456359 | 0.46515075 |
| chr15:57184612-61461128 | 87 | 20 | 1.24242972 | 0.69390311 | 2.22456359 | 0.46515075 |
| chr15:61583863-61913200 | 87 | 20 | 1.24242972 | 0.69390311 | 2.22456359 | 0.46515075 |
| chr15:65899096-66370691 | 87 | 20 | 1.24242972 | 0.69390311 | 2.22456359 | 0.46515075 |
| chr4:155375138-160500747 | 87 | 20 | 0.84307723 | 0.53276512 | 1.33413241 | 0.46604896 |
| chr7:86091574-88424037 | 87 | 20 | 1.17257184 | 0.76238417 | 1.80345391 | 0.46858067 |
| chr18:3240326-5937123 | 87 | 20 | 1.30662014 | 0.63229522 | 2.70009346 | 0.47019136 |
| chr18:32113759-32649538 | 87 | 20 | 0.85821683 | 0.56612134 | 1.30102166 | 0.47134633 |
| chr18:16100000-76117153 | 87 | 20 | 0.85791381 | 0.56376248 | 1.3055429 | 0.4743733 |
| chr4:79691766-80079606 | 87 | 20 | 1.19817592 | 0.73006513 | 1.9664349 | 0.47444004 |
| chr10:28141103-31360860 | 87 | 20 | 0.84057146 | 0.52137089 | 1.35519722 | 0.47603992 |
| chr18:3200634-3858775 | 87 | 20 | 1.29493375 | 0.62645755 | 2.67672314 | 0.48540976 |
| chr11:76699529-78005085 | 87 | 20 | 0.81963992 | 0.46753851 | 1.43690753 | 0.48743932 |
| chr15:88253886-88997242 | 87 | 20 | 1.22420035 | 0.69050233 | 2.17040036 | 0.48869471 |
| chr16:69398793-73198518 | 87 | 20 | 0.83517621 | 0.50135685 | 1.39126314 | 0.48909797 |
| chr1:26377344-27532551 | 87 | 20 | 0.8791472 | 0.60861903 | 1.26992379 | 0.49242873 |
| chr1:27351829-27460967 | 87 | 20 | 0.8791472 | 0.60861903 | 1.26992379 | 0.49242873 |
| chr7:156893473-158821424 | 87 | 20 | 1.17886968 | 0.73537422 | 1.88983198 | 0.4943464 |
| chr17:22200000-78774742 | 87 | 20 | 0.85570186 | 0.54687541 | 1.33892596 | 0.49510412 |
| chr 1:1-124300000 | 87 | 20 | 0.87102815 | 0.58348542 | 1.30027249 | 0.49937177 |
| chr1:124300000-247249719 | 87 | 20 | 1.15111249 | 0.76501008 | 1.73208173 | 0.4996419 |
| chr15:1-24740084 | 87 | 20 | 0.85870676 | 0.55023382 | 1.34011628 | 0.50235581 |
| chr5:155686345-159845035 | 87 | 20 | 0.8753867 | 0.59247239 | 1.29339676 | 0.50398763 |
| chr11:112785528-114880322 | 87 | 20 | 0.84435958 | 0.51266356 | 1.39066465 | 0.5063415 |
| chr11:116803699-119605859 | 87 | 20 | 0.84435958 | 0.51266356 | 1.39066465 | 0.5063415 |
| chr7:83246850-83689170 | 87 | 20 | 1.15144841 | 0.75576108 | 1.75430237 | 0.51153969 |
| chr15:51593230-55368004 | 87 | 20 | 1.20932204 | 0.68089594 | 2.14784626 | 0.51665206 |
| chr12: 10732932-10778896 | 87 | 20 | 0.87558441 | 0.58562257 | 1.3091163 | 0.51735149 |
| chr5:152850499-154326721 | 87 | 20 | 0.87884712 | 0.59415464 | 1.29995157 | 0.51790267 |
| chr3:86250885-95164178 | 87 | 20 | 0.86080338 | 0.54389957 | 1.36235162 | 0.52224001 |
| chr10:24604620-28074753 | 87 | 20 | 0.8538642 | 0.52113069 | 1.39904269 | 0.53059739 |
| chrX:60000000-154913754 | 87 | 20 | 0.86961138 | 0.56163233 | 1.34647511 | 0.5311083 |
| chr5:1212750-1378766 | 87 | 20 | 0.8598503 | 0.53496494 | 1.38203926 | 0.5328698 |
| chr5:1473801-6790134 | 87 | 20 | 0.8598503 | 0.53496494 | 1.38203926 | 0.5328698 |
| chr18:1-587750 | 87 | 20 | 1.22636519 | 0.64433083 | 2.33416049 | 0.53432807 |
| chr15:37880223-38115089 | 87 | 20 | 0.87376155 | 0.56961292 | 1.34031238 | 0.53645029 |
| chr6:137619861-138589104 | 87 | 20 | 0.9220539 | 0.71096817 | 1.19581077 | 0.54066787 |
| chr6:139381116-139689363 | 87 | 20 | 0.9220539 | 0.71096817 | 1.19581077 | 0.54066787 |
| chr10:12431738-17283687 | 87 | 20 | 1.15631423 | 0.72485334 | 1.84459742 | 0.54217949 |
| chr17:45731662-46009941 | 87 | 20 | 1.16726399 | 0.70622275 | 1.92928539 | 0.54633227 |
| chr9:97183463-97625342 | 87 | 20 | 1.15600295 | 0.71121479 | 1.87895815 | 0.55859001 |
| chr16:56791457-72871981 | 87 | 20 | 0.85665077 | 0.50945078 | 1.44047388 | 0.55954113 |
| chr4:91089383-93486891 | 87 | 20 | 1.15223082 | 0.71006751 | 1.86973188 | 0.56616896 |
| chr10:73394126-73443318 | 87 | 20 | 0.85317881 | 0.49564195 | 1.46862887 | 0.56663223 |
| chr16:65206155-69392572 | 87 | 20 | 0.85940985 | 0.51156684 | 1.44377083 | 0.56703763 |
| chr17:58249995-58447640 | 87 | 20 | 1.14987748 | 0.7103908 | 1.8612547 | 0.56979059 |
| chr1:148661965-149063439 | 87 | 20 | 1.13115985 | 0.73711709 | 1.73584715 | 0.5727244 |
| chr10:19022250-42889659 | 87 | 20 | 0.86511569 | 0.52150086 | 1.43513695 | 0.57475481 |
| chr16:45546775-51138307 | 87 | 20 | 0.85913743 | 0.50535844 | 1.46058136 | 0.5749601 |
| chr4:10979549-15266111 | 87 | 20 | 0.88325392 | 0.57006027 | 1.36851755 | 0.57843121 |
| chr14:35708407-36097605 | 87 | 20 | 0.87236913 | 0.53834631 | 1.41364004 | 0.57929963 |
| chr4:50700000-191273063 | 87 | 20 | 1.14485291 | 0.70793026 | 1.85143689 | 0.58123656 |
| chr11:73357303-74035012 | 87 | 20 | 1.1427962 | 0.70916392 | 1.84158151 | 0.58349644 |
| chr4:112023722-118112513 | 87 | 20 | 0.8815264 | 0.56121254 | 1.38466042 | 0.58414843 |
| chr4:114219937-114355024 | 87 | 20 | 0.8815264 | 0.56121254 | 1.38466042 | 0.58414843 |
| chr14:34100051-35410919 | 87 | 20 | 0.87008968 | 0.52856273 | 1.43229179 | 0.58423708 |
| chr5:143171919-147191453 | 87 | 20 | 0.89526213 | 0.60214847 | 1.33105759 | 0.58455049 |
| chr14:31868274-34078694 | 87 | 20 | 0.86840822 | 0.52256538 | 1.44313585 | 0.58612458 |
| chr12:50987234-51048711 | 87 | 20 | 1.15072247 | 0.69099399 | 1.91631508 | 0.58953287 |
| chr6:135561194-135665525 | 87 | 20 | 0.93026022 | 0.71343639 | 1.21297999 | 0.59339507 |
| chr19:43177306-45393020 | 87 | 20 | 0.8864003 | 0.56905621 | 1.38071684 | 0.59383761 |
| chr19:44889764-44995371 | 87 | 20 | 0.8864003 | 0.56905621 | 1.38071684 | 0.59383761 |
| chr4:23402742-26636101 | 87 | 20 | 0.88818625 | 0.57283135 | 1.37715022 | 0.5961947 |
| chr5:147238467-151765017 | 87 | 20 | 0.89818412 | 0.60375783 | 1.33618924 | 0.59620914 |
| chr12:11410696-12118386 | 87 | 20 | 0.89464684 | 0.58955557 | 1.35762089 | 0.60085245 |
| chr2:138479322-143365272 | 87 | 20 | 0.89917037 | 0.60351797 | 1.33965746 | 0.60133736 |
| chr2:1-93300000 | 87 | 20 | 1.11969627 | 0.73238159 | 1.71183951 | 0.60168053 |
| chr21:12300000-46944323 | 87 | 20 | 1.27598254 | 0.51076375 | 3.18764097 | 0.60185983 |
| chr15:96891354-97698742 | 87 | 20 | 1.16250708 | 0.6593099 | 2.0497534 | 0.60279786 |
| chr6:129217882-131730157 | 87 | 20 | 0.93087721 | 0.71071267 | 1.21924432 | 0.60290516 |
| chr5:18240864-21565169 | 87 | 20 | 0.87476054 | 0.52685753 | 1.45239644 | 0.60498568 |
| chr10:20145174-20609292 | 87 | 20 | 1.13096107 | 0.7072199 | 1.80859299 | 0.60740842 |
| chr10:21110098-22746531 | 87 | 20 | 1.13096107 | 0.7072199 | 1.80859299 | 0.60740842 |
| chr10:22863772-24876778 | 87 | 20 | 1.13096107 | 0.7072199 | 1.80859299 | 0.60740842 |
| chr4:13238819-37565122 | 87 | 20 | 0.89057646 | 0.57169397 | 1.3873269 | 0.60836484 |
| chr16:56705000-65205296 | 87 | 20 | 0.87383649 | 0.51685393 | 1.47738107 | 0.61471958 |
| chr4:15313739-17632477 | 87 | 20 | 0.89708978 | 0.57796321 | 1.3924244 | 0.62828539 |
| chr14:1-23145193 | 87 | 20 | 0.89376248 | 0.56688174 | 1.40913229 | 0.62873858 |
| chr16:31854743-53525739 | 87 | 20 | 0.87630325 | 0.51218431 | 1.4992794 | 0.62986899 |
| chr2:135029078-135167772 | 87 | 20 | 0.90718811 | 0.61023236 | 1.34865062 | 0.6301762 |
| chr19:28500000-63811651 | 87 | 20 | 1.1120941 | 0.71993427 | 1.71786974 | 0.63202441 |
| chr20:14210829-15988895 | 87 | 20 | 0.91240729 | 0.62024914 | 1.34218172 | 0.64157209 |
| chr7:129281946-131188950 | 87 | 20 | 1.11206031 | 0.70876011 | 1.74484726 | 0.64397409 |
| chr16:45393460-45522699 | 87 | 20 | 0.88276992 | 0.51920352 | 1.50091959 | 0.64519758 |
| chr10:1-40300000 | 87 | 20 | 1.12818865 | 0.66714755 | 1.90783826 | 0.65272822 |
| chr 1:58658784-60221344 | 87 | 20 | 1.11377634 | 0.69541615 | 1.7838207 | 0.65386205 |
| chr2:32460827-55039898 | 87 | 20 | 1.10169462 | 0.71789462 | 1.69068136 | 0.65760918 |
| chr3:36286137-40063081 | 87 | 20 | 1.10066208 | 0.7197495 | 1.68316478 | 0.65808347 |
| chr4:19864333-22430289 | 87 | 20 | 0.90935657 | 0.5891534 | 1.40358923 | 0.66788281 |
| chr20:1-27100000 | 87 | 20 | 0.92210487 | 0.63363131 | 1.3419119 | 0.67182906 |
| chr2:79257340-80427237 | 87 | 20 | 1.09749424 | 0.71118468 | 1.69364392 | 0.67428924 |
| chr17:51748757-53292386 | 87 | 20 | 1.10965563 | 0.67956153 | 1.81195604 | 0.67749191 |
| chr4:151218876-154929678 | 87 | 20 | 0.90807629 | 0.57153909 | 1.44277542 | 0.6831293 |
| chr1:119996566-120303234 | 87 | 20 | 0.90496573 | 0.55181047 | 1.48413815 | 0.69237307 |
| chr1:166729757-167532460 | 87 | 20 | 1.08720683 | 0.71620944 | 1.65038132 | 0.69460227 |
| chr12:64149851-66323376 | 87 | 20 | 1.10136867 | 0.67308434 | 1.8021708 | 0.70075964 |
| chr12:64461446-64607139 | 87 | 20 | 1.10136867 | 0.67308434 | 1.8021708 | 0.70075964 |
| chr15:35140533-43473382 | 87 | 20 | 0.91783998 | 0.59218616 | 1.42257671 | 0.70138024 |
| chr4:30331135-30753569 | 87 | 20 | 0.91751379 | 0.58459507 | 1.44002507 | 0.70815856 |
| chr5:43612947-44998256 | 87 | 20 | 1.0923449 | 0.68449312 | 1.7432131 | 0.711099 |
| chr7:137042370-158819393 | 87 | 20 | 1.08888996 | 0.69294059 | 1.71108659 | 0.71191119 |
| chr18:1-16100000 | 87 | 20 | 1.09562889 | 0.67176975 | 1.78692575 | 0.71441835 |
| chr10:17311250-19896508 | 87 | 20 | 1.08989638 | 0.67656574 | 1.75574086 | 0.72345094 |
| chr4:186684565-191273063 | 87 | 20 | 0.9236481 | 0.59224169 | 1.44050282 | 0.72613151 |
| chr5:176660805-180009206 | 87 | 20 | 0.92873419 | 0.61241373 | 1.4084387 | 0.72785244 |
| chr6:110391-1254918 | 87 | 20 | 1.07359703 | 0.71955093 | 1.60184711 | 0.7279591 |
| chr6:1543157-2570302 | 87 | 20 | 1.07359703 | 0.71955093 | 1.60184711 | 0.7279591 |
| chr3:70107050-74383584 | 87 | 20 | 1.09130343 | 0.66501208 | 1.79085944 | 0.72954633 |
| chr1:71284749-74440273 | 87 | 20 | 1.08322925 | 0.68843566 | 1.70442307 | 0.72957879 |
| chr6:42772314-43039595 | 87 | 20 | 1.08055258 | 0.69546912 | 1.678858 | 0.73039773 |
| chr6:43556800-44361368 | 87 | 20 | 1.08055258 | 0.69546912 | 1.678858 | 0.73039773 |
| chr5:174477192-180857866 | 87 | 20 | 0.93201195 | 0.6146667 | 1.41319885 | 0.74025166 |
| chr5:9224578-10673735 | 87 | 20 | 0.91980504 | 0.56052767 | 1.50936582 | 0.74079452 |
| chr5:159922707-167623739 | 87 | 20 | 0.93251821 | 0.61582476 | 1.41207411 | 0.74138108 |
| chr18:17749667-22797232 | 87 | 20 | 0.93136111 | 0.60692005 | 1.42923854 | 0.74484839 |
| chr4:1-50700000 | 87 | 20 | 0.92263724 | 0.56656084 | 1.50250322 | 0.74622453 |
| chr4:162524501-164492534 | 87 | 20 | 0.92886039 | 0.59382196 | 1.45292983 | 0.74646548 |
| chr4:147085600-153449318 | 87 | 20 | 0.92719956 | 0.58616491 | 1.46665044 | 0.74664671 |
| chr13:89500014-93206506 | 87 | 20 | 0.92846341 | 0.59136494 | 1.45771968 | 0.74707734 |
| chr13 :92308911-94031607 | 87 | 20 | 0.92846341 | 0.59136494 | 1.45771968 | 0.74707734 |
| chr5:177541057-180857866 | 87 | 20 | 0.93384775 | 0.61588832 | 1.41595738 | 0.74724921 |
| chr 17:39354620-40542083 | 87 | 20 | 0.92718365 | 0.57740865 | 1.48884073 | 0.75437188 |
| chr5:1-47700000 | 87 | 20 | 0.92926977 | 0.58475978 | 1.47674709 | 0.75625867 |
| chr7:144118814-148066271 | 87 | 20 | 1.07209409 | 0.68450202 | 1.67915609 | 0.76105549 |
| chr16:54782803-56638303 | 87 | 20 | 0.92092624 | 0.53952998 | 1.57193328 | 0.76268222 |
| chr1:168438419-168880930 | 87 | 20 | 0.93774078 | 0.60930044 | 1.44322524 | 0.77012609 |
| chr1:169549478-170484405 | 87 | 20 | 0.93774078 | 0.60930044 | 1.44322524 | 0.77012609 |
| chr1:174611641-175371076 | 87 | 20 | 0.93839431 | 0.61191333 | 1.43906633 | 0.77069584 |
| chr3:1-91700000 | 87 | 20 | 1.06363302 | 0.69277151 | 1.63302789 | 0.77793494 |
| chr12:27342867-29425476 | 87 | 20 | 1.06235307 | 0.69142833 | 1.63226469 | 0.78252309 |
| chr16:51646446-54466783 | 87 | 20 | 0.92828515 | 0.53898438 | 1.59877233 | 0.78848061 |
| chr5:39149695-41885012 | 87 | 20 | 1.06425443 | 0.66658447 | 1.69916574 | 0.79418557 |
| chr19:52031294-53331283 | 87 | 20 | 1.05967361 | 0.68233981 | 1.64567294 | 0.79634949 |
| chr2:156889194-159797412 | 87 | 20 | 1.05881824 | 0.68002472 | 1.64861075 | 0.80027622 |
| chr12:48421630-53099317 | 87 | 20 | 0.93932815 | 0.57300072 | 1.53985385 | 0.80398726 |
| chr20:22719364-23648289 | 87 | 20 | 0.95176633 | 0.64101365 | 1.4131667 | 0.80635576 |
| chr12:25189655-25352305 | 87 | 20 | 1.05428732 | 0.69036681 | 1.61004517 | 0.8066737 |
| chr2:93300000-242951149 | 87 | 20 | 1.05221176 | 0.69575996 | 1.59128099 | 0.80943757 |
| chr7:102988499-103837783 | 87 | 20 | 1.05653331 | 0.6721754 | 1.66067165 | 0.81161706 |
| chr12:67440273-67566002 | 87 | 20 | 1.06143069 | 0.64583972 | 1.74445002 | 0.81405959 |
| chr12:68249634-68327233 | 87 | 20 | 1.06143069 | 0.64583972 | 1.74445002 | 0.81405959 |
| chr6:30420923-31893702 | 87 | 20 | 1.04942867 | 0.68825144 | 1.60014273 | 0.82263464 |
| chr15:17000000-100338915 | 87 | 20 | 1.06040323 | 0.62926684 | 1.78692877 | 0.82565686 |
| chr4:1234177-1683843 | 87 | 20 | 0.94879782 | 0.59019262 | 1.52529408 | 0.82821693 |
| chrX:1-60000000 | 87 | 20 | 0.94879782 | 0.59019262 | 1.52529408 | 0.82821693 |
| chr19:37265855-38702029 | 87 | 20 | 0.95574444 | 0.62229419 | 1.46787075 | 0.83619521 |
| chr12:29425476-31105002 | 87 | 20 | 1.0464538 | 0.6767972 | 1.61801138 | 0.83818314 |
| chr1:43945805-44960161 | 87 | 20 | 1.04652038 | 0.67021565 | 1.63410823 | 0.84148811 |
| chr1:46541957-46555032 | 87 | 20 | 1.04652038 | 0.67021565 | 1.63410823 | 0.84148811 |
| chr4:104889590-106814504 | 87 | 20 | 1.04660246 | 0.66080243 | 1.65764631 | 0.84606706 |
| chr12:30999223-32594050 | 87 | 20 | 1.04313481 | 0.67126244 | 1.62102059 | 0.85106355 |
| chr12:33156845-33480710 | 87 | 20 | 1.04313481 | 0.67126244 | 1.62102059 | 0.85106355 |
| chr4:164612456-170167997 | 87 | 20 | 0.9576758 | 0.60956347 | 1.50458973 | 0.85117425 |
| chr7:115981465-116676953 | 87 | 20 | 0.9596709 | 0.62207573 | 1.48047608 | 0.85236275 |
| chr9:137859478-140273252 | 87 | 20 | 1.04045032 | 0.6847096 | 1.58101604 | 0.85264224 |
| chr7:141592807-142264966 | 87 | 20 | 1.04292291 | 0.66844185 | 1.62719942 | 0.85309189 |
| chr13:72212826-74986423 | 87 | 20 | 0.96533637 | 0.66126641 | 1.40922675 | 0.85497973 |
| chr4:170251982-170915637 | 87 | 20 | 0.96095771 | 0.61160637 | 1.50985955 | 0.86284846 |
| chr4:3220565-5553626 | 87 | 20 | 0.96125031 | 0.5965134 | 1.54900487 | 0.87103675 |
| chr18:75796373-76117153 | 87 | 20 | 1.03610943 | 0.66763021 | 1.60796012 | 0.87430377 |
| chr5:172591744-174888201 | 87 | 20 | 0.96691632 | 0.63338264 | 1.47608586 | 0.87613383 |
| chr4:5577784-10295484 | 87 | 20 | 0.96566939 | 0.59817582 | 1.55893524 | 0.88632162 |
| chr19:49818193-50114786 | 87 | 20 | 1.03251515 | 0.66350072 | 1.60676167 | 0.88722523 |
| chr1:118282114-118807006 | 87 | 20 | 0.96662135 | 0.60357796 | 1.54803008 | 0.88764123 |
| chr7:92829306-132566935 | 87 | 20 | 1.03267388 | 0.66014504 | 1.61542582 | 0.88800126 |
| chr5:45312870-49697231 | 87 | 20 | 1.03372667 | 0.64741565 | 1.65054836 | 0.88950161 |
| chr22:22437934-22507511 | 87 | 20 | 1.03221691 | 0.63171984 | 1.68662068 | 0.89928074 |
| chr22:23139372-23249329 | 87 | 20 | 1.03221691 | 0.63171984 | 1.68662068 | 0.89928074 |
| chr1:41717036-43943775 | 87 | 20 | 1.02768764 | 0.65946166 | 1.60152128 | 0.90396183 |
| chr11:74085123-74337880 | 87 | 20 | 0.97046245 | 0.58642936 | 1.60598605 | 0.90712835 |
| chr6:31910383-31915520 | 87 | 20 | 1.02538472 | 0.66203146 | 1.58816295 | 0.91058597 |
| chr19:3 9664720-3 9962225 | 87 | 20 | 0.97566991 | 0.63232029 | 1.50545821 | 0.91137511 |
| chr19:39677306-41155075 | 87 | 20 | 0.97566991 | 0.63232029 | 1.50545821 | 0.91137511 |
| chr5:167651670-171366482 | 87 | 20 | 0.97828038 | 0.64242225 | 1.48972502 | 0.9184875 |
| chr15:49136093-51017946 | 87 | 20 | 1.02477663 | 0.61448339 | 1.70902447 | 0.9252748 |
| chr7:89924533-98997268 | 87 | 20 | 1.02074354 | 0.65293493 | 1.59574459 | 0.92823771 |
| chr2:204533830-206266883 | 87 | 20 | 0.9809187 | 0.63956347 | 1.50446599 | 0.92964968 |
| chr11:74091491-74180816 | 87 | 20 | 0.97879061 | 0.59220624 | 1.61773212 | 0.93335718 |
| chr3:43362938-44130419 | 87 | 20 | 0.98261158 | 0.64605831 | 1.49448664 | 0.93465452 |
| chr2:154436672-155018734 | 87 | 20 | 1.01647799 | 0.65432123 | 1.57908296 | 0.94202917 |
| chr17:77653047-78605474 | 87 | 20 | 1.01772292 | 0.62282273 | 1.66300922 | 0.9441 |
| chr17:78087533-78774742 | 87 | 20 | 1.01772292 | 0.62282273 | 1.66300922 | 0.9441 |
| chr21:16248572-25859193 | 87 | 20 | 1.01939788 | 0.59476504 | 1.74719759 | 0.94428372 |
| chr4:174326479-176135905 | 87 | 20 | 1.01433106 | 0.60836964 | 1.6911881 | 0.95649241 |
| chr4:176791083-177486490 | 87 | 20 | 1.01433106 | 0.60836964 | 1.6911881 | 0.95649241 |
| chr7:59100000-158821424 | 87 | 20 | 1.01022784 | 0.65336492 | 1.56200656 | 0.96349717 |
| chr19:47121673-49919582 | 87 | 20 | 0.99085701 | 0.64002471 | 1.53399953 | 0.96714494 |
| chr1:241364021-247249719 | 87 | 20 | 0.99260318 | 0.65725549 | 1.49905339 | 0.97184308 |
| chr7 :8779703 8-89779004 | 87 | 20 | 1.00778641 | 0.64774414 | 1.56795467 | 0.97256425 |
| chr17:55144989-55540417 | 87 | 20 | 1.00851769 | 0.6200117 | 1.64046569 | 0.97274157 |
| chr4:175486008-184028292 | 87 | 20 | 1.00755532 | 0.60614729 | 1.67478721 | 0.97683967 |
| chr19:34975531-35098303 | 87 | 20 | 1.00485281 | 0.64917827 | 1.55539582 | 0.98267295 |
| chr1:223876038-247249719 | 87 | 20 | 0.99566217 | 0.65715386 | 1.50854042 | 0.98363887 |
| chr17:75915141-77213225 | 87 | 20 | 1.00400227 | 0.6145576 | 1.64023773 | 0.98727486 |
| chr17:70767943-71305641 | 87 | 20 | 0.99739128 | 0.61066752 | 1.62901959 | 0.99167362 |
| chr1:39907605-40263248 | 87 | 20 | 1.00173609 | 0.66790643 | 1.50241883 | 0.99330797 |
| chr19:32764651-33569283 | 87 | 20 | 0.99929175 | 0.64459055 | 1.54917567 | 0.99747287 |

In a preferred embodiment, the biological sample is selected from plasma, serum, breast milk, cerebrospinal fluid or blood samples.

In a preferred embodiment, the patient is suffering from breast cancer.

In a preferred embodiment, the breast cancer subtype is selected from: HR+/HER2-, HER2+ and triple-negative.

The fifth embodiment of the present invention refers to a kit, suitable for performing the above described methods, comprising tools and reagents for assessing the presence of CNAs in the following the DNA segments: chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 and/or chr3:58626894-61524607, preferably in the DNA segments of **Table 5, Table 6** or **Table 7**.

The sixth embodiment of the present invention refers to the use of the above defined kit for predicting the response of patients with HR+/HER2- breast cancer to a treatment comprising targeted therapy such as a CDK4/6 inhibitor and/or endocrine therapy, for the prognosis of patients with HR+/HER2- breast cancer, for monitoring patients with HR+/HER2- breast cancer, or for classifying HR+/HER2- breast cancer into biologically relevant groups which are associated with response to a treatment comprising targeted therapy such as a CDK4/6 inhibitor and/or endocrine therapy.

The last embodiment of the present invention refers to targeted therapy such as a CDK4/6 inhibitor and/or endocrine therapy for use in the treatment of patients with HR+/HER2- breast cancer, wherein the patients have been identified as responder patients according to the method described in any of the above embodiments. Alternatively, the present invention refers to a method for treating patients with HR+/HER2- breast cancer which comprises the administration of a therapeutically effective dose or amount of targeted therapy such as a CDK4/6 inhibitor and/or endocrine therapy once the patients have been identified as being responder by means of the method described in any of the above embodiments.

In a preferred embodiment the endocrine therapy comprises letrozole, anastrozole, exemestane, tamoxifen, selective estrogen receptor degraders such as fulvestrant, and targeted therapies comprises CDK4/6 inhibitors (palbociclib, abemaciclib, ribociclib or trilaciclib), PI3K/mTOR inhibitors (alpelisib, everolimus) and antibody-drug conjugates targeting HER2 (trastuzumab deruxtecan, trastuzumab duocarmazine, disitamab vedotin, ARX788 or BAT8001), HER3 (patritutumab or deruxtecan) and TROP2 (sacituzumab govitecan, datopotamab deruxtecan or SKB264) and LIV-1 (ladiratuzumab vedotin).

For the purpose of the present invention the following terms are defined:
- Copy number alterations (CNA): is a phenomenon in which sections of the genome are repeated and the number of repeats in the genome varies between individuals. CNAs are a type of structural variation: specifically, it is a type of duplication or deletion event that affects a considerable number of base pairs. CNAs can be generally categorized into two main groups: short repeats and long repeats. However, there are no clear boundaries between the two groups and the classification depends on the nature of the loci of interest. Short repeats include mainly dinucleotide repeats (two repeating nucleotides e.g. A-C-A-C-A-C...) and trinucleotide repeats. Long repeats include repeats of entire genes. This classification based on size of the repeat is the most obvious type of classification as size is an important factor in examining the types of mechanisms that most likely gave rise to the repeats, hence the likely effects of these repeats on phenotype.
- The expression "pre-established reference value" refers to a threshold value obtained after assessing the presence of CNAs in "normal DNA" segments (i.e. non-tumoral DNA) segments, which are present in the biological sample.
- The expression "score" refers to a value obtained after assessing the presence of CNAs in tumoral DNA segments which are present in the biological sample. The signal of each segment is calculated by averaging the signal of each gene within each segment. The final score is calculated by multiplying the signal of each DNA segment by its weight and summing up all of them. After processing all the values, a single score is obtained. This single value will be compared with the "pre-established reference value" to finally make clinical decisions. Particularly, if a deviation or variation of the "score" value is identified, typically a "score" value higher or lower than the "pre-established reference value", this is indicative that the patient with HR+/HER2- breast cancer will respond or not to the treatment, or that the patient will have a good or poor prognosis.

- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of'. Thus, the phrase "consisting of" indicates that the listed elements are required or mandatory, and that no other elements may be present.
- By "therapeutically effective dose or amount" is intended an amount that, when administered as described herein, brings about a positive therapeutic response in a subject suffering from breast cancer. The exact amount required will vary from subject to subject, depending on the age, and general condition of the subject, the severity of the condition being treated, mode of administration, and the like.

### Description of the figures

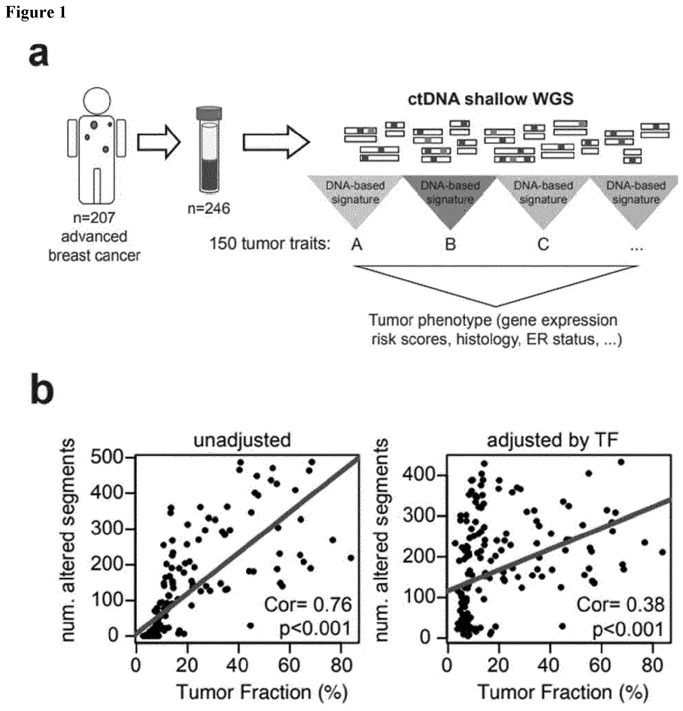

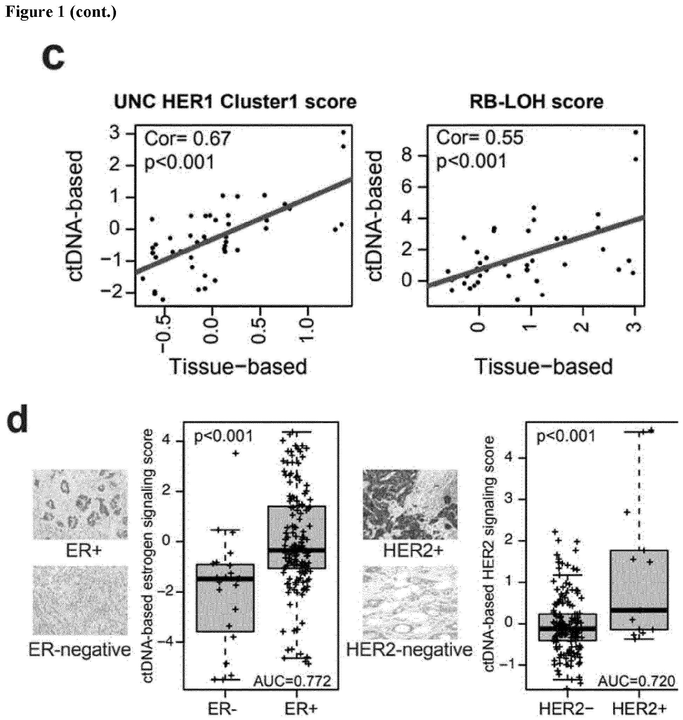

**Figure 1****. Circulating tumor DNA (ctDNA) in metastatic breast cancer.** (**a**) Plasma samples were obtained from 207 patients (174 with HR+/HER2-, 16 HER2+, 16 TNBC, 1 N/A). After purification of plasma cell-free DNA, shallow whole genome sequencing (shWGS) was performed. Using the ctDNA-based sequencing data from 514 DNA segments, 150 previously developed DNA copy number-based signatures [Xia Y, Fan C, Hoadley KA, Parker JS, Perou CM. Genetic determinants of the molecular portraits of epithelial cancers. Nature Communications 2019;10(1):5666 doi 10.1038/s41467-019-13588-2] tracking a variety of biological processes were applied in patients with a Tumor cell Fraction (TF) ≥3%. Individual scores for each signature were obtained. (**b**) Relationship between TF and number of altered DNA segments detected across 246 plasma ctDNA samples before (left) and after (right) adjusting the DNA copy-number signal by TF and ploidy using the ichorCNA tool. (**c**) Examples of correlation between the scores of two DNA-based signatures when determined in plasma versus tumor tissue. Of note, tissue samples were obtained at different timepoints than the plasma samples. (**d**) Association between the ER (left) and HER2 (right) tumor tissue status and expression of two ctDNA-based signatures tracking ER-related and HER2-related biology, respectively.

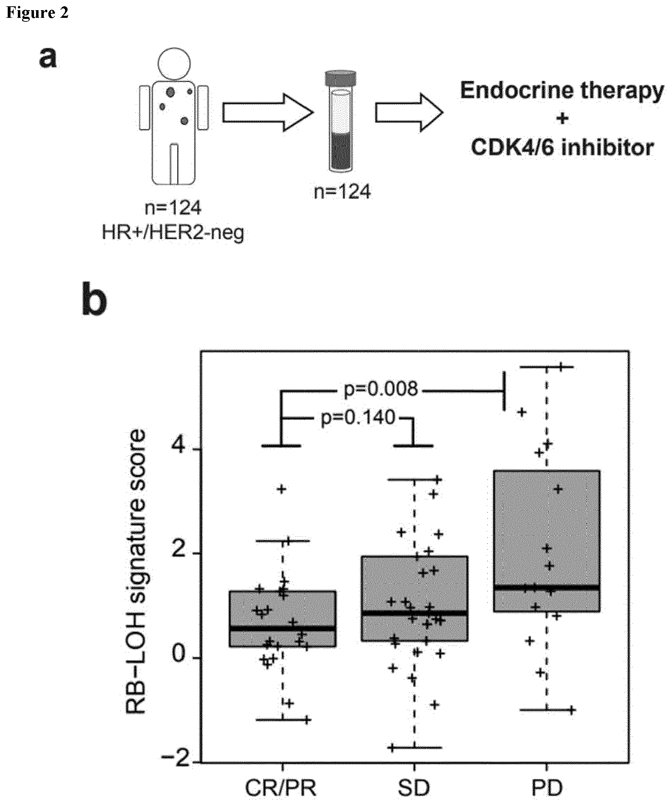

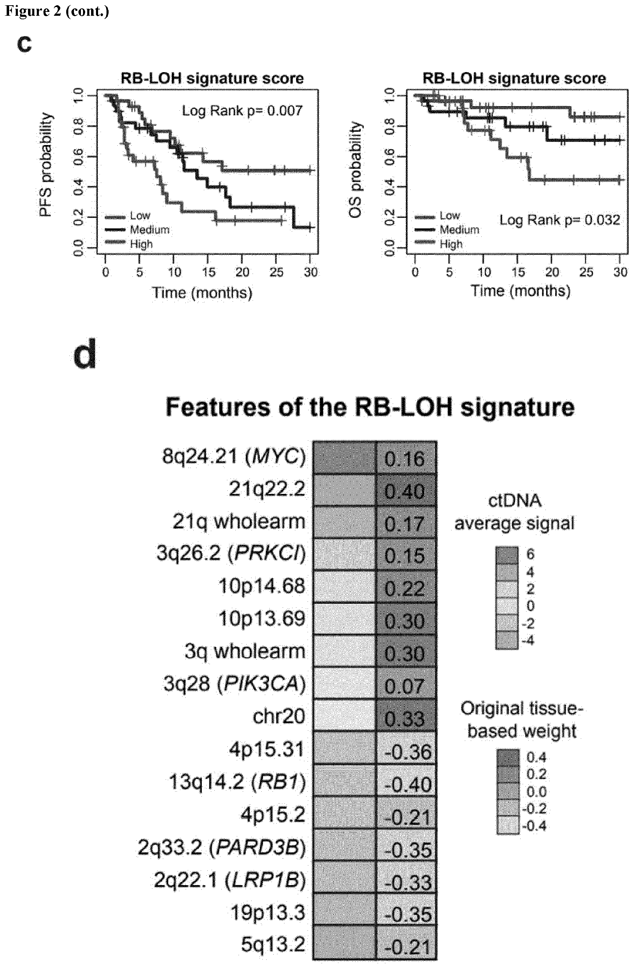

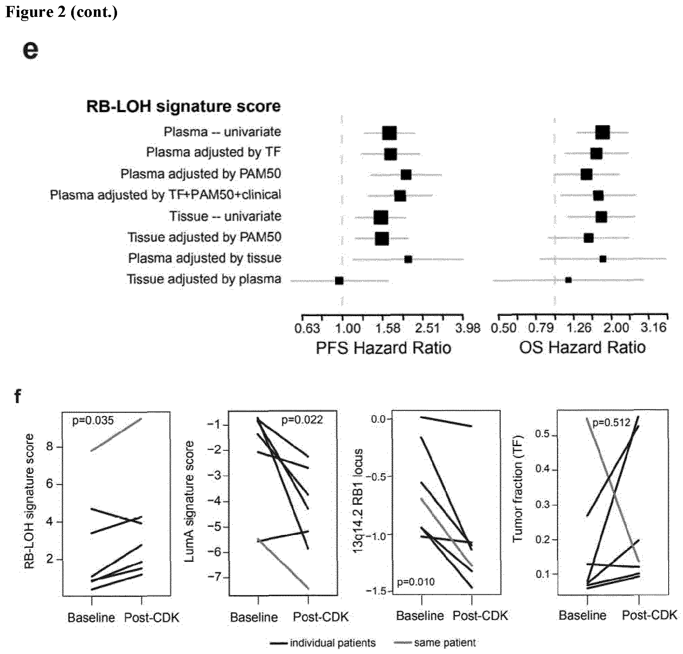

**Figure 2****. The ctDNA-based RB-LOH signature predicts clinical outcome in advanced HR+/HER2- breast cancer treated with endocrine therapy and a CDK4/6 inhibitor.** (**a**) A plasma sample was obtained from 124 patients within 48 hours prior to starting endocrine therapy and CDK4/6 inhibition. ctDNA-based signatures were applied in plasma samples with a TF≥3% (n=87). (**b**) RB-LOH ctDNA-based signature score in patients with complete or partial response (CR/PR), stable disease and progressive disease (PD). (**c**) Kaplan-Meier curves of PFS (left) and OS (right) of the RB-LOH ctDNA-based signature. Each patient group is based on tertiles. (**d**) Average ctDNA signal of 16 features of the original RB-LOH DNA-based signature (column on the left) and the weight and direction of each feature (column on the right) as previously reported in Xia et al [Xia Y, Fan C, Hoadley KA, Parker JS, Perou CM. Genetic determinants of the molecular portraits of epithelial cancers. Nature Communications 2019;10(1):5666 doi 10.1038/s41467-019-13588-2]*.* (**e**) Forest plots of hazard ratios (HRs) for PFS (left) and OS (right) of the RB-LOH DNA-based signature when evaluated in plasma alone (i.e., plasma - univariate; n=87), in plasma when adjusted for TF (n=87), in plasma when adjusted for PAM50 RNA-based subtypes (n=53), in plasma when adjusted for TF+PAM50+clinical variables (n=53), in tissue alone (i.e., tissue - univariate; n=63) and in plasma when adjusted for tissue and vice-versa (n=28). (**f**) ctDNA-based signature scores of the RB-LOH signature, the Luminal A signature, the 13q14.2 *RBI* locus and TF across 7 patients with paired plasma samples (baseline vs post-CDK4/6 inhibitor treatment). P-values (p) were determined by two-tailed paired t-tests.

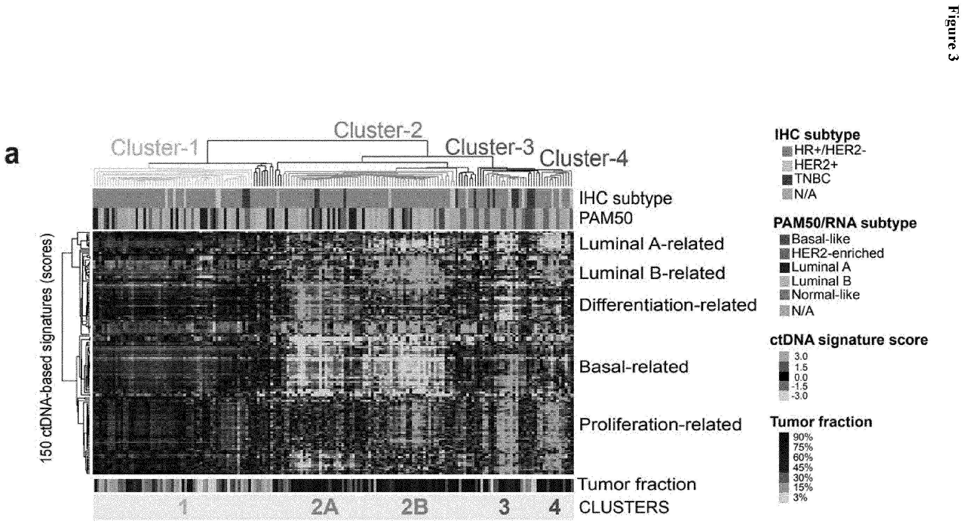

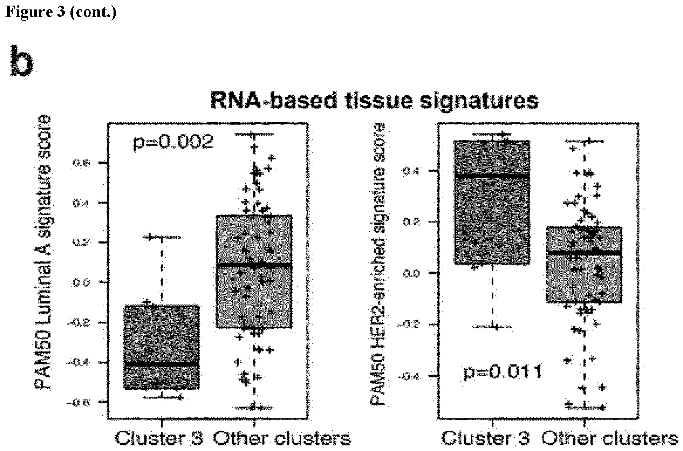

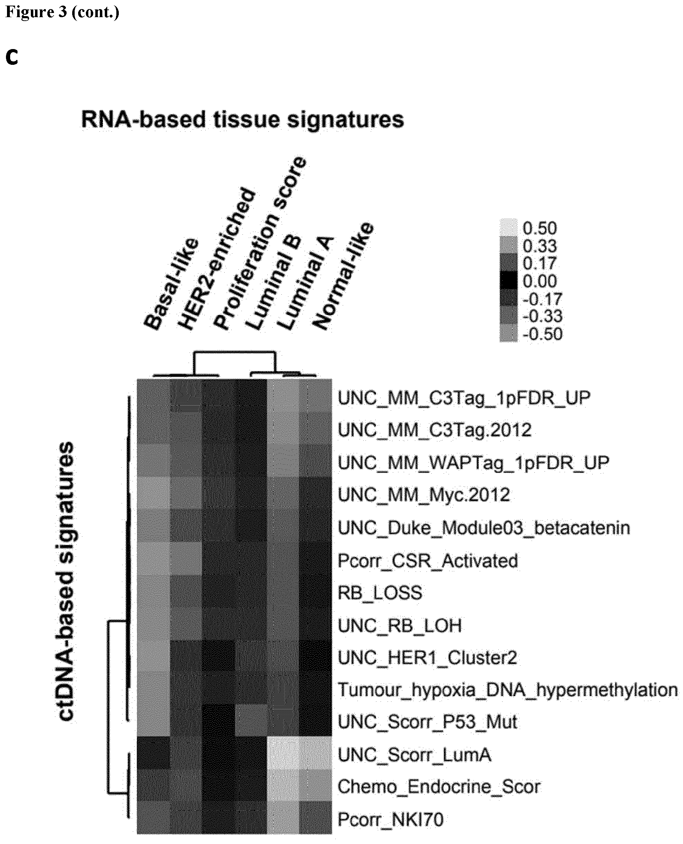

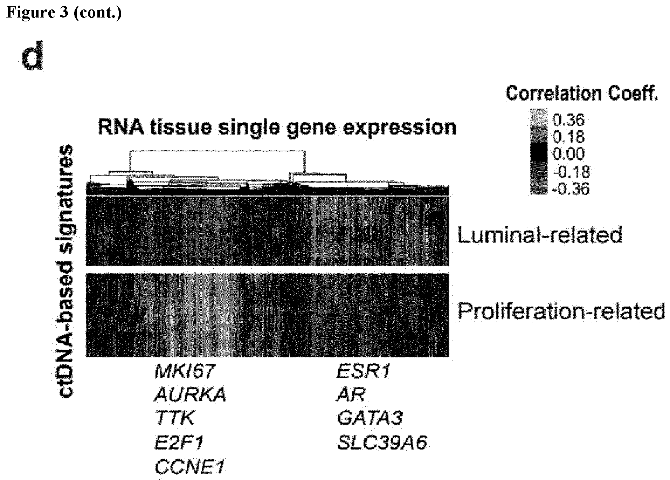

**Figure 3****. ctDNA-based profiling of metastatic breast cancer.** (**a**) Unsupervised cluster analysis of 178 plasma samples with a TF≥3% (columns) and the scores of 150 ctDNA-based signatures (rows). Orange and violet colors represent scores above and below the median score of the signature across the dataset. Below the array tree, the IHC subtype and the PAM50 molecular subtype are shown for each sample. Four clusters of samples (clusters 1 to 4) were identified. Within cluster 2, two subgroups of samples were also identified (clusters 2A and 2B). (**b**) Expression of 2 tissue PAM50 RNA-based signatures (i.e., Luminal A and HER2-enriched) in cluster 3 versus the other clusters. This analysis was performed in 107 paired plasma and tumor tissue samples. Of note, 58 tumor tissue samples were obtained at the same timepoint as the plasma sample and 49 tumor tissue samples were obtained at different timepoints prior to obtaining the plasma sample. (**c**) Unsupervised cluster heatmap analysis of Pearson's correlation coefficients obtained by comparing the scores of the top individual ctDNA-based signature versus the scores of each individual PAM50 RNA-based tissue signature across 58 matched-timepoint paired plasma-tissue cases. (**d**) Detail of an unsupervised cluster heatmap analysis representing the correlation coefficients obtained by comparing the scores of luminal-related and proliferation-related ctDNA-based signatures versus the log2 values of the mRNA expression of each of the 771 genes.

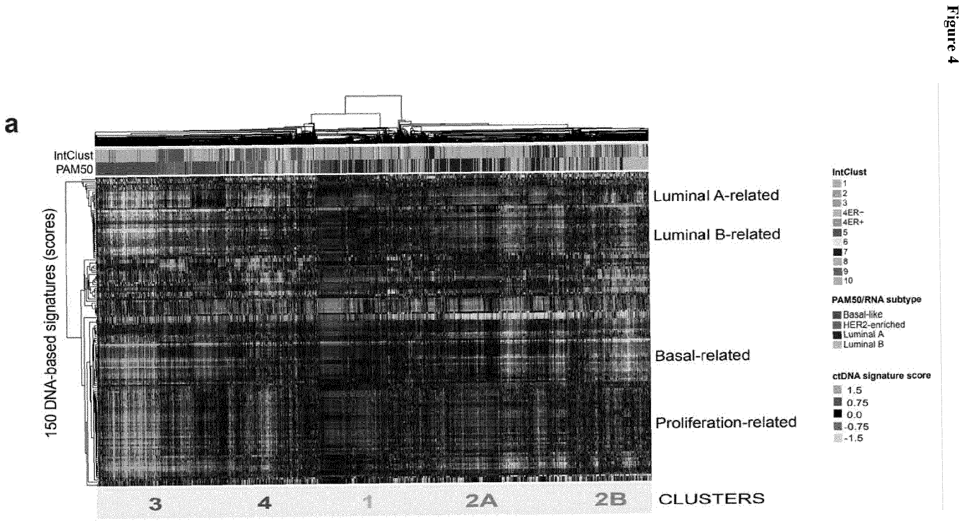

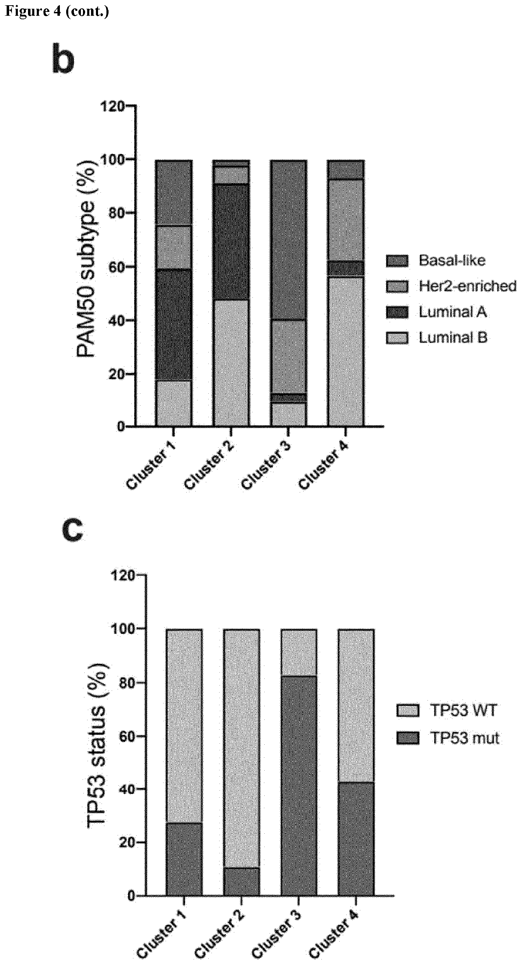

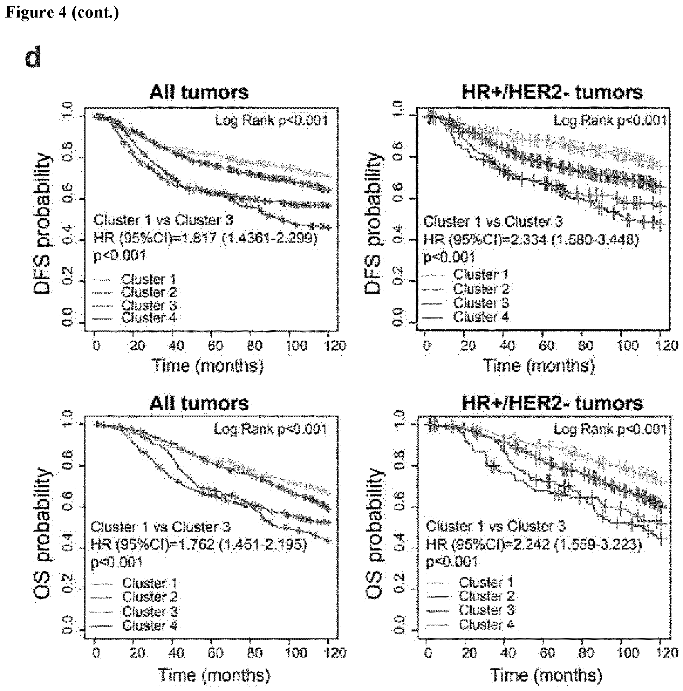

**Figure 4****. DNA-based tumor profiles in tissue samples and association with clinical outcomes.** (a) Unsupervised cluster analysis of 1,689 tumor samples (columns) from METABRIC dataset and the 150 DNA-based signatures scores (rows). Orange and violet colors represent scores above and below the median value of the signature across the dataset. Below the array tree, the InctClust classification and the PAM50 molecular subtypes are shown for each sample. The 4 clusters are shown below the data matrix. **(b)** PAM50 molecular subtype distribution across the 4 DNA-based clusters in 1,517 breast tumors of the METABRIC dataset. **(c)** *TP53* mutation distribution across the 4 DNA-based clusters in 1,517 breast tumors of the METABRIC dataset. **(d)** Kaplan-Meier curves of DFS (left) and OS (right) of the 4 DNA-based clusters assessed in all tumors (n=1,683) and HR+/HER2-negative tumors (n=1,131) of the METABRIC database.

### Detailed description of the invention

The present invention is illustrated by means of the Examples set below, without the intention of limiting its scope of protection.

### Example 1. Material and Methods

### Example 1.1. Study participants and samples

We collected baseline pre-treatment blood plasma samples from 124 patients with HR+/HER2-advanced breast cancer treated with endocrine therapy in combination with a CDK4/6 inhibitor (i.e., palbociclib, ribociclib or abemaciclib) at Hospital Clinic of Barcelona between the years of 2018 and 2021. All plasma samples were obtained before the start of treatment. In 7 patients, we obtained an additional plasma sample after progressing while on therapy.

To complement the blood plasma dataset of 124 patients treated with endocrine therapy and a CDK4/6 inhibitor, we collected 121 additional plasma samples from patients treated at Hospital Clinic of Barcelona at different stages of the disease: 85 plasmas from 77 patients with advanced HR+/HER2- breast cancer, 19 plasmas from 16 patients with HER2+ advanced breast cancer, 17 plasmas from 16 patients with advanced TNBC and 1 plasma from 1 patient with unknown ER and HER2 status. In addition, FFPE tumor tissues from 110 patients with available plasma samples were collected, including 71 patients treated with endocrine therapy and a CDK4/6 inhibitor. Finally, we collected FFPE tumors from 17 patients with HR+/HER2- advanced breast cancer who did not have plasma samples but were treated with endocrine therapy and a CDK4/6 inhibitor. The hospital institutional ethics committee approved the study in accordance with the principles of Good Clinical Practice, the Declaration of Helsinki, and other applicable local regulations. Written informed consent was obtained from all patients before enrolment. The medical records were retrospectively reviewed to obtain the necessary clinical data.

### Example 1.2. DNA-sequencing of plasma samples

Approximately 30 mL of peripheral blood was collected into K2-EDTA Vacutainer tubes (Becton Dickinson) and plasma isolation was performed within 2 hours of blood collection through two centrifugation steps. Centrifugation at 1,600xg for 10 minutes at 4°C separated plasma from peripheral-blood cells. Approximately 12 mL of plasma were obtained per patient, which were subsequently centrifugated at 16,000xg for 10 minutes at 4°C to remove the residual supernatant and any remaining contaminants including cells. Plasma samples were then aliquoted in 1.5 mL tubes and immediately stored at -80 °C. cfDNA was obtained from 3 mL of plasma using the QIAamp Circulating Nucleic Acid Kit (QIAGEN Inc.) according to the manufacturer's instructions and quantified with a Qubit dsDNA high-sensitivity assay kit and the Qubit 4.0 fluorometer (Life Technologies, Carlsbad, CA, USA). cfDNA was concentrated using SpeedVac to fulfil the requirements for library preparation. Library preparation was performed by ligating unique dual indexes (UDI) custom adapters to a minimum of 10 ng of the isolated cfDNA (10-50 ng dsDNA). More specifically, the fragment ends of cfDNA were blunted and 5' phosphorylated and, after that, 3' ends were A-tailed to favour adapter ligation. Adapters were 10 bp - UDI as recommended to mitigate errors introduced by index-hopping or switching in Illumina instruments with patterned flow cells, such as the NovaSeq 6000. Indexed libraries were quantified by qPCR using the KAPA Library Quantification Kit (Roche Sequencing Solutions), pooled, and sequenced in a NovaSeq 6000 Illumina at 0.5x mean coverage with read length of 2 x 150 bp. ShWGS was analyzed with hmmcopy _utils (https://github.com/shahcompbio/hmmcopy_utils) and ichorCNA v0.2.0 (https://github.com/broadinstitute/ichorCNA), with a bin size of 500kb and default parameters.

### Example 1.3. DNA-sequencing of FFPE tumor samples

DNA obtained from FFPE-derived tissues was purified with the QIAamp DNA FFPE Tissue kit (QIAGEN Inc.) for all samples available, following manufacturer's instructions. Quantification was performed with a Qubit dsDNA broad-range assay kit and the Qubit 4.0 fluorometer (Life Technologies, Carlsbad, CA, USA). A minimum of 100 ng of extracted DNA was processed for library preparation using a custom hybridization-based capture panel targeting 435 genes with reported somatic mutations in different tumor types (VHIO-300 v4 panel) performed with Agilent SureSelectXT Low Input Target Enrichment System (Agilent Technologies, Inc). Indexed libraries were quantified by qPCR using the KAPA Library Quantification Kit (Roche Sequencing Solutions), pooled and sequenced in a HiSeq 2500 Illumina (2 x 100bp) at an average coverage of 500x. Reads were aligned to the hg19 reference genome with BWA, applied GATK base quality score recalibration, indel realignment, duplicate removal, and performed variant calling using VarScan2 (v2.4.3) and Mutect2 (v4.1.0.0) with the following parameters: minimum variant allele frequency (VAF) of 5% for single nucleotide variants (SNVs) and 10% for Indels. Germline variants were excluded by filtering with single nucleotide polymorphisms (SNP) databases.

### Example 1.4. DNA-based signature estimation

For both tumor DNA sequencing and plasma cell-free ctDNA sequencing, segmentation files from CNVkit output (for tumor DNA) and ichorCNA output (ctDNA) were first mapped to gene-level feature. Values from 514 DNA segments were then determined as described in Xia et al [Xia Y, Fan C, Hoadley KA, Parker JS, Perou CM. Genetic determinants of the molecular portraits of epithelial cancers. Nature Communications 2019;10(1):5666 doi 10.1038/s41467-019-13588-2]. Briefly, each segment score was calculated as the mean copy number score across genes within the segment. The coefficients of DNA segments for predicting gene signatures were obtained from Xia et al. DNA-based signature scores were calculated as the weighted average of DNA segment values for each sample.

For ctDNA, TF and tumor ploidy were estimated by ichorCNA. For ctDNA samples with TF>0, TF and tumor ploidy adjusted signature scores were calculated by first adjusting copy number values in ichorCNA segmentation file: adjusted_copy_number_ratio = log2(logR_copy_number/tumor_ploidy). Then DNA-based signature scores were derived the same as described for tumor tissue. For calculating the number of altered segments, we used arbitrary gain/loss threshold of +/- 0.07 for unadjusted segment values and 0.32/-0.42 for adjusted segment values [Xia Y, Fan C, Hoadley KA, Parker JS, Perou CM. Genetic determinants of the molecular portraits of epithelial cancers. Nature Communications 2019;10(1):5666 doi 10.1038/s41467-019-13588-2]*.* Segments with values above the gain threshold or below the loss threshold were called altered.

### Example 1.5. Gene expression analysis of FFPE tumor samples

RNA was extracted using the High Pure FFPET RNA isolation kit (Roche, Indianapolis, IN, USA) following manufacturer's protocol. One to five 10-µm FFPE slides depending on tumor cellularity were used for each tumor sample, and macrodissection was performed, when needed, to avoid normal tissue contamination. A minimum of ~100 ng of total RNA was analyzed on the nCounter platform (Nanostring Technologies, Seattle, USA) using the 770-gene Breast Cancer 360^{™} Gene Panel, which includes the 50 PAM50 genes. Gene expression for each sample was independently normalized to the geometric mean of 5 housekeeping (*ACTB, MRPL19, PSMC4, RPLP0,* and *SF3A1*)*.* Research-based PAM50 subtyping was performed as previously described.

### Example 1.6. METABRIC breast cancer dataset

Clinical-pathological data was obtained from cbioportal. Processed DNA segment values were downloaded, and DNA-based signature scores were calculated as the weighted average of DNA segment values for each sample.

### Example 1.7. A DNA-based 4 subtype predictor

To identify the 4 subtype clusters using DNA-based data, we selected signatures that were significantly differentially expressed across the 4 clusters identified in ctDNA using a multi-class significance analysis of microarrays (SAM) with < 5% FDR. Then we used the selected gene list and calculated 4 centroids from the training data. For every new sample in METABRIC, we calculated the Euclidean distances to the 4 centroids and assigned a cluster class to each sample based on the nearest centroid.

### Example 1.8. General statistical procedures

Categorical variables were expressed as number (%) and compared by χ² test or Fisher's exact test. Differentially expressed signatures between two groups were identified using a two-class unpaired SAM with a FDR<5%. Differentially expressed signatures between two timepoints (i.e., baseline versus post-progression to endocrine therapy and a CDK4/6 inhibitor) were identified using a two-class paired SAM with an FDR<5%. Estimates of survival were from the Kaplan-Meier curves and tests of differences by the log-rank test. Univariate and multivariable Cox models for PFS and OS were used to test the prognostic significance of each variable. The Bonferroni correction method was used to control the family-wise error rate in case of multiple comparisons. PFS was defined as the period from initiation of endocrine therapy and a CDK4/6 inhibitor until disease progression or date of last follow-up. OS was defined as the period from initiation of endocrine therapy and a CDK4/6 inhibitor until death or date of last follow-up. All cluster analyses were displayed using Java Treeview version 1.1.3. Average linkage hierarchical clustering was performed using Cluster v3.0. Two-sided p-values <0.05 were considered statistically significant. Statistical computations were carried out in R 4.0.3 (http://cran.r-proiect.org).

### Example 2. Results

To demonstrate that ctDNA can capture complex tumor phenotypes, shallow whole genome sequencing (shWGS) was performed on 209 plasma samples from 174 patients with advanced hormone receptor-positive and HER2-negative breast cancer (HR+/HER2-). Additional samples from the other clinical subtypes were also assayed including 19 plasma samples from 16 patients with HER2-positive (HER2+) breast cancer, 17 plasma samples from 16 patients with triple-negative breast cancer (TNBC) and 1 plasma sample from 1 patient with unknown HR and HER2 status were also included.

### Example 2.1. Plasma tumor fraction

From 246 plasma samples (**Figure 1a**), 178 (72.4%) had a Tumor cell Fraction (TF) of≥3% (range 4-84%; median 9.4%), according to ichorCNA. In plasma samples with a TF≥3%, we calculated the scores for each of the 150 previously reported Elastic Net Regression analysis based DNA signatures that predict tumor RNA and protein phenotypes [Xia Y, Fan C, Hoadley KA, Parker JS, Perou CM. Genetic determinants of the molecular portraits of epithelial cancers. Nature Communications 2019;10(1):5666 doi 10.1038/s41467-019-13588-2], noting all signatures/models were applied exactly as previously reported; thus, in these cases, the 246 samples can be considered a 'test/validation' dataset. TF as a continuous variable was found strongly correlated with the number of altered DNA copy-number segments found in each sample (Pearson's rho = 0.76; **Figure 1b**). Strong correlations (i.e., Pearson's rho ≥0.70 or ≤-0.70) with TF were also identified in 46 of 150 (31.0%) ctDNA-based signatures, most of which were tracking biological processes associated with Luminal B (i.e., high TF) versus Luminal A (i.e., low TF) disease. This result reaffirms the hypothesis that TF not only reflects the amount of disease burden in each patient but also its biological aggressiveness. As expected, adjustment of the tumor copy-number signal detected in plasma by the TF in each sample decreased the strength of association between TF and the number of altered copy-number segments, and between TF and each ctDNA-based signature score (**Figure 1b**).

### Example 2.2. Plasma versus tissue DNA-based signatures

We next explored the correlation of each of the 150 DNA-based signatures determined using plasma ctDNA versus tumor DNA across 54 patients with available paired sample-types obtained at different timepoints (Figure 1c). Tumor DNA sequencing was performed from formalin-fixed paraffin-embedded (FFPE) tumors using a capture-based approach that covers the entire chromosomal landscape while the ctDNA shWGS was not FFPE DNA. Across all 150 signatures, the average correlation coefficient was 0.40 (range 0.02 to 0.66) and 40 signatures (26.7%) had a correlation coefficient ≥0.50. When the correlations were evaluated in 27 cases where plasma and tumor were obtained within a timeframe of <8.0 weeks, the number of signatures with a correlation coefficient ≥0.50 was 63 (42% versus 19.3% in the 27 cases where plasma and tumor were obtained within >8.0 weeks; p-value<0.001). Overall, these results suggest a moderate association between ctDNA-based and tumor DNA-based signatures across timepoints and DNA sequencing approaches (i.e., ctDNA shWGS versus capture-based using FFPE DNAs).

### Example 2.3. ctDNA-based signatures versus tissue ER and HER2 status

Estrogen receptor (ER) expression by immunohistochemistry (IHC), and HER2 overexpression by IHC and/or amplification by in-situ hybridization, are key biological features of breast cancer. To evaluate the relationship between ctDNA-based information and ER or HER2 tumor clinical biomarker status we evaluated the association of each of the 150 ctDNA-based signatures with either ER clinical status (i.e., positive versus negative) or HER2 status (i.e., positive versus negative) in the 177 samples with TF>3% and that had tumor ER and HER2 IHC available. As expected, ctDNA-based signatures tracking luminal biological processes (e.g., luminal-cluster-signature) and GSEA-median-GP7-estrogen-signaling) were found enriched in ER+ disease (p<0.001; false discovery rate [FDR]<1%; highest AUC=0.77) compared to ER-negative disease (**Figure 1d**). Similarly, ctDNA-based signatures tracking HER2 expression or amplification (e.g., HER2-signature and HER2-amplified-HER2-amplicon) were found significantly enriched (p<0.001; FDR<1%; highest AUC=0.72) in HER2+ disease compared to HER2-negative disease (**Figure 1d**). Overall, these results suggest that ctDNA-based profiling captures and predicts specific phenotypic tumor traits.

### Example 2.4. Prognosis of ctDNA-based signatures

To evaluate the association of ctDNA-based signatures with prognosis, we evaluated baseline pre-treatment plasma samples from 124 patients with advanced HR+/HER2- breast cancer treated with endocrine therapy and a CDK4/6 inhibitor (**Figure 2a**). Eighty-seven plasma samples had a TF>3%. The median follow-up was 12.5 months (range 1.0 to 56.7 months), and most patients were defined as endocrine-sensitive (83.9%) and treated in the first-line setting (59.8%) (**Table 8**).

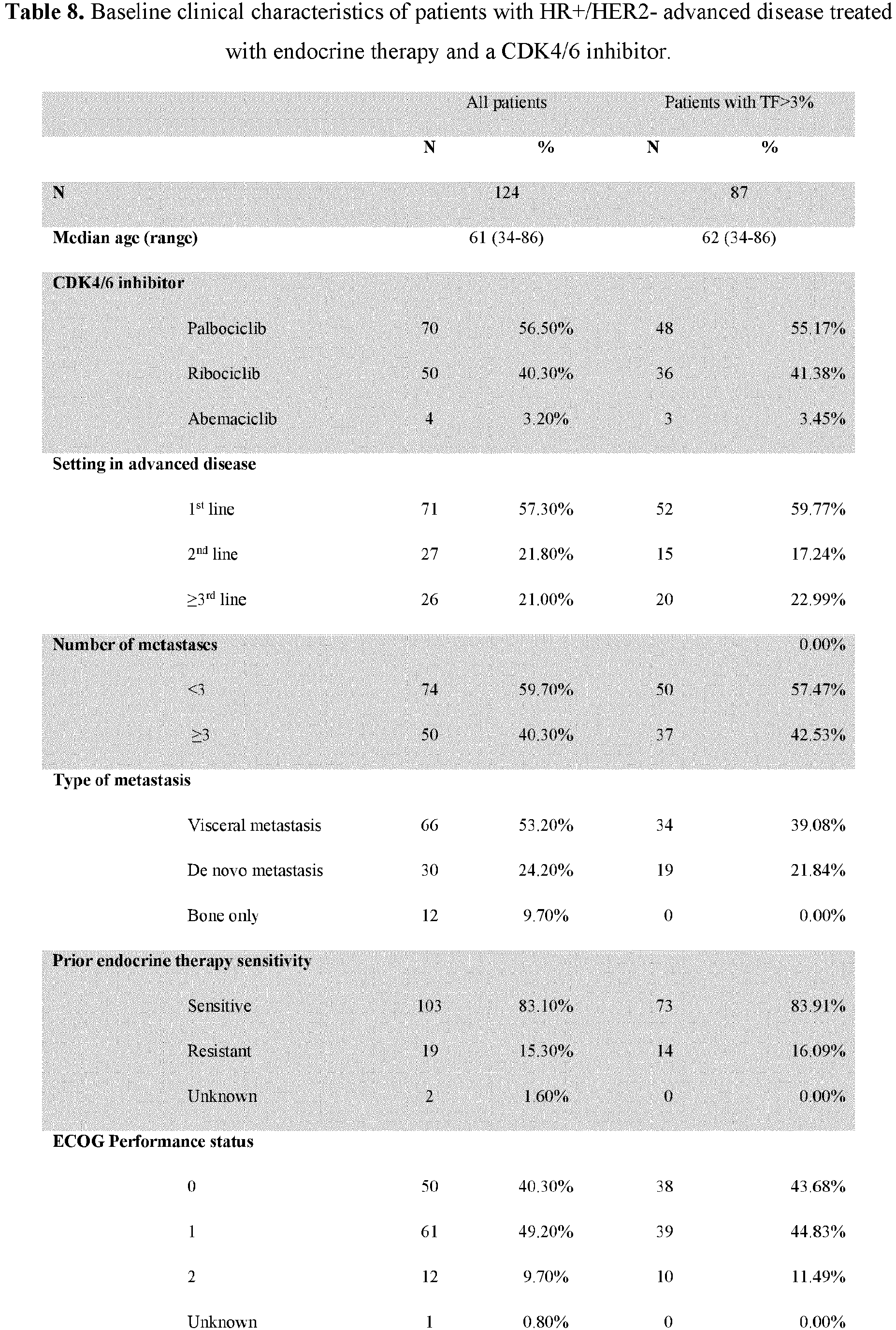

From the 150 ctDNA-based signatures, 36 (24%) and 37 (25%) were found significantly associated with progression-free survival (PFS) and overall survival (OS), respectively, and 27 (18%) signatures were found significantly associated with both PFS and OS. In general, signatures associated with poor survival outcome were those hypothesized to be tracking proliferation- and non-ER+/non-luminal-related biological processes, such as the MM_p53null.Luminal (i.e., TP53-deficient) and MM_Myc signatures (i.e., high MYC/MYC amplification). Conversely, ctDNA-signatures associated with better outcome were tracking luminal A-related biological processes.

Consistent with the known mechanism of resistance of CDK4/6 inhibitors, high enrichment of a signature tracking RB-LOH was associated with poor outcome and treatment response (**Figure 2b****-c**). The DNA-based RB-LOH signature is composed of 224 copy number features, including amplification of 2p (e.g., ETV6), 3q (e.g., PIK3CA), 8q (e.g., MYC), 20q (e.g., AURKA) and 21q (e.g., TMPRSS2 and ERG), and deletion of 2q (e.g., PARD3B), 4q, 5q, 12q, 13q (e.g., RB1), 15q and 17p. As expected, the direction (i.e., amplification or deletion) and strength (i.e., coefficient) of the 48 main features of the original tissue-based DNA RB-LOH signature [Xia Y, Fan C, Hoadley KA, Parker JS, Perou CM. Genetic determinants of the molecular portraits of epithelial cancers. Nature Communications 2019;10(1):5666 doi 10.1038/s41467-019-13588-2] were properly detected in ctDNA (correlation coefficient = 0.75, p-value<0.001; **Figure 2d**). Finally, the association of the ctDNA RB-LOH signature with PFS and OS was independent of TF (as a continuous variable), type of CDK4/6 inhibitor, line of treatment (first line versus second line versus later lines), presence of visceral disease and number of metastasis (**Figure 2e**).

### Example 2.5. ctDNA RB-LOH signature versus ctDNA RB1 individual region

The DNA-based RB-LOH signature considers the signal of the *RB1* locus (13q14.2) among 224 other features [Xia Y, Fan C, Hoadley KA, Parker JS, Perou CM. Genetic determinants of the molecular portraits of epithelial cancers. Nature Communications 2019;10(1):5666 doi 10.1038/s41467-019-13588-2]. The correlation coefficient between the ctDNA signal of 13q14.2 and the ctDNA RB-LOH signature score was -0.12 across the 178 samples with TF>3%. In the previous cohort of patients with advanced HR+/HER2- breast cancer treated with endocrine therapy and a CDK4/6 inhibitor, the ctDNA signal of the individual 13q14.2 segment was not significantly associated with PFS (p-value=0.061) but was significantly associated with OS (p-value=0.020). However, the RB-LOH signature was the only variable significantly associated with PFS and OS in a bivariate cox model. Overall, the RB-LOH ctDNA-based signature better captured the clinical behavior than did an individual DNA region looking only at *RB1*, thus highlighting the power of a multi-feature algorithm for sensing pathway activity.

### Example 2.6. Signal from individual DNA segments as prognostic drivers

To further understand the prognostic value of individual DNA segments, we focused on the 27 ctDNA-based signatures (see **Table 2** and **Table 3** above) significantly associated with both PFS and OS. From each signature, we evaluated the signal from 534 DNA segments and their original weights. We identified 16 DNA segments whose weights are high (i.e., defined as >0.10 or <-0.10) in at least 11 of 40 (40%) signatures. Among them, deletion of 13q14.2 (where RB1 is located) and 17p13.1 (e.g., TP53) and amplification of 8q24.21 (e.g., MYC) and 12p13.33 (e.g., FOXM1). Next, we evaluated the association of each of the 16 DNA segments with PFS in 87 patients with advanced HR+/HER2- breast cancer treated with endocrine therapy and a CDK4/6 inhibitor. The signal from 3 of 16 (18.8%) DNA segments were associated with PFS. We then combined signals from 2 segments (i.e., a total of 120 different combinations), and evaluated their individual association with PFS. A total of 40 different combinations of 120 (30%) were significantly associated with PFS and all 16 DNA segments were found in at least 1 combination (see **Table 1, Table 3** and **Table 4** above).

### Example 2.7. Prognosis of RB-LOH in tumor versus plasma

To compare the prognostic value of the DNA-based RB-LOH signature when determined in tumor versus plasma, 63 of 124 patients (51.0%) with advanced HR+/HER2- breast cancer treated with endocrine therapy and a CDK4/6 inhibitor had paired tumor DNA samples (**Figure 2e**). In a univariate analysis, both RB-LOH tumor and ctDNA plasma signatures (as continuous variables) were significantly associated with PFS and OS. When both signatures were evaluated head-to-head in a bivariate cox model, the RB-LOH ctDNA plasma signature was found significantly associated with PFS, but not the RB-LOH tumor signature (**Figure 2e**). Overall, baseline pre-treatment ctDNA better captures the prognosis of patients than archival tumor tissue DNA.

### Example 2.8. Capturing biological features before and after endocrine therapy and CDK4/6 inhibition

Scores from the 150 ctDNA-based signatures were evaluated in paired plasma samples (i.e., baseline versus post-treatment after progressive disease) across 7 patients with advanced HR+/HER2- breast cancer treated with endocrine therapy and a CDK4/6 inhibitor (**Figure 2f**). Among them, 103 signatures (57.2%) were found differentially enriched between the two timepoints (FDR<5%). As might be expected, enrichment of signatures tracking non-luminal/proliferation-related biological processes (e.g., RB-LOH) and luminal A related biological processes were found significantly increased and decreased, respectively, in post-treatment samples compared to pre-treatment samples (**Figure 2f**). Of note, TF did not significantly change between the two timepoints across the 7 patients (**Figure 2f**), and 1 patient with a substantial decrease in TF still showed an increase in the RB-LOH score and a decrease of the luminal A signature. These biological changes identified in ctDNA are concordant with similar biological changes identified across 18 patients with paired tumor-based RNA expression before and at progression to endocrine therapy and a CDK4/6 inhibitor. Specifically, PAM50 Luminal A and proliferation signatures were found significantly decreased and increased, respectively, in progression samples.

### Figure 2.9. ctDNA-based tumor profiling

To explore the biology identified by the 150 ctDNA-based signatures, we performed an unsupervised hierarchical cluster analysis of all 150 signatures across 178 plasma samples with a TF≥3% (**Figure 3a**). Four main clusters of samples were identified using consensus clustering plus; Clusters 3 and 4 showed high scores of ctDNA-based proliferation-related signatures and low scores of differentiation status and of luminal A-related signatures. Compared to Cluster 4, Cluster 3 showed high expression of basal-like gene expression subtype related biology (p-value<0.001). Cluster 2 showed high enrichment of differentiation and luminal B-related signatures, and low enrichment of basal-like related biology. Visually, cluster 2 could be further subdivided (minimum 20 samples and a correlation coefficient >0.75) into Cluster2A and Cluster2B, both of which showed differences in the enrichment of ctDNA-based proliferation features, and luminal A-related signatures. Consistent with the Luminal A-related biology identified in Custer 2A, this group was characterized by 16p amplification and 16q deletion, both of which are known features of low-grade and low-proliferative breast cancers. Finally, Cluster 1 showed low enrichment of proliferation and luminal B-related signatures and high enrichment of luminal A subtype related signatures. Plasma TF in Cluster 1 was significantly lower compared to the other clusters combined (average 6.8% vs 9.8%, p-value<0.001; **Figure 3a**), which might be predicted for slow growing luminal A tumors.

### Figure 2.10. ctDNA-based data versus tissue RNA-based expression data

RNA-based expression data from FFPE tissue using a research-based PAM50 intrinsic subtype assay was available for 108 cases with a TF ≥3% in plasma. Tissue samples were obtained at various timepoints. As expected, Cluster 3 was enriched for tumors with a PAM50 non-luminal subtype (i.e., HER2-enriched or Basal-like) compared to the other clusters (85.7% versus 25%, p-value<0.001). Concordant with this finding, the PAM50 Luminal A and HER2-enriched signatures (as a continuous variable) were found differentially expressed in Cluster 3 versus the other clusters (**Figure 3b**). In addition, we observed that Cluster 2B was enriched for PAM50 Luminal B tumors compared to Cluster 2A (53.3% versus 18.8%, p-value=0.044).

To further explore the association between ctDNA enrichments and RNA expression data, we evaluated the correlation of 6 PAM50 RNA-based tissue signatures with each of the 150 ctDNA-based signatures. To summarize these results, we plotted in an unsupervised cluster analysis the correlation coefficients of the most correlated signatures across 58 matched-timepoint paired plasma-tissue cases (**Figure 3c**). In general, ctDNA-based signatures were positively and negatively correlated with the known biology that each PAM50 subtype signature is hypothesized to be tracking. For example, a ctDNA-based signature tracking RB-LOH gene expression signature, which is enriched in E2F target genes and that tracks tumor proliferation rates, was found positively correlated to the PAM50 RNA-based Basal-like, HER2-enriched and proliferation tumor signatures, and negatively correlated to the PAM50 RNA-based Luminal A tumor signature (**Figure 3c**).

Expression of 771 genes in tumors using the nCounter Breast Cancer 360 Panel was determined in 107 cases with a TF ≥3% in plasma. Correlation coefficients of the mRNA expression of each individual gene with each 150 ctDNA-based signature scores were also determined. Like the PAM50 RNA tumor signatures, the mRNA expression of luminal genes (e.g., *ESR1* and *GATA3*) was positively correlated with luminal ctDNA-based signatures, while proliferation and cell cycle-related genes by mRNA (e.g., *MKI67, AURKA, TTK, E2F1 and CCNE1*) were positively correlated with proliferation-related ctDNA-based signatures (**Figure 3d**). Overall, these findings confirm that DNA-copy number-based signatures coming from ctDNA can track the main breast cancer phenotypes and their known gene expression features.

### Example 2.11. Prognosis of ctDNA-based tumor subtypes

This work demonstrates that tumor profiles identify samples with similar patterns of expression, and these patterns are associated with clinically relevant genotypes. We then hypothesized that ctDNA-defined subtypes, representing repeatably observed combinations of these patterns, may explain variation in clinical outcome. To explore this, we evaluated the prognostic value of the 4 ctDNA-based tumor groups (**Figure 3a**). Compared to Clusters 1-2-4 (as a group), Cluster 3 was significantly associated with worse PFS (median 2.4 vs. 11.6 months; hazard ratio=9.23; 95% confidence interval [CI] 3.1-27.3; p-value<0.001). Regarding OS, a tendency was observed which did not reach statistical significance (median 16.8 vs. 55.4 months; hazard ratio=2.73; 95% CI 0.79-9.38; p-value=0.112). As expected, the expression of the ctDNA-based RB-LOH signature was significantly higher in Cluster 3 compared to the other clusters (p<0.001).

### Example 2.12. DNA-based tumor subtypes in tissue samples

To explore how tumor subtypes identified in ctDNA data perform when applied to tumor tissue DNA, the scores of the 150 DNA-based signatures were determined and evaluated using tumor DNAs from 1,038 patients with early-stage breast cancer from the publicly available METABRIC dataset (**Figure 4a**). We developed a 4-class subtype classifier from the ctDNA groups (**Figure 3a**) and applied this predictor onto METABRIC's tumor DNA data. Overall, the 4 main clusters were identified in METABRIC, including Cluster 1, suggesting that the profiles identified in ctDNA are observed in primary tumors. Concordant with this finding, the PAM50 non-luminal subtypes in the METABRIC cohort were enriched in Cluster 3 compared to the other clusters (79.6% versus 11.6%, p-value<0.001) (**Figure 4b**). In addition, *TP53* somatic mutations in METABRIC were also enriched in Cluster 3 compared to the other clusters (80.7% versus 51.2%, p-value<0.001) (**Figure 4c**). Finally, the 4 main clusters were found significantly associated with disease-free survival (DFS) and OS in all patients, and in patients with HR+/HER2-negative breast cancer (**Figure 4d**).

## Claims

1. *In vitro* method for predicting the response of patients with HR+/HER2- breast cancer to a treatment selected from targeted therapy, comprising CDK4/6 inhibitors, and/or endocrine therapy, which comprises:
a. Assessing the presence of DNA copy number alterations (CNA) over any of the following DNA segments in a biological sample obtained from the patient: chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 or chr3:58626894-61524607;
b. Processing the measured CNAs in order to obtain a score;
c. Wherein if a deviation or variation of the score value is identified, as compared with a pre-established reference value, this is indicative of whether the patient with HR+/HER2- breast cancer will respond to the treatment.

2. *In vitro* method for the prognosis of patients with HR+/HER2- breast cancer which comprises:
a. Assessing the presence of CNAs over any of the following DNA segments in a biological sample obtained from the patient: chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 or chr3:58626894-61524607;
b. Processing the measured CNAs in order to obtain a score;
c. Wherein if a deviation or variation of the score value is identified, as compared with a pre-established reference value, this is indicative of the prognosis of the patient with HR+/HER2- breast cancer.

3. *In vitro* method for monitoring patients with HR+/HER2- breast cancer to assess whether they are responding to a treatment selected from targeted therapy, comprising CDK4/6 inhibitors, and/or endocrine therapy which comprises:
a. Assessing the presence of CNAs over any of the following DNA segments in a biological sample obtained from the patient: chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 or chr3:58626894-61524607;
b. Processing the measured CNAs in order to obtain a score;
c. Wherein if a deviation or variation of the score value is obtained, as compared with a pre-established reference value, this is indicative of whether the patient with HR+/HER2- breast cancer is responding to the treatment.

4. *In vitro* method for classifying patients with HR+/HER2- breast cancer into groups associated with a different response to a treatment selected from targeted therapy, comprising CDK4/6 inhibitors, and/or endocrine therapy, which comprises:
a. Assessing the presence of over any of the following DNA segments in a biological sample obtained from the patient: chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 or chr3:58626894-61524607;
b. Processing the measured CNAs in order to obtain a multiple scores;
c. Classifying breast cancer samples based on their scoring profile into different groups; and
d. Wherein if a deviation or variation of the score value obtained in each group is identified, as compared with a pre-established reference value, this is indicative of whether the patient group with HR+/HER2- breast cancer will respond to the treatment.

5. *In vitro* method, according to any of the previous claims, **characterized in that** it is a computer-implemented method which comprises:
a. Receiving a plurality of CNAs data sets from the patient;
b. Processing the information according to step a) for finding a statistically significant variations or deviations;
c. Providing a result by the computer system based on the information received according to the step a) and a pre-established standard already stored in the computer.

6. *In vitro* method, according to any of the previous claims, wherein the presence of CNAs is assessed in all the following the DNA segments: chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 or chr3:58626894-61524607.

7. *In vitro* method, according to any of the previous claims, wherein the presence of CNAs is assessed in any or all the DNA segments of Table 5, Table 6 or Table 7.

8. *In vitro* method, according to any of the previous claims, wherein the biological sample is selected from plasma, serum, breast milk, cerebrospinal fluid or blood samples.

9. *In vitro* method, according to any of the previous claims, wherein the cancer subtype is selected from: HR+/HER2-, HER2+ and triple negative.

10. Kit, suitable for performing any of the methods of claims 1 to 9, comprising tools and reagents for assessing the presence of CNAs in the following the DNA segments: chr20:33386980-33969561, chr13:46362859-48209064, chr17:63942109-65847254, chr17:1-22200000, chr10:129812260-135374737, chr17:7471230-7717938, chr2:32460827-55039898, chr7:16017926-18944036, chr2:1-93300000, chr8:128774432-128849112, chr12:1-1311104, chr16:1-38200000, chr4:83634873-83961360, chr5:76408288-81082828, chr19:1-526082 and/or chr3:58626894-61524607.

11. Kit, according to claim 10, comprising tools and reagents for assessing the presence of CNAs in any or all the DNA segments of Table 5, Table 6 or Table 7.

12. Use of the kit of claims 10 or 11 for predicting the response of patients with HR+/HER2-breast cancer to a treatment selected from targeted therapy, comprising CDK4/6 inhibitors, and/or endocrine therapy; for the prognosis of patients with HR+/HER2- breast cancer; for monitoring patients with HR+/HER2- breast cancer to assess whether they are responding to a treatment selected from targeted therapy, comprising CDK4/6 inhibitors, and/or endocrine therapy; or for classifying patients into groups associated with a different response to a treatment selected from targeted therapy, comprising CDK4/6 inhibitors, and/or endocrine therapy.

13. Targeted therapy, comprising CDK4/6 inhibitors, and/or endocrine therapy, for use in the treatment of patients with HR+/HER2- breast cancer, wherein the patients have been identified as responder patients according to any of the method of claims 1 to 9.
